# EUROPEAN PATENT APPLICATION

(11) **EP 4 752 154 A1**
(43) Date of publication of application: **03.06.2026**
(21) Application number: 24846049.5
(22) Date of filing: 24.07.2024
(51) Int. Cl.: C07K 7/54, C07K 5/11, A61K 49/00, A61K 51/08

(54) **PEPTIDE STRUCTURE TARGETING CARBONIC DEHYDROGENASE IX AND USE THEREOF**

(30) Priority: 25.07.2023 KR 20230096689
(71) Applicant: C-Biomex Co., Ltd., Pohang-si, Gyeongsangbuk-do 37673 (KR)
(72) Inventor: LEE, Song Gil, Pohang-si, Gyeongsangbuk-do 37666 (KR); HA, Hye Sook, Pohang-si, Gyeongsangbuk-do 37671 (KR); KIM, Eung Kyun, Pohang-si, Gyeongsangbuk-do 37669 (KR); PARK, Jin Hwi, Pohang-si, Gyeongsangbuk-do 37651 (KR); HWANG, Yun Jeong, Pohang-si, Gyeongsangbuk-do 37655 (KR); LEE, Seo Jin, Pohang-si, Gyeongsangbuk-do 37837 (KR); KIM, Jae Beom, Pohang-si, Gyeongsangbuk-do 37656 (KR); HONG, Su Won, Pohang-si, Gyeongsangbuk-do 37681 (KR); SEO, Seung Oh, Pohang-si, Gyeongsangbuk-do 37663 (KR); AHN, Jun Young, Pohang-si, Gyeongsangbuk-do 37689 (KR); KANG, Kyoung Min, Gyeongju-si, Gyeongsangbuk-do 38004 (KR); CHA, Jun Hoe, Seoul 06294 (KR)
(74) Representative: Meissner Bolte Partnerschaft mbB
(86) International application number: PCT/KR2024/010757
(87) International publication number: WO 2025/023747

(57) **Abstract**

The present invention relates to a peptide structure specifically binding to carbonic anhydrase IX (CAIX), and a use thereof. The CAIX-binding peptide ligand of the present invention contains D-amino acids, and thus has high binding specificity to CAIX while being stable in the body, and a cyclic CAIX-binding peptide structure comprising same can bind to CAIX with high affinity in the body, and thus is effective in the diagnosis, prevention, suppression or treatment of diseases mediated by CAIX.

## Description

### TECHNICAL FIELD

The present invention relates to a peptide construct specifically binds to carbonic anhydrase IX (CAIX). Specifically, the present invention relates to a high-affinity CAIX-binding peptide construct comprising a CAIX-binding peptide ligand that is specific for CAIX and has improved stability due to the inclusion of D-amino acids and uses thereof for diagnosing, preventing, inhibiting or treating diseases mediated by CAIX.

This research was supported by Korea Drug Development Fund funded by Ministry of Science and ICT, Ministry of Trade, Industry, and Energy, and Ministry of Health and Welfare (RS-2023-00218641, Republic of Korea).

### BACKGROUND ART

Carbonic Anhydrase (CA), which is a zinc (Zn²⁺) metalloenzyme, commonly exists in higher vertebrates including humans. It is an enzyme that catalyzes a reversible hydration reaction that converts carbon dioxide into a hydrogen ion and a bicarbonate ion (CO₂+H₂O↔HCO³⁻+H⁺). CAs have been identified in 16 isozyme forms and exist in various tissues in humans such as gastrointestinal tract, reproductive tract, nervous system, kidney, lung, skin, and eyeball. CA isoenzymes are mostly known to be involved in important physiological processes such as respiration, calcification, acid-base balance, bone resorption, formation of aqueous humor, cerebrospinal fluid, saliva and gastric acid (Thiry et al., TRENDS in Pharmacological Sciences, 27(11): 566-573, 2006).

Among the CA family, carbonic anhydrase IX (CAIX) is expressed very restrictedly in normal tissues and abnormally overexpressed in the majority of solid tumors. It has been reported that this is mainly due to strong transcriptional activation by hypoxia-inducible factor (HIF-1), which is a transcription factor induced by hypoxia caused by excessive proliferation of solid tumors (De Simone et al., Biochimica et Biophysica Acta, 1804:404-409, 2010; Thiry et al., *ditto*).

Tumor hypoxia results from the creation of an oxygen-poor environment as solid tumors grow at a rate that exceeds the blood supply capacity provided by the host's vascular system. Even in a hypoxic microenvironment, the solid tumors maintain continuous growth and proliferation through various genetic mutations. These hypoxic tumor cells are challenging to target with anti-cancer chemotherapy drugs through the bloodstream, and they lack the oxygen required for the cytotoxic action of radiation-derived free radicals during radiotherapy. Consequently, this leads to increased resistance to both anti-cancer chemotherapy and radiotherapy. In addition, the hypoxic tumor cells induce overexpression of CAIX on cell surfaces, thereby lowering the pH of the extracellular environment of tumor cells due to hydration of CO₂ by the extracellular catalytic domain of CAIX. The acidic tumor microenvironment thus formed can promote invasion and metastasis of tumor cells and neutralize pH-sensitive drugs (Thiry *et al., ditto*). Therefore, tumor hypoxia is generally known to be a poor prognostic factor for cancer patients.

Recently, studies to inhibit the growth and proliferation of CAIX-related tumors have been actively conducted by targeting CAIX overexpressed in tumor cells or by disrupting the pH regulation induced by tumor cells by way of interfering with the catalytic activity of CAIX. Most of these studies concentrate on the development of CAIX-binding monoclonal antibodies and sulfonamide-based small molecule inhibitors. However, delivering high-molecular-weight monoclonal antibodies efficiently to solid tumors poses challenges and sulfonamide-based small molecule inhibitors are relatively unstable in solution, which limit their usefulness as pharmaceutical compounds.

Therefore, there is a demand for the development of novel CAIX-specific binding agents and inhibitors with high affinity and stability and suitability for medical and pharmaceutical applications in cancer treatment, which include treatment, prevention, diagnosis, prognosis prediction, and imaging of cancer diseases.

### [Prior Art Literature]

### [Non-Patent Literature]

(Non-patent literature 1) Thiry et al., TRENDS in Pharmacological Sciences, 27(11): 566-573, 2006
(Non-patent literature 2) De Simone et al., Biochimica et Biophysica Acta, 1804:404-409, 2010

### DETAILED DESCRIPTION

### TECHNICAL OBJECTIVE

One objective of the present invention is to provide a stable CAIX-specific binding agent and inhibitor suitable for uses in treatment, prevention, diagnosis or prognosis prediction of cancer diseases.

One objective of the present invention is to provide a high-affinity CAIX-specific peptide construct comprising one or more effectors or functional groups, including sulfonamide functional groups, together with a CAIX-specific binding peptide ligand.

Another objective of the present invention is to provide a conjugate comprising the peptide construct.

Another objective of the present invention is to provide a composition for diagnosing, preventing or treating cancer comprising the peptide construct or the conjugate.

Another objective of the present invention is to provide a method for diagnosing cancer using the peptide construct or the conjugate.

Another objective of the present invention is to provide a method for treating cancer using the peptide construct or the conjugate.

Another objective of the present invention is to provide a method for predicting prognosis after treating cancer using the peptide construct or the conjugate.

### MEANS FOR SOLVING THE PROBLEMS

As a result of research to achieve these goals, the inventors of the present application have developed a novel CAIX-specific binding agent and inhibitor, which is stable due to the inclusion of D-amino acids and can specifically bind to CAIX with high affinity.

In one aspect, the present invention provides a peptide construct characterized by comprising:
a peptide ligand comprising an amino acid sequence of any one of SEQ ID NOs: 1 to 29, wherein at least one of the constituent amino acids is composed of D-amino acids,
and a lysine (Lys) residue among the constituent amino acids is optionally substituted with a chemical functional group at the side chain ε-amino group, or a 2,3-diaminopropionic acid (DAP) residue among the constituent amino acids is optionally substituted with a chemical functional group at the side-chain β-amino group; and
a sulfonamide functional group-containing amino acid residue linked directly or via a spacer to the peptide ligand,
and having a structure represented by Formula 1 below: 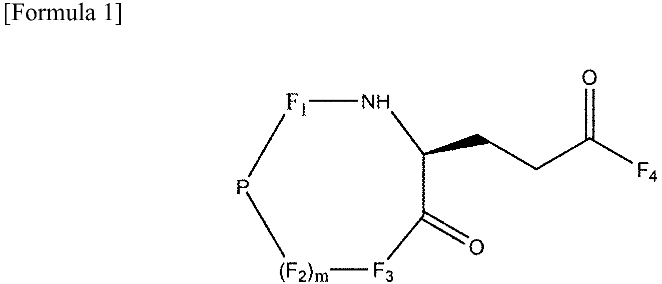
wherein P is the peptide ligand,
F₁, F₂ and F₃ are each independently a sulfonamide functional group-containing amino acid residue,
m is 0 or 1,
F₄ is a group of the general formula -(F₅)ₒ-(S₁)ₚ-(F₆)_{q}-(S₂)ᵣ-(F₇)ₛ-(S₃), wherein
F₅ is a sulfonamide functional group-containing amino acid residue,
S₁ and S₂ are each independently a spacer,
F₆ and F₇ are each independently a functional group-containing amino acid residue other than sulfonamide,
S₃ is -NH₂ or -OH,
o is an integer of 0 or 1, and
p, q, r, and s each independently represent an integer from 0 to 6.

Non-limiting examples of the chemical functional groups that may be substituted at the ε-amino group of the side chain of a lysine (Lys) residue among the constituent amino acids include 4-(p-iodophenyl)butyric acid (IB) or 2-(4-iodophenyl)acetic acid (sIB). In addition, non-limiting examples of the chemical functional groups that may be substituted at the β-amino group of the side chain of a 2,3-diaminopropionic acid (DAP) residue among the constituent amino acids include α-D-galacturonic acid (Gal).

In the CAIX-specific peptide construct, the sulfonamide functional group-containing amino acid residue may have the following construct, but is not limited thereto:

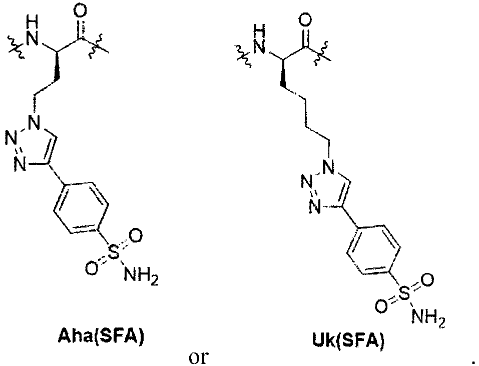

In the CAIX-specific peptide construct, a functional group other than sulfonamide may be introduced through the side-chain ε-amino group of lysine residues or the side-chain β-amino group of 2,3-diaminopropionic acid (DAP) residues. Examples thereof include chelator, biotin, glucoheptonic acid, 4-(p-iodophenyl)butyric acid (IB), 2-(4-iodophenyl)acetic acid (sIB), amphoteric ions (SZW, zwitterionic ions), α-D-galacturonic acid (Gal), cycloalkane having 5 to 15 carbon atoms, fluorescent dyes or cytotoxic agents.

In one aspect, the present invention provides a conjugate comprising the CAIX-specific peptide construct linked, directly or via a linker, to fluorescent dyes, cytotoxic agents or radioactive isotopes.

In one aspect, the present invention provides a pharmaceutical composition for diagnosis, prevention or treatment of cancer comprising the peptide construct or conjugate. The cancer may be a cancer expressing carbonic anhydrase IX. The cancer can be selected from the group consisting of liver cancer, lung cancer, colorectal cancer, stomach cancer, breast cancer, colon carcinoma, bone cancer, pancreatic cancer, head and neck cancer, uterine cancer, ovarian cancer, rectal cancer, esophageal cancer, small intestine cancer, perianal cancer, fallopian tube carcinoma, endometrial carcinoma, cervical carcinoma, vaginal carcinoma, vulvar carcinoma, prostate cancer, biliary cancer, bladder cancer, kidney cancer, ureteric cancer, renal cell carcinoma, renal pelvic carcinoma, melanoma, thyroid cancer, astrocytoma or glioblastoma, but is not limited to thereto.

In one aspect, the present invention provides a method for diagnosing cancer comprising administering the CAIX-specific peptide construct or the conjugate to a subject.

In one aspect, the present invention provides a method for treating cancer comprising administering the CAIX-specific peptide construct or the conjugate to a subject.

In one aspect, the present invention provides a method for predicting prognosis after cancer treatment comprising administering the CAIX-specific peptide construct or the conjugate to a subject.

### WORKING EFFECT OF THE INVENTION

According to the present invention, the novel, high-affinity CAIX binding agent and inhibitor are provided, which specifically bind to CAIX and are stable. The CAIX binding agent of the present invention comprises a CAIX-specific binding peptide ligand comprising one or more D-amino acids, thereby exhibiting excellent stability in the body and being useful for CAIX targeting. In addition, the present invention provides the peptide construct in which one or more effectors or functional groups, including a sulfonamide functional group, are introduced into the CAIX-specific peptide ligand through the side chain of an amino acid residue, thereby the CAIX binding agent that are particularly useful for imaging or diagnosing cancer with high binding affinity to CAIX can be provided. The CAIX-specific peptide construct of the present invention can be used as an effective drug for diagnosing, preventing or treating cancer by conjugating fluorescent dyes, cytotoxic agents or radioactive isotopes thereto.

### BRIEF DESCRIPTION OF DRAWINGS

Fig. 1a shows graphs of the binding affinity and binding kinetics of Peptide Constructs according to one embodiment of the present invention to the extracellular domain (ECD) of *h*CAIX and the ECD of human carbonic anhydrase XII (*h*CAXII), analyzed using the BLItz^{®} system.
Fig. 1b shows graphs of the binding affinity and binding kinetics of Peptide Constructs 103 to 105 according to one embodiment of the present invention, and of Peptide Constructs 103 to 105 labeled with lutetium-175 (Lu-175), to the *h*CAIX extracellular domain (ECD), analyzed using the Octet^{®} R8 system.
Fig. 1c shows graphs of the binding affinity and binding kinetics of Peptide Constructs 106 to 107 according to one embodiment of the present invention to the *h*CAIX extracellular domain (ECD), analyzed using the Octet^{®} R8 system.
Fig. 2a shows graphs of fluorescence-activated cell sorting (FACS) analysis results using the SK-RC-52 cell lines of the Peptide Constructs 4 to 19 according to an embodiment of the present invention.
Fig. 2b shows graphs of FACS analysis results using the SK-RC-52 cell lines of the Peptide Constructs 20 to 28, 61 to 66 and 71 according to an embodiment of the present invention.
Fig. 2c shows graphs of FACS analysis results using the SK-RC-52 cell lines of the Peptide Constructs 72 to 87 according to an embodiment of the present invention.
Fig. 2d shows graphs of FACS analysis results using the SK-RC-52 cell lines of the Peptide Constructs 88 to 102 according to an embodiment of the present invention.
Figs. 3a to 3m show the results of the biodistribution analysis of the Peptide Constructs according to the embodiment of the present invention in mice xenografted with the SK-RC-52 cell line.

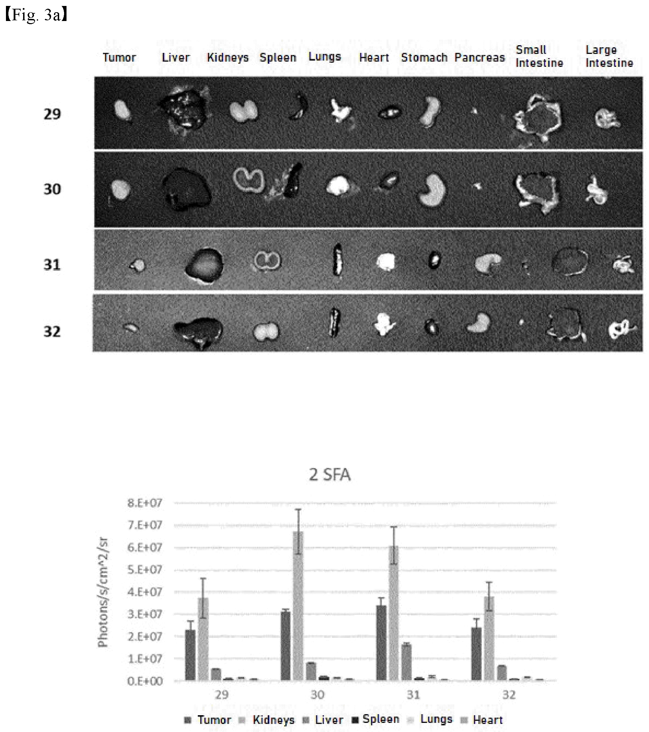

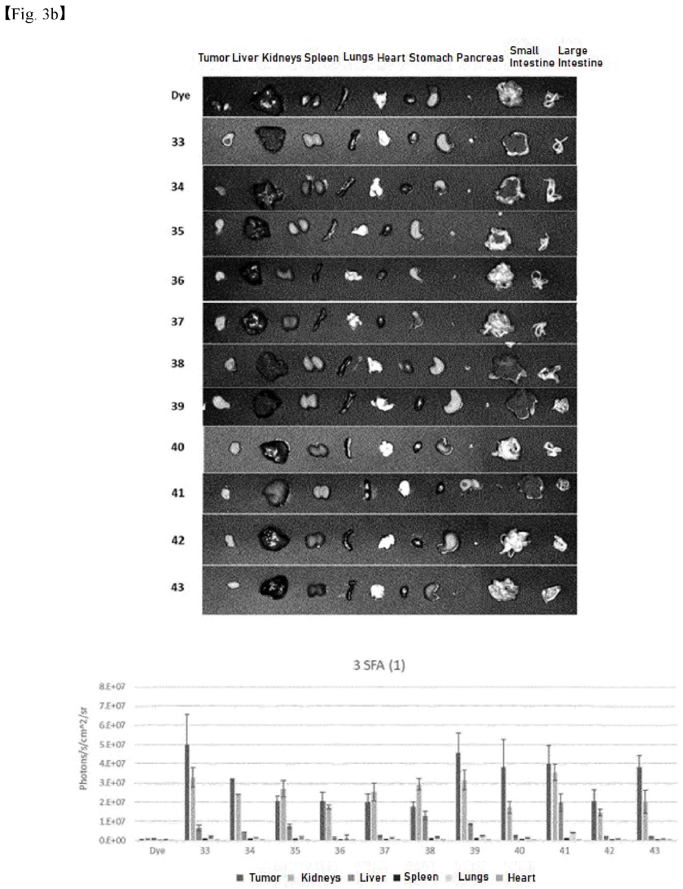

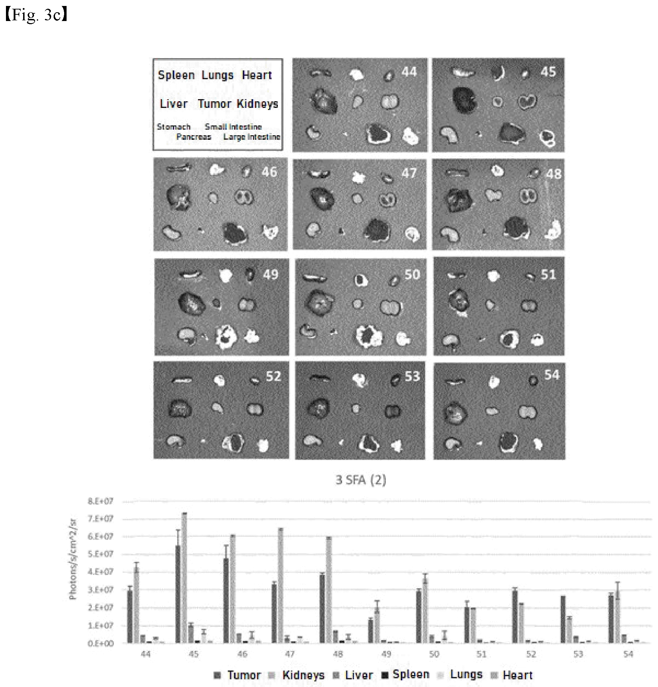

Figs. 3a to 3c show *ex vivo* images and fluorescence intensity graphs of tumors and organs extracted 24 hours after injection in mice each injected with Peptide Constructs 29 to 54.

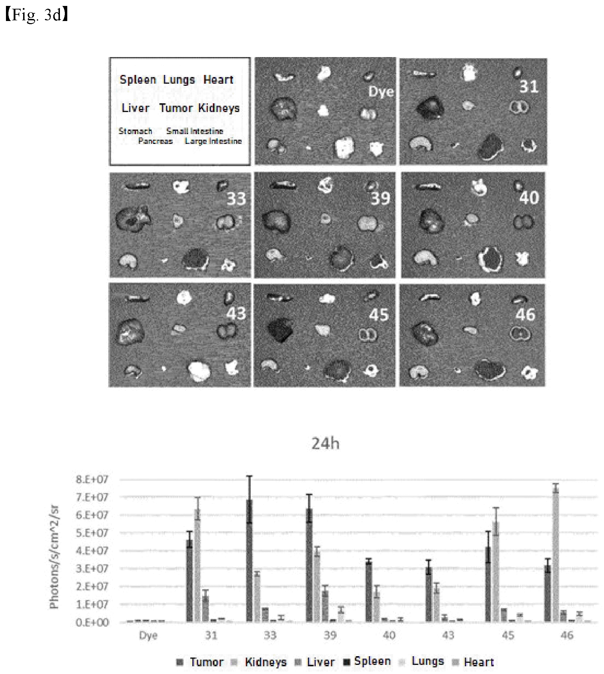

Fig. 3d shows *ex vivo* images and fluorescence intensity graphs of tumors and organs extracted 24 hours after injection in mice each injected with seven Peptide Constructs (31, 33, 39, 40, 43, 45, and 46), which exhibited high tumor signals based on the negative control (Dye) and the results of Figs. 3a to 3c.

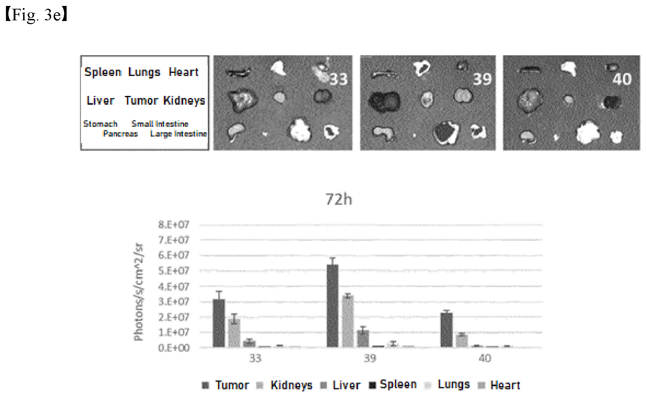

Fig. 3e shows *ex vivo* images and fluorescence intensity graphs of tumors and organs extracted 72 hours after injection in mice each injected with Peptide Constructs 33, 39, and 40.

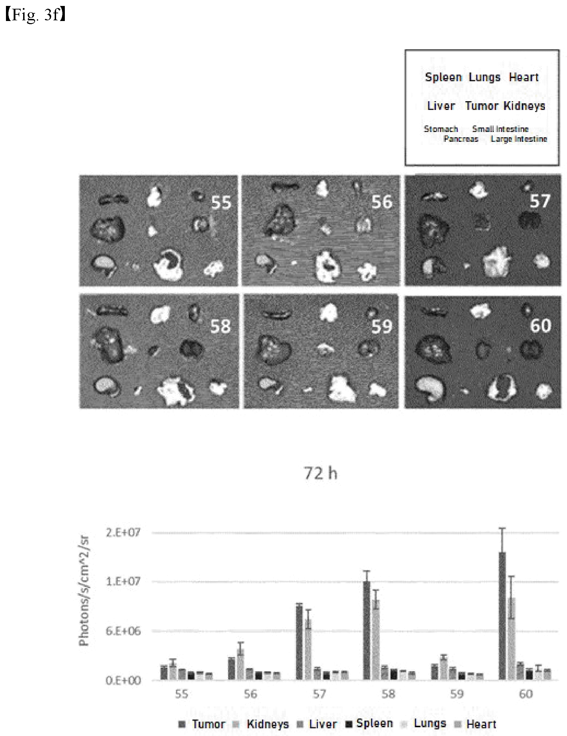

Fig. 3f shows *ex vivo* images and fluorescence intensity graphs of tumors and organs extracted 72 hours after injection in mice each injected with Peptide Constructs 55, 56, 57, 58, 59, and 60.

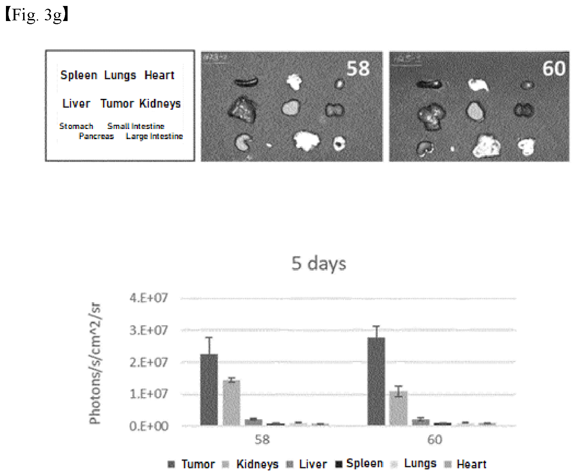

Fig. 3g shows *ex vivo* images and fluorescence intensity graphs of tumors and organs extracted 5 days after injection in mice each injected with Peptide Constructs 58 and 60.

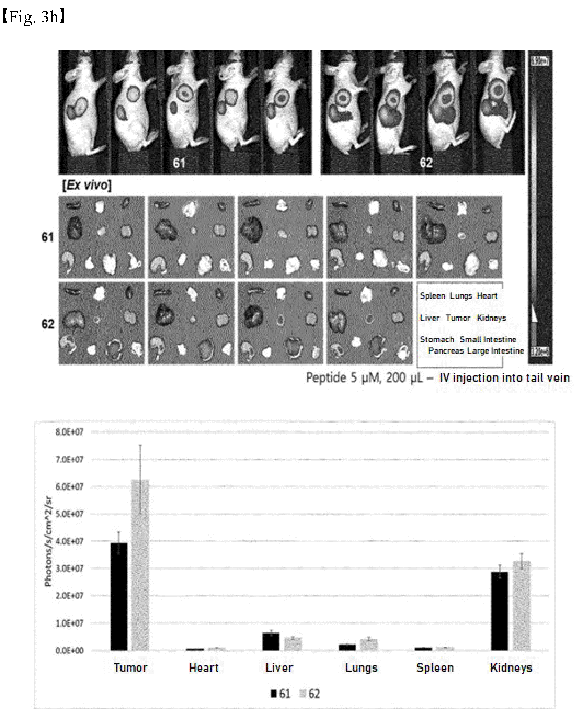

Fig. 3h shows whole-body imaging, *ex vivo* images, and fluorescence intensity graphs of tumors and organs extracted 24 hours after injection in mice each injected with Peptide Constructs 61 and 62.

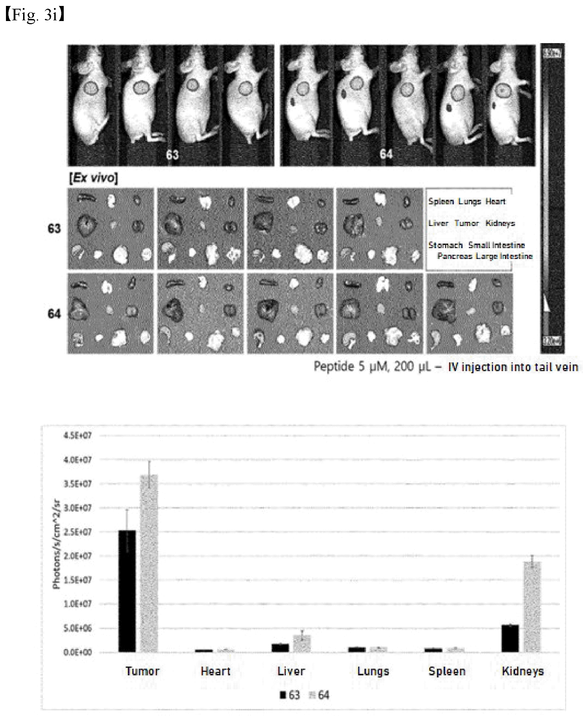

Fig. 3i shows whole-body imaging, *ex vivo* images, and fluorescence intensity graphs of tumors and organs extracted 24 hours after injection in mice each injected with Peptide Constructs 63 and 64.

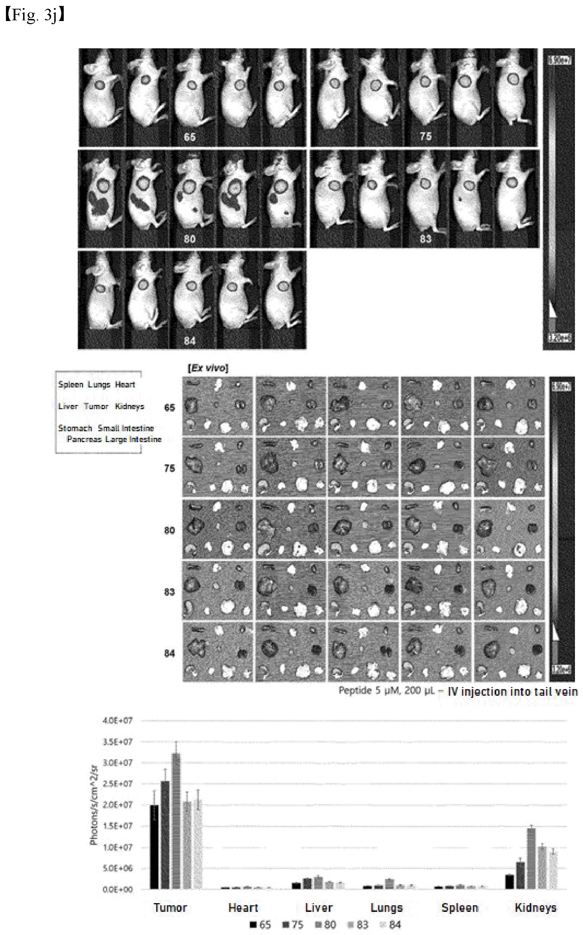

Fig. 3j shows whole-body imaging, *ex vivo* images, and fluorescence intensity graphs of tumors and organs extracted 24 hours after injection in mice each injected with Peptide Constructs 65, 75, 80, 83 and 84.

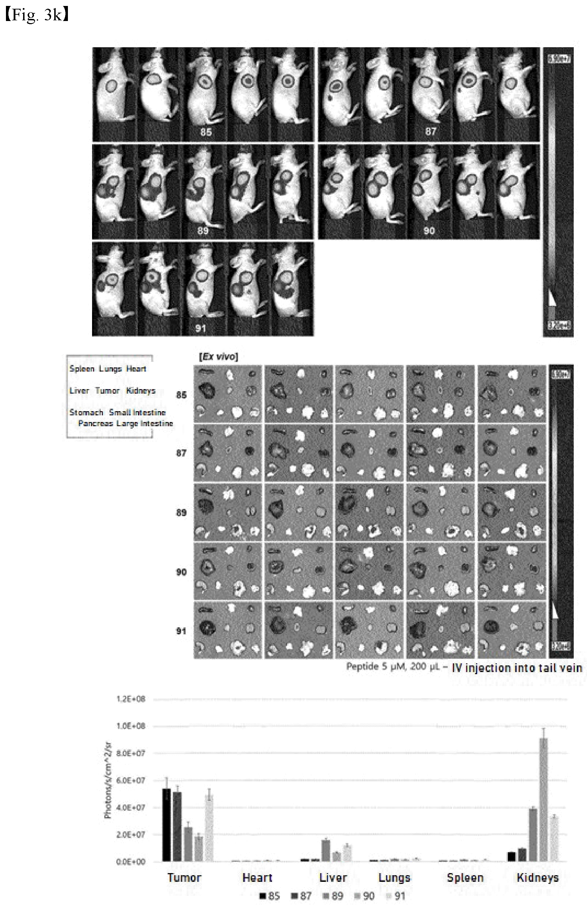

Fig. 3k shows whole-body imaging, *ex vivo* images, and fluorescence intensity graphs of tumors and organs extracted 24 hours after injection in mice each injected with Peptide Constructs 85, 87, 89, 90 and 91.

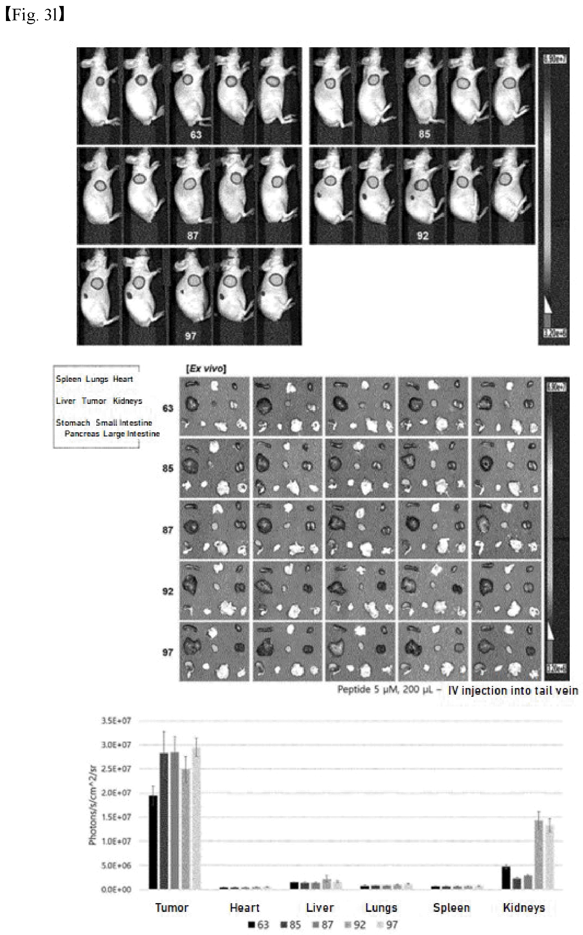

Fig. 3l shows whole-body imaging, *ex vivo* images, and fluorescence intensity graphs of tumors and organs extracted 72 hours after injection in mice each injected with Peptide Constructs 63, 85, 87, 92 and 97.

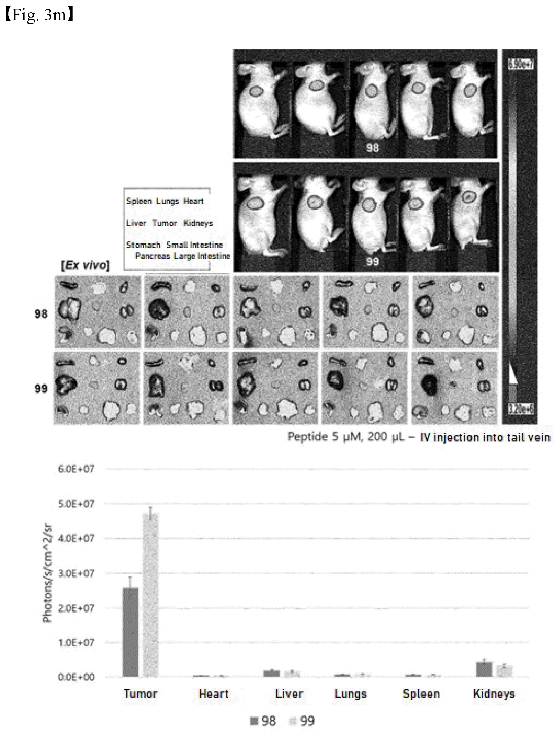

Fig. 3m shows whole-body imaging, *ex vivo* images, and fluorescence intensity graphs of tumors and organs extracted 72 hours after injection in mice each injected with Peptide Constructs 98 and 99.

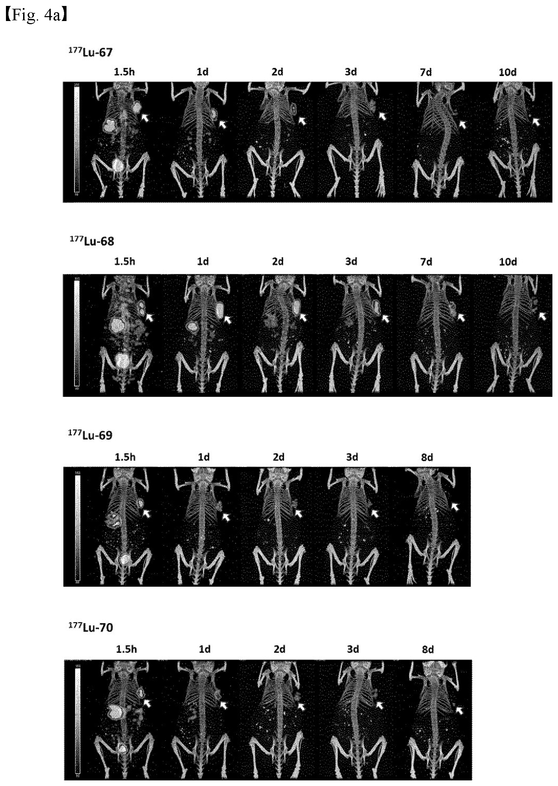

Fig. 4a shows SPECT/CT images obtained at 1.5 hours, 1 day, 2 days, 3 days, 7 days, 8 days, or 10 days after injection in mice each injected with Peptide Constructs 67, 68, 69, and 70 labeled with ¹⁷⁷Lu. Arrows indicate tumors.
Fig. 4a shows the results of SPECT/CT imaging of mice each injected with Peptide Constructs 67, 68, 69, and 70 labeled with ¹⁷⁷Lu after 1.5 hours, 1 day, 2 days, 3 days, 7 days, 8 days, or 10 days. Arrows indicate tumors.

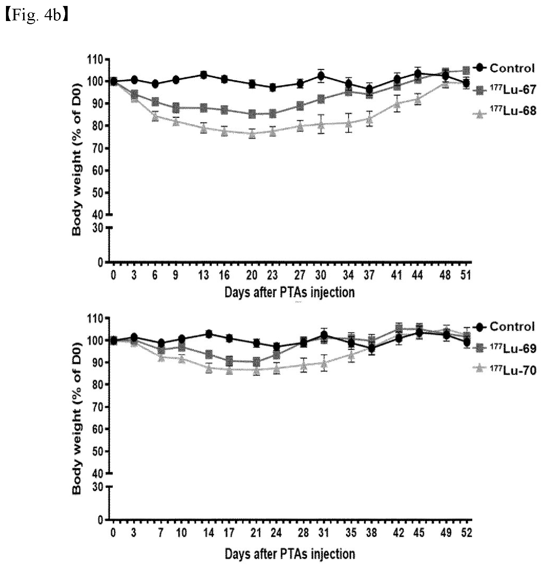

Fig. 4b shows the results of measurements of body weight changes in mice each injected with Peptide Constructs 67, 68, 69, and 70 labeled with ¹⁷⁷Lu.

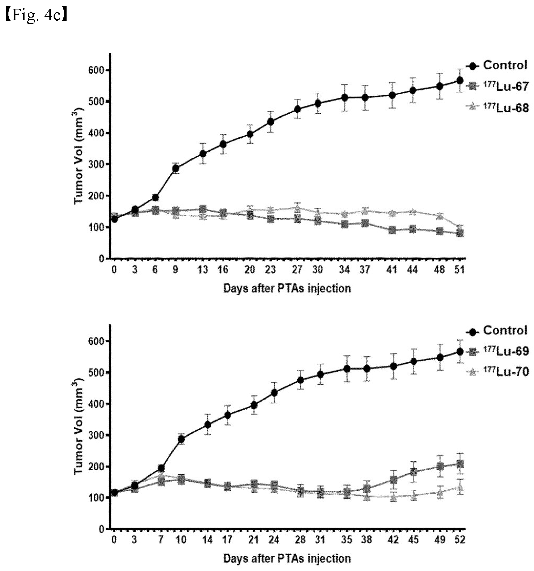

Fig. 4c shows the results of measurements of changes in tumor size (anticancer effect) in mice each injected with Peptide Constructs 67, 68, 69, and 70 labeled with ¹⁷⁷Lu.

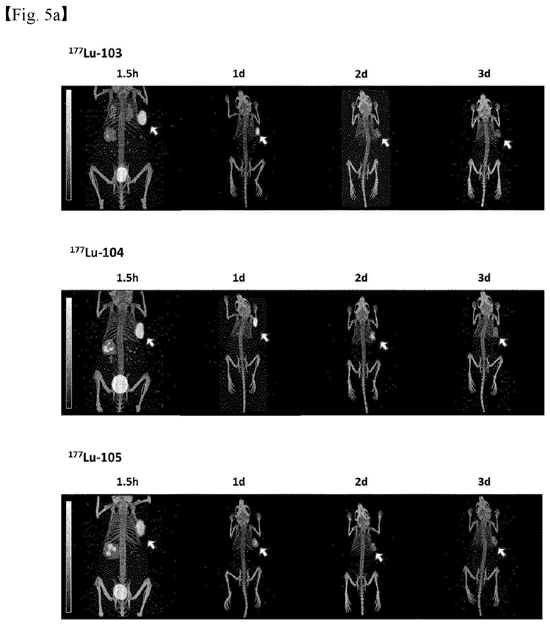

Fig. 5a shows the results of SPECT/CT imaging of mice each injected with Peptide Constructs 103, 104 and 105 labeled with ¹⁷⁷Lu after 1.5 hours, 1 day, 2 days, or 3 days. Arrows indicate tumors.

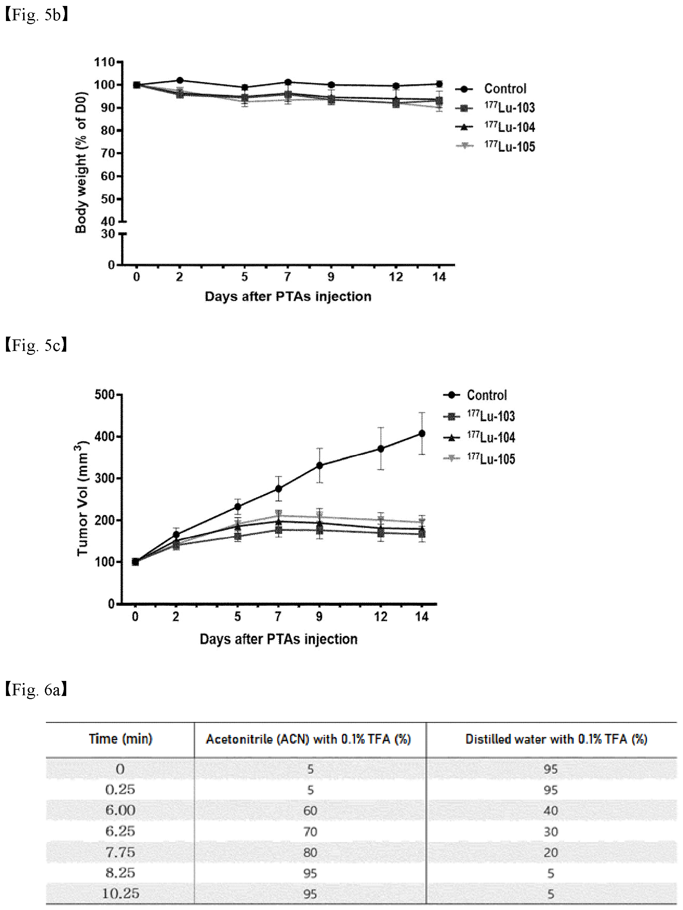

Fig. 5b shows the results of measurements of body weight changes in mice each injected with Peptide Constructs 103, 104 and 105 labeled with ¹⁷⁷Lu.
Fig. 5c shows the results of measurements of changes in tumor size (anticancer effect) in mice each injected with Peptide Constructs 103, 104 and 105 labeled with ¹⁷⁷Lu.
Fig. 6a shows the HPLC analysis conditions for the synthesized compounds.

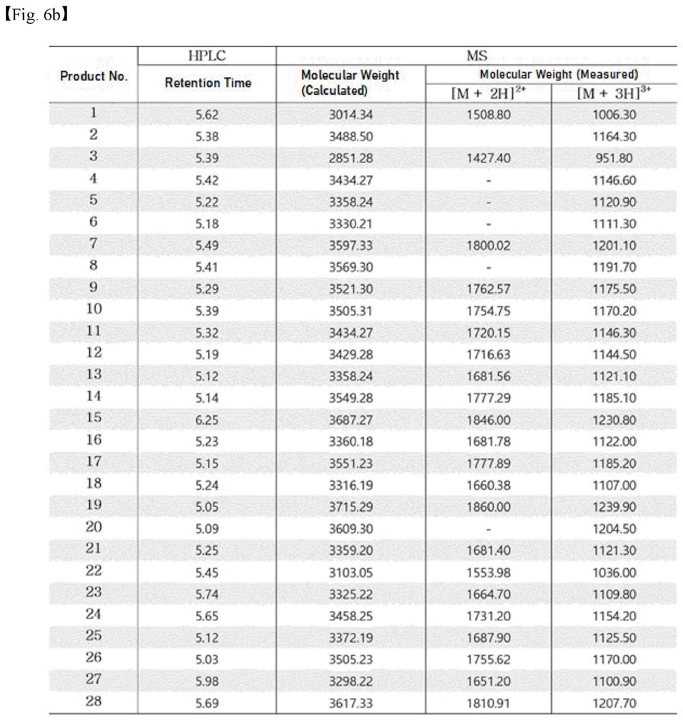

Fig. 6b shows the retention times in HPLC and the molecular weights observed using electrospray ionization mass spectrometry (MS) for synthesized compounds 1 to 28.

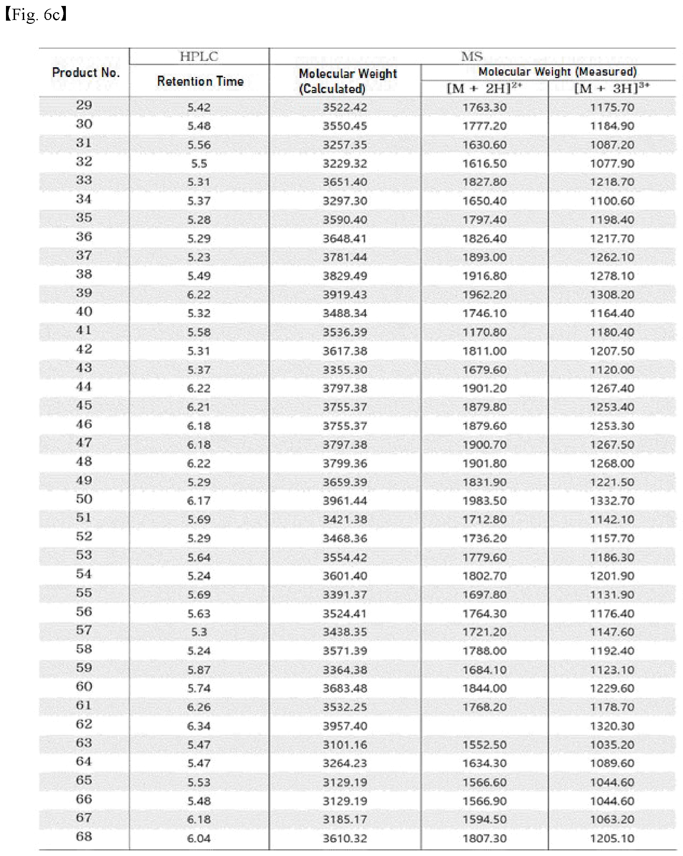

Fig. 6c shows the retention times in HPLC and the molecular weights observed using electrospray ionization mass spectrometry (MS) for synthesized compounds 29 to 68.

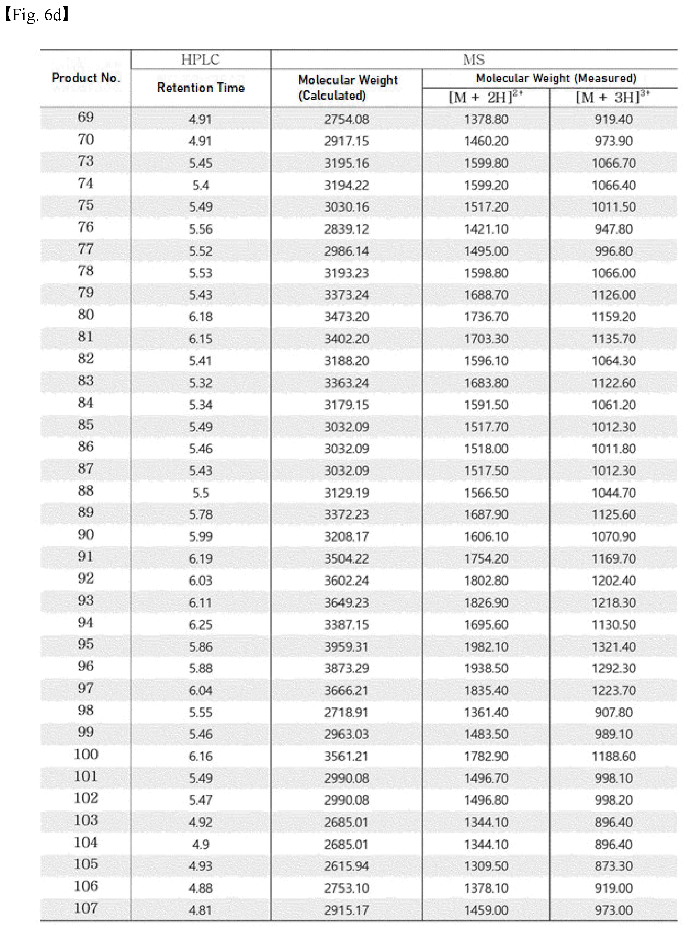

Fig. 6d shows the retention times in HPLC and the molecular weights observed using electrospray ionization mass spectrometry (MS) for synthesized compounds 69 to 107.

### BEST MODE FOR CARRYING OUT THE INVENTION

Unless defined otherwise, all technical and scientific terms used herein have the same meaning as commonly understood by one of ordinary skill in the art to which this invention belongs. Any methods and materials similar or equiv. to those described herein can be used in the practice or testing of the present invention.

The present invention is capable of various modifications and applications within the scope of the claims described below and equiv. interpreted therefrom.

### CAIX-specific peptide construct of the present invention

The present invention provides a peptide construct comprising a sulfonamide functional group-containing amino acid residue linked directly or via a spacer to a CAIX-specific peptide ligand.

The CAIX-specific peptide ligand may comprise an amino acid sequence selected from sequences 1 to 29. The peptide ligand may comprise D-amino acids or consist entirely of D-amino acids. In addition, one or more chemical functional groups, for example 4-(p-iodophenyl)butyric acid (IB) or 2-(4-iodophenyl)acetic acid (sIB) but not limited thereto, may be substituted at the ε-amino group of a lysine (Lys) residue among the constituent amino acid residues of the peptide ligand. Furthermore, one or more chemical functional groups, for example α-D-galacturonic acid (Gal) but not limited thereto, may be substituted at the β-amino group of a 2,3-diaminopropionic acid (DAP) residue among the constituent amino acid residues of the peptide ligand.

The peptide ligand may be prepared such that amino acid residues having specific sequences are linked to each other to form a part of the peptide construct. The peptide ligand may be produced by a known peptide synthesis method and is not particularly limited. In one embodiment, the peptide ligand may be produced by repeating the peptide synthesis process on a solid-phase single bead until a peptide of the desired length and sequence is completed.

The CAIX-specific peptide ligand may also comprise salt forms thereof.

In the sulfonamide functional group-containing amino acid residues of the peptide construct, the introduction of the sulfonamide functional group may be carried out by a known synthetic reaction and is not particularly limited. In one embodiment of the present invention, the sulfonamide functional group may be introduced into the peptide construct of the present invention via a click chemical reaction. Examples of preferred sulfonamide functional group-containing amino acid residues that may be introduced into the peptide construct of the present invention comprise the following structures, but are not limited thereto:

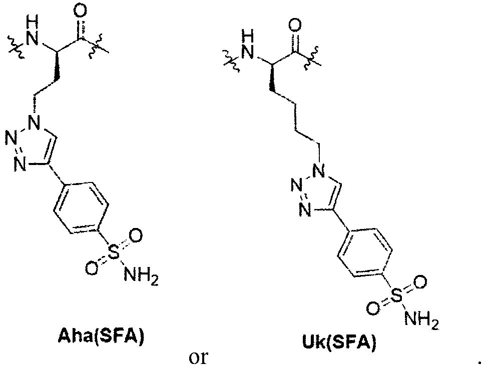

The peptide construct may further comprise one or more functional group-containing amino acid residues other than the sulfonamide functional group. The functional group other than the sulfonamide may be introduced via the side chains of the amino acid residues. The amino acid residue into which the functional groups is introduced may preferably be lysine or 2,3-diaminopropionic acid (DAP).

In one embodiment, non-limiting examples of functional groups other than the sulfonamide that may be introduced into the peptide construct of the present invention may include chelators, biotin, glucoheptonic acid, 4-(p-iodophenyl)butyric acid (IB), 2-(4-iodophenyl)acetic acid (sIB), zwitterionic ion(SZW), α-D-galacturonic acid(Gal), cycloalkane having a carbon number of 5 to 15, fluorescent dyes, or cytotoxic agents.

For example, the chelator may be selected from one or more of 1,4,7,10-tetraazacyclododecane-1,4,7,10-tetraacetic acid (DOTA), 1,4,7-triazacyclononane-1,4,7-triacetic acid (NOTA), ethylenediaminetetraacetic acid-2,2',2",2"'-(ethane-1,2-diyldinitrilo)tetraacetic acid (EDTA), 1,4,7,10,13,16-hexaazacyclooctadecane-N,N',N",N‴,Nʺʺ,N‴ʺ-hexaacetic acid (HEHA), 2-[4-nitrobenzyl]-1,4,7,10,13-pentaazacyclopentadecane-N,N',N",N‴,Nʺʺ-pentaacetic acid (PEPA), 1,4,7,10-tetraazacyclododecane-1,4,7,10-tetra(methylenephosphonic acid) (DOTP), (1R,4R,7R,10R)-α,α',α",α‴-tetramethyl-1,4,7,10-tetraazacyclododecane-1,4,7,10-tetraacetic acid tetrasodium salt (DOTMA), 2-[bis[2-[bis(carboxymethyl)amino]ethyl]amino]acetic acid (DTPA), triethylenetetramine (TETA), 1,4,7,10-tetraazacyclododecane-7-acetamide-1,4,10-triacetic acid (Pb specific chelator, PSC), and 2-[4,7,10-tris(2-amino-2-oxoethyl)-1,4,7,10-tetraazacyclododec-1-yl]acetamide (DOTAM or TCMC), but is not limited thereto.

The cycloalkane having 5 to 15 carbon atoms may be selected from one or more of cyclopentane, cyclohexane, cycloheptane, cyclooctane, cyclononane, cyclodecane, adamantane, norbornane, isobornane, and tricyclodecane, but is not limited thereto.

The peptide construct of the present invention may also link an amino acid residue containing a chemical functional group, such as a sulfonamide functional group, to a CAIX-specific binding peptide ligand via a spacer. The spacer may be selected from at least one of polyethylene glycol (PEG) linker, glycine, sarcosine, γ-carboxyglutamic acid, and a peptide linker consisting of 1 to 8 D-amino acids or L-amino acids but is not limited thereto.

The peptide construct of the present invention has the construct of the following Formula 1: 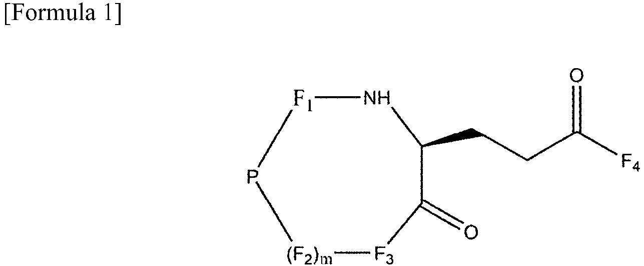 wherein P, F₁, F₂, F₃, F₄ and n are as defined above.

In one embodiment of the present invention, the peptide construct has the construct of the following Formula 2:

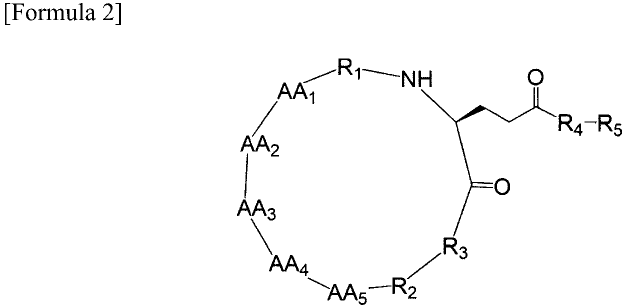

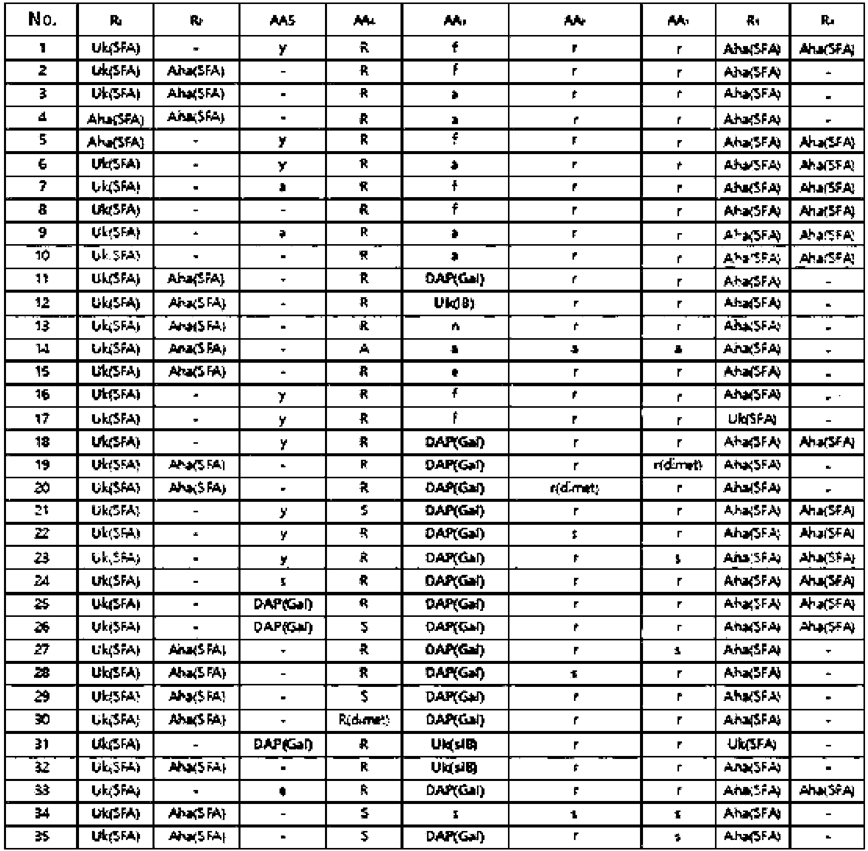

wherein R₅ is a group of the general formula -(S₁)ₚ-(F₆)_{q}-(S₂)ᵣ-(F₇)ₛ-(S₃),
S₁, S₂, S₃, F₆, F₇, p, q, r, and s are each as defined above,
the lowercase letters represent D-amino acids, the uppercase letters represent L-amino acids, and
each of the other substituents indicated by abbreviations at R₁ to R₄ and AA₁ to AA₅ is defined as described below:

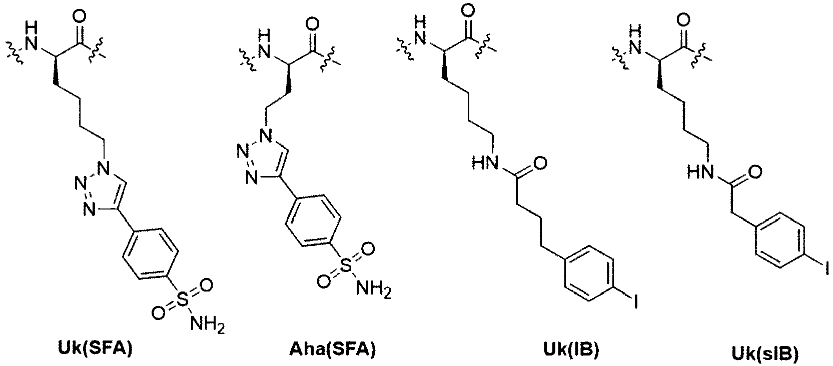

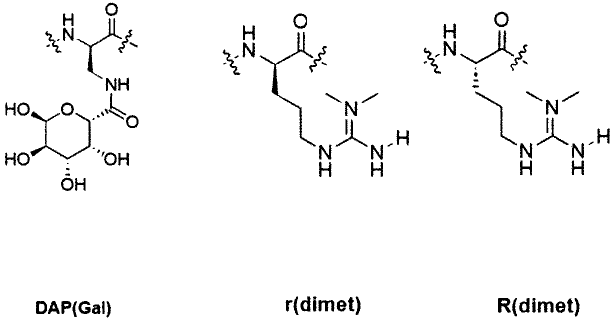

Although not bound by a particular theory, the cyclic construct, such as the cyclic peptide construct of the present invention, has less flexibility than the linear peptide. Thus, it is considered that cyclic construct has a lower entropy loss during target binding and has higher binding affinity with increased binding specificity to the target.

The peptide construct of the present invention exhibits high selectivity, in that it specifically binds to the CAIX but does not bind to other isoenzymes of the CAIX (e.g., carbonic anhydrase XII).

### Conjugate

The CAIX-specific peptide construct of the present invention may form a conjugate by being linked directly or via a linker to fluorescent dyes, cytotoxic agents, or radioactive isotopes. The conjugate can label the CAIX-expressing cancer with fluorescent dyes or radioactive isotopes during targeting the CAIX or effectively deliver drugs such as radioactive isotopes or cytotoxic agents to the cancer, being useful for diagnosis, prevention or treatment of cancer.

In one embodiment, the linker may be one or more selected from 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio)pentanoate (SPP), N-succinimidyl 4-(N-maleimidomethyl)cyclohexane-1 carboxylate (SMCC), 4-(2-pyridyldithio)butyric acid-N-hydroxysuccinimide ester (SPDB), and N-succinimidyl (4-iodoacetyl)aminobenzoate (SIAB), but is not limited thereto.

In one embodiment, non-limiting examples of the fluorescent dye include one or more selected from near-infrared fluorescent dye, fluorescein type, rhodamine type, Alexa Fluor, 4,4-difluoro-4-boro-3a, 4a-diaza-S-indacene (BODIPY), Texas Red, dansyl, Lissamine, cyanine (Cy), and phycoerythrin.

In one embodiment, the cytotoxic agent may be one or more selected from toxin, chemotherapeutic agent, drug moiety, antibiotic, and nuclease, but is not limited thereto.

In one embodiment, the radioisotope may be one or more selected from Fluorine-18 (F-18), Carbon-11 (C-11), Carbon-14 (C-14), Techthenium-99m (Tc-99m), Copper -64 (Cu-64), Copper-67 (Cu-67), Dysprosium-168 (Dy-168), Bismuth-213 (Bi-213), Samarium-153 (Sm-153), Strontium-89 (St- 89), Strontium-90 (St-90), Erbium-169 (Er-169), Phosphorus-32 (P-32), Palladium-103 (Pd-103), Rhenium-186 (Re-186), Rhenium -188 (Re-188), Oxygen-15 (O-15), Selenium-75 (Se-75), Sodium-24 (Na-24), Strontium-85 (Sr-85), Lutetium-177 (Lu-177), Yttrium-90 (Y-90), Iodine-123 (I-123), Iodine-125 (I-125), Iodine-131 (I-131), Iridium-192 (Ir-192 ), Iridium-196 (Ir-196), Ytterbium-166 (Yb-166), Indium-111 (In-111), Xenon-133 (Xe-133), Nitrogen-13 (N-13), Calcium -47 (Ca-47), Cobalt-57 (Co-57), Cobalt-60 (Co-60), Chromium-51 (Cr-51), Krypton-81 (Kr-81), Potassium-42 (K-42) 42), Holmium-166 (Ho-166), Gallium-67 (Ga-67), Gallium-68 (Ga-68), Actinium-225 (Ac-225), Zirconium-89 (Zr-89), Thorium-227 (Th-227), Radium-223 (Ra-223), Tin-117m (Sn-117m), Lead-103 (Pb-103), Terbium-161 (Tb-161), Lead- 212 (Pb-212), and Astatine-211 (At-211).

### Therapeutic Administration and Formulation

The present invention provides a pharmaceutical composition for diagnosis, prevention or treatment of cancer comprising the CAIX-specific peptide construct or the conjugate. The cancer of the present invention is preferably solid cancer. More preferably, the cancer of the present invention is the cancer expressing CAIX. For example, the cancer of the present invention may be solid cancer such as liver cancer, lung cancer, colorectal cancer, stomach cancer, breast cancer, colon carcinoma, bone cancer, pancreatic cancer, head and neck cancer, uterine cancer, ovarian cancer, rectal cancer, esophageal cancer, small intestine cancer, perianal cancer, fallopian tube carcinoma, endometrial carcinoma, cervical carcinoma, vaginal carcinoma, vulvar carcinoma, prostate cancer, biliary cancer, bladder cancer, kidney cancer, ureteric cancer, renal cell carcinoma, renal pelvic carcinoma, melanoma, thyroid cancer, astrocytoma and glioblastoma, but is not limited to thereto.

A subject to whom the pharmaceutical composition for diagnosis, prevention or treatment of cancer of the present invention is administered may be a mammal at risk of developing cancer, which is diagnosed with cancer, or that is treated for cancer. The mammal may be a human or a non-human mammal.

The pharmaceutical composition for diagnosis, prevention, or treatment of cancer according to the present invention can be formulated and used according to conventional methods in the form of an oral formulation, such as powder, granule, tablet, capsule, suspension, emulsion, syrup and aerosol, external preparation, suppository, and sterile injection solution, respectively. For formulation, suitable carriers, excipients or diluents commonly used in the preparation of pharmaceutical compositions may be included.

Examples of the carriers, excipients, or diluents may be various compounds or mixtures including lactose, dextrose, sucrose, sorbitol, mannitol, xylitol, erythritol, maltitol, starch, gum acacia, alginate, gelatin, calcium phosphate, calcium silicate, cellulose, methyl cellulose, and microcrystalline cellulose, polyvinyl pyrrolidone, water, methylhydroxybenzoate, propylhydroxybenzoate, talc, magnesium stearate and mineral oil, and the like.

Formulation of the pharmaceutical composition may be prepared using diluents or excipients such as fillers, weighting agents, binders, wetting agents, disintegrants, and surfactants commonly used in the pharmaceutical industry.

The preferred dosage of the pharmaceutical composition for preventing or treating cancer according to the present invention varies depending on the patient's condition, body weight, disease severity, drug form, administration route and duration, which can be appropriately selected by those skilled in the art. However, for desirable effects, it can be administered at 0.0001 to 2,000 mg/kg per day, preferably 0.001 to 2,000 mg/kg. The administration may be done once a day or divided into several times. However, the scope of the present invention is not limited by the dosage.

The pharmaceutical composition for preventing or treating cancer according to the present invention can be administered to mammals such as rats, mice, livestock, and humans through various routes. The method of administration may be, for example, oral, rectal or intravenous, intramuscular, subcutaneous, intrauterine intrathecal or intracerebroventricular injection.

The present invention also provides the method for treating cancer, comprising administering the CAIX-specific peptide construct or the conjugate of the present invention to a subject in need of such treatment.

The CAIX-specific peptide construct and the conjugate of the present invention can be also used for diagnosing cancer by targeting and imaging cancer or can be used for predicting or observing the treatment prognosis of the subject after cancer treatment by administering to a subject who has undergone cancer treatment.

Hereinafter, the present invention will be described in more detail through embodiments. These embodiments are only intended to more specifically illustrate the present invention and are not provided to intend to limit the legitimate scope of the present invention. It will be apparent to those skilled in the art that various modifications are possible within the scope of the present invention.

### Examples

### Example 1. Synthesis and Purification of Peptide Constructs Comprising Selected Peptides

### 1-1. Synthesis of Reagents Inserted into Peptide Constructs

N-succinimidyl 2, 3:4,5:6,7-tri-O-isopropylidene-D-glycero-D-gulo-heptonate (6OH(Protected)-NHS) (**S1**) and Fmoc-DAP(DOTA(Protected))-OH (**S2**), Fmoc-DAP (Gal(Protected))-OH (**S3**), 3-(dimethylamino)propane-1-sulfonic acid (**S4**), and Fmoc-D-ADMA(pbf)-OH (Fmoc-r(dimet)-OH) (**S5**), inserted into the peptide construct were synthesized in-house and used. First, the synthesis of **S1** was carried out by adding methyl 2,3:4,5:6,7-tri-O-isopropylidene-D-glycero-D-gulo-heptonate (1 equiv.) and lithium hydroxide monohydrate (2. 5 equiv.) into a 50 mL round-bottom flask containing a magnetic stirrer bar, then MeOH was added and the reaction was carried out at room temperature for 24 hours. After completion of the reaction, the volatiles and solvents from the reactants were removed using an evaporator. The reaction mixture was dissolved in a 10% aqueous citric acid solution, and transferred to a separatory funnel, and then the organics were extracted with ethyl acetate. The extracted ethyl acetate solution was washed with a saturated aqueous solution of NaCl in a separatory funnel and then dried over sodium sulfate in a triangular flask. Then, the sodium sulfate was removed from the ethyl acetate solution with filter paper and the volatile solvent was removed using the evaporator. The reaction product was dissolved with dichloromethane (DCM) once again, and the volatile solvent was removed using the evaporator.

For the following reaction, the synthesized 2,3:4,5:6,7-tri-O-isopropylidene-D-glycero-D-gulo-heptonic acid (1 equiv.) was added to a 50 mL round-bottom flask containing a magnetic stirrer bar together with N-hydroxysuccinimide (NHS) and N-(3-Dimethylaminopropyl)-N'-ethylcarbodiimide hydrochloride (EDC hydrochloride, 1.6 equiv.), and then DCM anhydrous was added and the reaction was carried out at room temperature for 24 hours. After completion of the reaction, the reaction mixture was dissolved in ethyl acetate, transferred to a separatory funnel, and washed with a saturated aqueous solution of NaCl. The organic layer was washed with sodium bicarbonate saturated aqueous solution, followed by NaCl saturated aqueous solution. The organic layer was washed with a saturated aqueous solution of sodium bicarbonate, followed by a wash with a saturated aqueous solution of NaCl. The organic layer was collected in a conical flask and dried over anhydrous sodium sulfate. The sodium sulfate was then removed by filtration, and the volatile solvent was removed using the evaporator. The reaction product was dissolved again in DCM, and the volatile solvent was completely removed using the evaporator before use. An example of the reaction for synthesizing the **S1** moiety as described above is as follows.

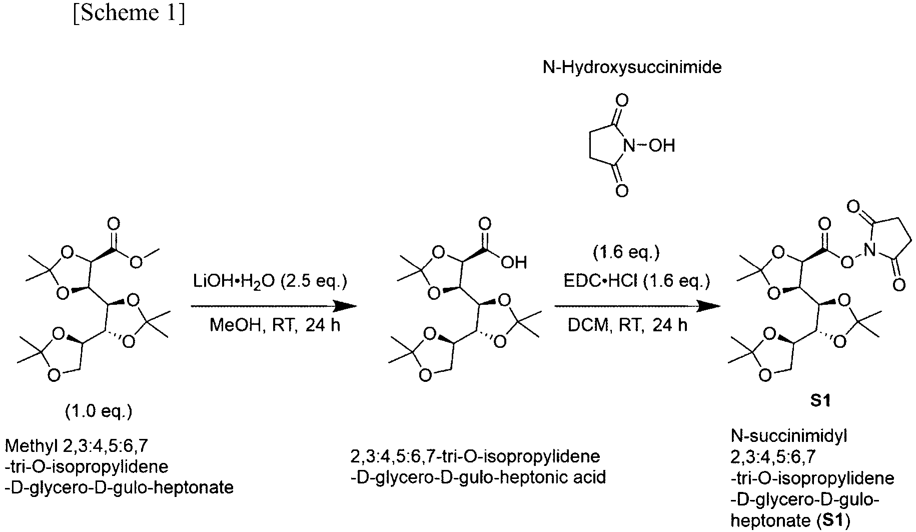

The synthesis of Fmoc-DAP (DOTA (Protected))-OH (**S2**) and Fmoc-DAP (Gal (Protected))-OH (**S3**) was carried out by first dissolving unprotected Fmoc-DAP-OH (1 equiv.) in N,N-dimethylformamide (DMF). tri-tert-butyl 1, 4,7,10-tetraazacyclododecane-1,4,7,10-tetraacetate, DOTA-tris (tert-butyl ester) or 1,2:3,4-di-O-isopropylidene-a-D-galacturonide (1.5 equiv.) was dissolved in DMF together with 2-(1H-Benzotriazole-1-yl)-1,1,3,3-tetramethylaminium tetrafluoroborate (TBTU, 1.2 equiv.) and N,N-Diisopropylethylamine (DIPEA, 3 equiv.) to give an activated solution, which was then mixed and reacted for 2 hours. The progress of the reaction was then monitored by LC-MS (1260 Infinity II, Infinity Lab LC/MSD, Agilent), and the product was purified by a 1260 Infinity II LC system (Agilent). LC-MS (1260 Infinity II, Infinity Lab LC/MSD, Agilent) was used for analysis, and the data were analyzed using MNOVA (v. 14.2.0, Mestrelab Research, Spain). An Agilent Poroshell 120 EC-C18 column (4.6 × 50 mm, 2.7 µm) was used as the stationary phase, and the column temperature was maintained at 40 °C. The mobile phase was a mixture of acetonitrile (ACN) containing 0.1% trifluoroacetic acid (TFA) and triple-distilled water containing 0.1% TFA, with wavelengths of 214 and 254 nm and a flow rate of 1 mL/min. The purification was performed on a 1260 Infinity II LC system (Agilent) under the following conditions: (1) the stationary phase, Kromasil 100-5-C18 column (21.2 × 250 mm, 5 µm); (2) the mobile phase, a mixture of triple-distilled water with 0.1% TFA and ACN; (3) the flow rate, 15 mL/min; and (4) the detection wavelengths, 214 and 254 nm.

The synthesis procedures of **S2** and **S3** are as follows.

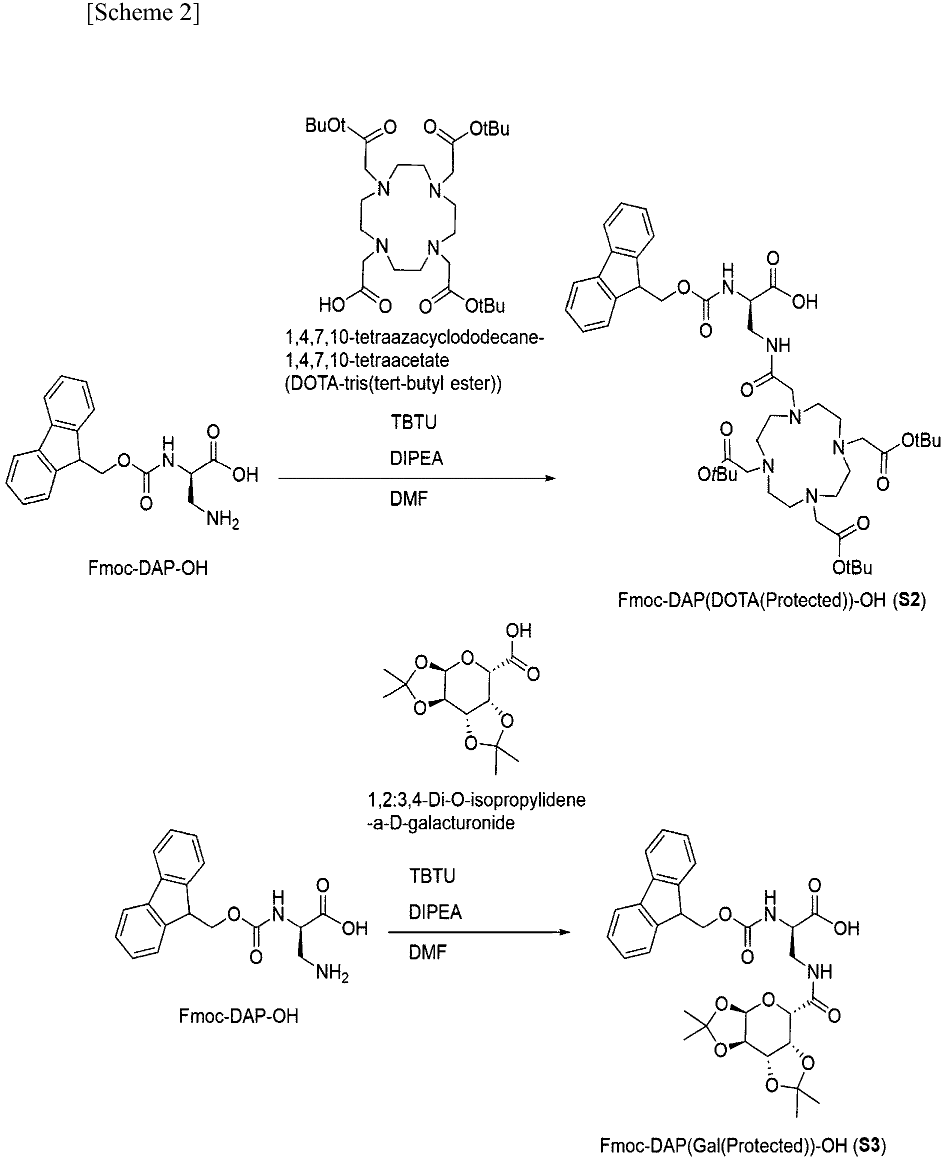

For the synthesis of **S4,** 1g of 1,3-Propanesultone (Cat# P0324, TCI) and 16 mL of 2 M dimethylamine in THF solution (Cat# D3948, TCI) were added to 100 mL of a round-bottom flask and stirred at room temperature for 24 hours. The reaction mixture solution was concentrated using an evaporator, and the resulting gel-like material was first precipitated with ethyl acetate, and the precipitate was separated using a nylon membrane filter. The isolated precipitate was dissolved in a minimal amount of water, and DMF was added to recrystallize the material. The recrystallized material was separated using a nylon membrane filter, washed with ethyl acetate and dried. An example synthesis of **S4** is as follows.

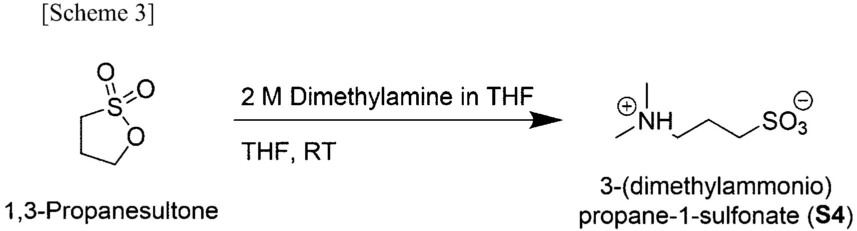

The synthesis process of **S5** is as follows.

Fmoc-D-Orn(boc)-OH (1.0 equiv.., Cat# BD131117, BLD Pharmatech), N,N-dicyclohexylcarbodiimide (DCC, 1.5 equiv., Cat# 36650, Sigma-Aldrich), 1-hydroxybenzotriazole (HOBt, 1.5 equiv., Cat# CXZ010, Aapptec), 4-(dimethylamino)pyridine (DMAP, 0.05 equiv., Cat# 107700, Sigma-Aldrich), and DCM anhydrous were added to a round-bottom flask and stirred thoroughly at room temperature for 3 hours. After sufficient precipitation was monitored, allyl alcohol (2.0 equiv., Cat# 240532, Sigma-Aldrich) was added, and the reaction was carried out at room temperature for 16 hours. After completion, most of the precipitate was removed by filtration through a paper filter, and the filtrate was concentrated using an evaporator. The resulting gel-like material was dissolved in a minimal amount of ethyl acetate, and stored at 20 °C for 16 hours to induce precipitation. The precipitate formed in the ethyl acetate solution was filtered through a paper filter and transferred to a separatory funnel, to which 100 mL of ethyl acetate was added. The organic layer was washed using a separatory funnel once with saturated aqueous sodium bicarbonate solution (Cat# S0341, Samjeon Pure Chemical Industry Co., Ltd.), twice with distilled water, and once with saturated aqueous sodium chloride solution (Cat# S0476, Samjeon Pure Chemical Industry Co., Ltd.). The washed organic layer was dried over anhydrous magnesium sulfate (Cat# M1806, Samchun Pure Chemical Co., Ltd.), and the precipitate was filtered through a paper filter, and the filtrate was concentrated using the evaporator. The concentrated gel-like material was completely dissolved in a minimal amount of DCM, and half of the solution was purified by column chromatography (40 × 210 mm) containing silica gel 200 g (0.063-0.200 mm, Cat# 1.07734.1000, Sigma-Aldrich), using ethyl acetate: hexane (Cat# 1.07734.1000, Sigma Aldrich) = 1 : 1 as the eluent. The remaining DCM solution was purified in the same manner on a silica gel column. The reaction product, Fmoc-D-Orn(boc)-OAll, exhibited an Rf value of 0.59 (ethyl acetate/hexane = 1:1). The fractions corresponding to this Rf were collected and concentrated using the evaporator to afford Fmoc-D-Orn(boc)-OAll.

In the subsequent reaction, Fmoc-D-Orn(Boc)-OAll (1 equiv.) was added to a round-bottom flask, and a 1:1 mixture of TFA and DCM was slowly added at room temperature. The mixture was stirred for 1 hour. After the reaction, the solution was concentrated using an evaporator. The concentrated material was dissolved in DCM and concentrated again using an evaporator. This procedure was repeated 3 times. The concentrated material was then dissolved in DCM anhydrous (Cat# 270997, Sigma Aldrich), sealed, and stored in a freezer. The concentrated material was confirmed for molecular weight and purity by LC-MS as in Example 1-1 above, and was subsequently used in the following reactions without further purification.

2,2,4,6,7-pentamethyl-2,3-dihydrobenzofuran-5-sulfonyl isothiocyanate (pbf-NCS), required for the following reaction, was first synthesized as follows.

tetrabutylammonium thiocyanate (Bu₄NNCS, 5 equiv., Cat# T1278, TCI) and 2,2,4,6,7-pentamethyl-2,3-dihydrobenzofuran-5-sulfonyl chloride (pbf-Cl, 1 equiv., Cat# BD41320, BLD Pharmatech) were added to a round-bottom flask containing anhydrous DCM. After attaching a reflux condenser, the reaction mixture was refluxed at 75 °C for 1 hour. The reaction product, pbf-NCS [Rf = 0.49 (hexane: DCM = 1 : 1)], was confirmed by TLC. After completion of the reaction, the concentrated gel-like material was completely dissolved in a minimal amount of DCM and purified by column chromatography (40 × 210 mm) containing silica gel (200 g, 0.063-0.200 mm), using ethyl acetate: hexane = 1 : 1 as the eluent. The purified solution with Rf = 0.49 was collected and concentrated using an evaporator to obtain pbf-NCS as a clear liquid. The reaction procedure was as follows.

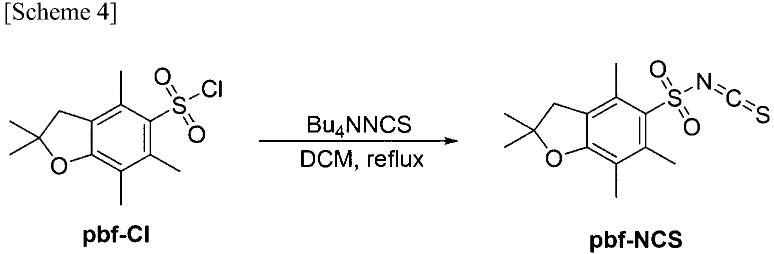

In the subsequent reaction, Fmoc-D-Orn-OAll (1 equiv.), the previously synthesized compound, pbf-NCS (1.2 equiv.), and DIPEA (10 equiv.) were dissolved in DCM in a round-bottom flask and the reaction was carried out at room temperature for 1 hour. After the reaction, the mixture was concentrated using an evaporator, and the resulting material was completely dissolved in a minimal amount of DCM and purified by column chromatography (40 × 210 mm) containing silica gel (200 g, 0.063-0.200 mm). Ethyl acetate: hexane = 1 : 1 was used as the eluent. The reaction product, Fmoc-D-Orn(SCN(pbf))-OAll, showed Rf = 0.62 (ethyl acetate: hexane = 1 : 1), and the purified solution corresponding to this Rf were collected and concentrated using an evaporator for use in the subsequent reaction.

A round-bottom flask was charged with the synthesized Fmoc-D-Orn(SCN(pbf))-OAll (1 equiv.) and 1-(3-dimethylaminopropyl)-3-ethylcarbodiimide hydrochloride (EDCl, 2 equiv., Cat# E7750, Sigma-Aldrich) dissolved in DCM, to which 2 M dimethylamine in THF (2 equiv., Cat# 391956, Sigma-Aldrich) was added. The mixture was reacted at room temperature for 10 minutes. The reaction mixture was confirmed for molecular weight and purity by LC-MS as in Example 1-1, and after completion of the reaction, it was concentrated using an evaporator. The concentrated material was dissolved in a mixture of CAN / triple-distilled water (1:1), filtered through a 45 µm syringe filter (Cat# DISMIC-3HP, Advantec), and was purified by HPLC as in Example 1-1. The purified compound, Fmoc-D-ADMA(pbf)-OAll, was obtained as a powder by lyophilization.

Finally, under Ar, the synthesized Fmoc-D-ADMA(pbf)-OAll (1 equiv.) in a glass vial, Pd(PPh₃)₄ (0.05 equiv), and N-methyl aniline (3 equiv, Cat# M29304, Sigma-Aldrich) were dissolved in anhydrous THF and reacted at room temperature for 1 hour. The reaction mixture was confirmed for molecular weight and purity by LC-MS and was subsequently concentrated using an evaporator. The concentrated material was dissolved in a mixture of ACN triple-distilled water (1:1), filtered through a 45 µm syringe filter, and was subsequently purified by HPLC under the same conditions as described above. The purified compound, Fmoc-D-ADMA(pbf)-OH (N-α-Fmoc-N,N-ω-dimethyl-N-ωÆ-(2,2,4,6,7-pentamethyldihydrobenzofuran-5-sulfonyl)-D-arginine, Fmoc-r(dimet)-OH, **S5**), was obtained as a powder by lyophilization, The overall synthetic procedure of **S5** was as follows.

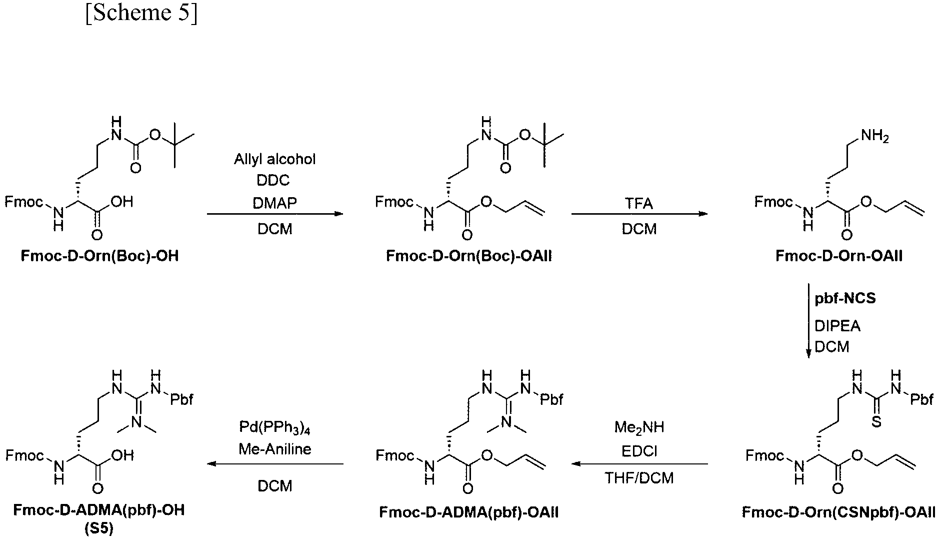

### 1-2. Synthesis of Solid-phase Peptides

Solid-phase peptide chains were synthesized using either an automated microwave peptide synthesizer (Liberty Blue^{™}, CEM Corporation) or an automated peptide synthesizer (Apex 396, AAPPTEC). Two types of solid-phase resins were used, resulting in differences at the C-terminus of the synthesized peptides. Rink Amide-ChemMatrix^{®} Resin (0.41 mmol/g, Cat# 7-600-1310, Biotage), which was used for most compounds, provides a C-terminal - CONH₂, whereas 2-Chlorotrityl Chloride Resin provides a C-terminal -COOH. Rink Amide-ChemMatrix^{®} Resin was ready to use for synthesis, whereas 2-Chlorotrityl Chloride Resin (1.07 mmol/g, Cat# GLS210819-48101, BL BioChem) was first loaded with the initial amino acid (5 equiv.) and DIPEA (10 equiv.) in DCM under an Ar atmosphere by reacting for 12 hours, and then used for synthesis. In an automated microwave peptide synthesizer, the resin swollen in N-methyl-2-pyrrolidone (NMP) was treated with Fmoc-amino acid (5 equiv.), Oxyma Pure (5 equiv., Cat# cxz021, AApptec), and diisopropylcarbodiimide (DIC, Cat# D0254, TCI) (10 equiv.), followed by coupling at 75 °C for 5 minutes in NMP. The resin was then washed 3 times with NMP, and the Fmoc protecting group was removed by treating with a piperidine / NMP solution (v / v = 1 : 4, 0.1 M Oxyma Pure) at 90 °C for 1 minute. The coupling of Fmoc-amino acids was repeated in the same manner to elongate the peptide chain, and the resin beads were collected from the synthesizer. In an automated synthesizer (Apex 396, AAPPTEC), the resin swollen in NMP was treated with Fmoc-amino acid (5 equiv.), TBTU (5 equiv.), and DIPEA (10 equiv.) in NMP, followed by coupling at room temperature for 45 minutes. The resin was then washed with NMP and DCM, and the Fmoc protecting group was removed by treating with a piperidine / NMP solution (v / v = 1 : 4) at room temperature for 10 minutes. The coupling of Fmoc-amino acids was repeated in the same manner, and the resin beads were collected from the synthesizer. When the compounds synthesized in Example 1-1 were used in solid-phase peptide synthesis, the synthesis was carried out manually instead of using an automated synthesizer, and the equivalents of reagents employed were reduced to 2 equivalents. In particular, for the synthesis of cyclic peptides, Fmoc-E(OAll)-OH (Cat#36613, GL Biochem) was first introduced, followed by the cyclization reaction.

### 1-3. Synthesis of Solid-Phase Peptide Constructs and Functional Groups

### 1-3-1. Cyclization Reaction

The allyl protecting group of the glutamic acid residue on the solid-phase beads synthesized using the automated synthesizer was removed. Tetrakis(triphenylphosphine)palladium(0) (Pd(PPh₃)₄, 1 equiv., Cat# 216666, Sigma-Aldrich) and PhSiH₃ (30 equiv., Cat# 335150, Sigma-Aldrich) were dissolved in DCM under Ar atmosphere and placed in a cylinder equipped with a filter containing the solid-phase beads. The cylinder was sealed with a lid and suspended on a 180° shaker at room temperature for 1 hour. The beads were then washed 3 times with NMP, followed by at least five washes with a 0.1 M sodium diethyldithiocarbamate solution (Cat# D3506, Sigma-Aldrich) in NMP to remove Pd impurities. The beads were then washed 3 times with NMP, and the N-terminal Fmoc protecting group was removed by adding 3 mL of a piperidine / NMP solution (v / v = 1 : 4). The beads were suspended on a 180° shaker at room temperature for 5 minutes, after which the solution was discarded and replaced, and the reaction was continued for an additional 10 minutes. After completion of the reaction, the beads were washed 3 times each with NMP, DCM, and again with NMP. The cyclization reaction of the beads from which both the Fmoc and OAll protecting groups had been removed was carried out using an NMP solution containing (7-azabenzotriazol-1-yloxy)tripyrrolidinophosphonium hexafluorophosphate (PyAOP, 5 equiv., Cat# 36813, GL Biochem) and DIPEA (10 equiv.). The cylinder was sealed with a cap and suspended on a 180° shaker at room temperature for 12 hours. After completion of the reaction, the solid-phase beads were washed three times with NMP and DCM, and then used for the subsequent reaction. An example of the cyclization reaction scheme for the synthesis of cyclic peptides was as follows.

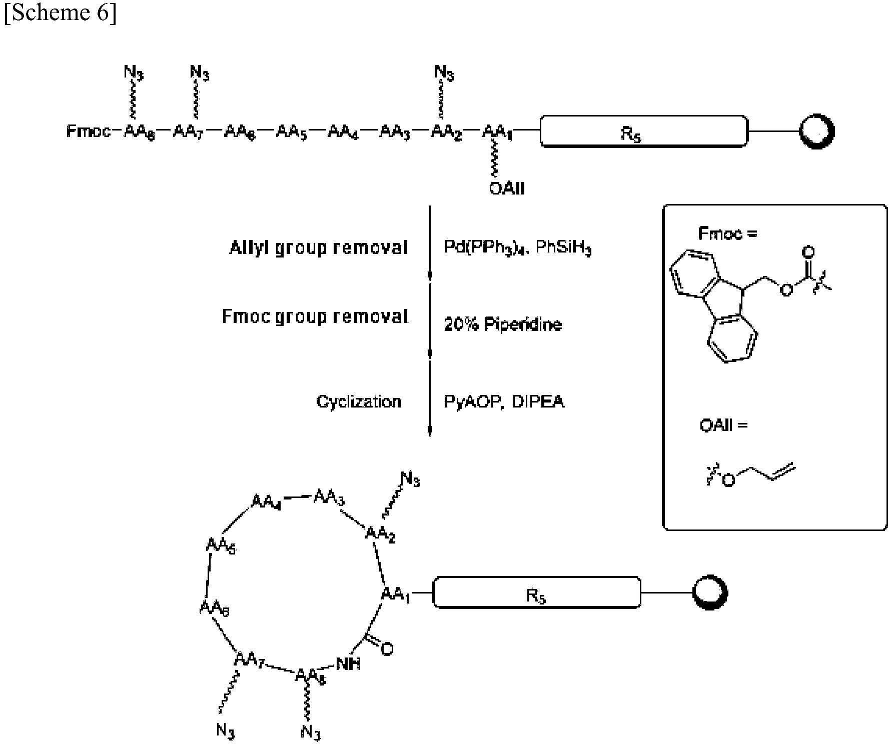

### 1-3-2. Synthesis of Functional Groups

After the cyclization reaction, solid-phase peptides requiring functional group synthesis were subjected to the following reactions on the beads.

Functional groups such as biotin, DOTA, PSC, TCMC, IB, and sIB were introduced by first removing the 4-methyltrityl (Mtt) or 1-(4,4-dimethyl-2,6-dioxocyclohex-1-ylidene)ethyl (Dde) protecting groups from beads loaded with peptides containing lysine residues protected with Mtt or Dde. The Mtt protecting group was removed by treating with a solution of 1.5% TFA / 2.5% TIPS / 96% DCM at room temperature for 5 minutes each, repeated six times. The Dde protecting group was removed after mixing hydroxylamine hydrochloride and imidazole (1.3 : 1, w / w) in NMP, followed by reaction at room temperature for two cycles of 2 hours each, or for a single cycle of 12 hours. The beads with deprotected amine functional groups were then reacted in NMP at room temperature for 1 hour with Biotin-NHS ester (2 equiv., Cat# 246201, SY Innovation), or DOTA-tris(tert-butyl ester) (2 equiv.), or DO2Atbu-mono amide mono acid (PSC with two tert-butyl protecting groups, 2 equiv., Cat# C134, Chematech), or S-2-(4-isothiocyanatobenzyl)-1,4,7,10-tetraaza-1,4,7,10-tetra(2-carbamoylmethyl)cyclododecane (TCMC-NSC, 2 equiv., Cat# BM02751, A2B Chem), or 4-(4-iodophenyl)butanoic acid (IB-OH, 2 equiv., Cat# BD11901, BLD Pharmatech), or 2-(4-iodophenyl)acetic acid (sIB-OH, 2 equiv., Cat# BD6173, BLD Pharmatech), together with TBTU (2 equiv.) and DIPEA (10 equiv.). After completion of the reaction, the beads were washed three times each with NMP and DCM, and then used for the subsequent reaction.

The introduction of the zwitterionic ion (SZW) functional group was carried out using peptides containing lysine or DAP residues protected with Mtt or Dde. As described above, the Mtt or Dde protecting groups were removed to generate deprotected amine groups at the N-terminus of the peptides synthesized on the beads. Subsequently, 2-bromoacetic acid (30 equiv., Cat# 17000, Sigma-Aldrich) and DIC (30 equiv.) were dissolved in DMF and added to the beads containing the deprotected amines, followed by reaction for 3 hours. After completion of the reaction, the beads were washed three times each with NMP and DCM. The in-house synthesized S4 (30 equiv.) was then dissolved in triple-distilled water and added to the beads suspended in DMF together with DIPEA (30 equiv.), followed by reaction for 12 hours. After completion of the reaction, the beads were washed three times each with NMP and DCM, and then used for the subsequent reaction.

The sulfonamide (SFA) functional group was then introduced by a click reaction between peptides containing an azido functional group on solid-phase beads and 4-ethynylbenzenesulfonamide (Cat# E1130, TCI). Under an Ar atmosphere, the peptide containing an azido group on the solid-phase beads (1 equiv.), 4-ethynylbenzenesulfonamide (2 equivalents per azido group), CuI (1 equivalent per azido group), tris(benzyltriazolylmethyl)amine (TBTA, 2 equivalents per azido group, Cat# T2993, TCI), and DIPEA (10 equiv.) were mixed in NMP and reacted at room temperature for 12 hours. After completion of the reaction, the beads were washed three times with NMP and at least five times with a 0.1 M sodium diethyldithiocarbamate solution in NMP to remove copper impurities. An example of Mtt deprotection of the peptide followed by introduction of IB and SFA functional groups was as follows.

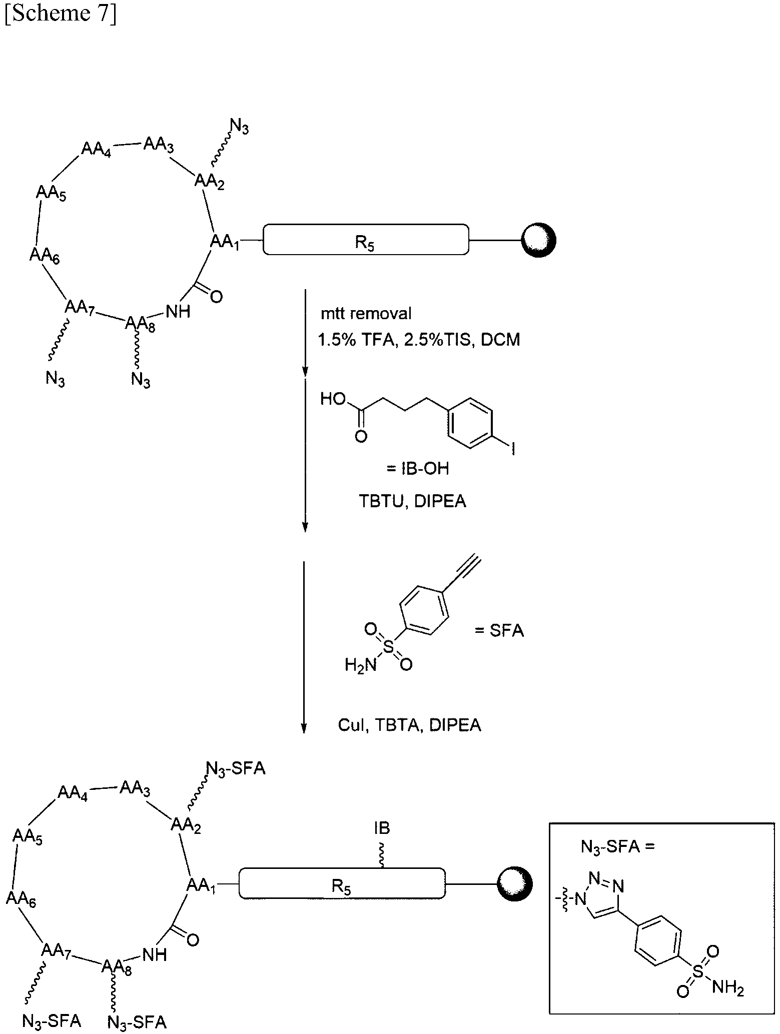

### 1-4. Cleavage and Purification of Peptides from Solid-Phase Beads

The dried, peptide-loaded beads were treated with a mixture of 95% TFA, 2.5% TIPS, and 2.5% triple-distilled water at room temperature for 6 hours. The beads were removed using a filter, and the TFA mixture containing the peptides was collected in a conical tube and evaporated under a nitrogen stream to remove most of the solvent. The peptides were then precipitated by the addition of diethyl ether and centrifuged at 3000 rpm for 5 minutes. The supernatant containing diethyl ether and a small amount of residual TFA was discarded, and the resulting solid peptide was dried under vacuum.

The cleaved peptides from the solid-phase beads were dissolved in a mixture of acetonitrile (ACN) / triple-distilled water (1 : 1), and insoluble debris was removed using a 45 µm syringe filter. The solution was then purified and analyzed using the HPLC system described in Example 1-1. The LC-MS solvent conditions and analytical results are shown in Figs. 6a to 6d. The purified peptide solution was lyophilized to remove the solvent, and the product was obtained in powder form.

### 1-5. Attaching 6OH Functional Groups to Purified Peptides

Powdered purified peptides containing Lys or DAP residues were placed in a glass vial and dissolved in DMF with 6OH(Protected)-NHS (**S1**, 6 equiv.) and DIPEA (15 equiv.), and the mixture was reacted under magnetic stirring for 2 hours. After the reaction, insoluble debris was removed using a 45 µm syringe filter, and the reaction procedure was monitored and purified using the same system described in Example 1-1. The purified peptide solution was lyophilized to remove the solvent, affording the product in powder form. To remove the acetal protecting group of 6OH(Protected), the purified peptide was treated with a mixed solution of 95% (v/v) TFA, 2.5% (v/v) TIS, and 2.5% (v/v) water for 2 minutes. Caution was required, as extending the reaction beyond 2 minutes resulted in cleavage of the 6OH group from the Lys or DAP residues. Immediately after the reaction, a mixture of CAN / triple-distilled water (1:1) was added, and to neutralize the solution, an equivalent volume of trimethylamine (Cat# T0424, TCI) was slowly added in an ice-water bath at 0 °C, corresponding to the amount of TFA solution used. The resulting solution was purified again under the same conditions, and the obtained peptide solution was lyophilized to obtain the product in powder form.

### 1-6. Attaching Fluorescent Dyes (AF488, Cy5) to Purified Peptides

Peptides requiring fluorescent dye labeling were first purified, and the fluorescent dye was conjugated to the peptide via click chemistry between the alkyne functional group introduced into the peptide chain (using Fmoc-propargyl-Gly-OH (Fmoc-Pra-OH, Cat# 21528, GL Biochem)) and the azido functional group of the fluorescent dye. The powdered peptide (1 equiv.), fluorescent dye Alexa Fluor 488 azide (AF488 azide, Cat# 1275, Click Chemistry Tools) or Sulfo-Cyanine5 azide (Cy5 azide, Cat# E3330, Lumiprobe) (1 equiv.), 1 M CuSO₄ aqueous solution (3 equiv.), and 1 M ascorbic acid (6 equiv.) were reacted under an Ar atmosphere for 1 hour. After the reaction, the reaction progress was monitored by LC-MS as described in Example 1-1. For peptides containing a DOTA functional group, sodium diethyldithiocarbamate (5 equiv.) was added and reacted for 20 min to remove copper coordinated to the DOTA moiety, and copper removal was confirmed by LC-MS. The confirmed peptides were purified using the same system described in Example 1-1. Examples of peptide fluorescent dye labeling was as follows.

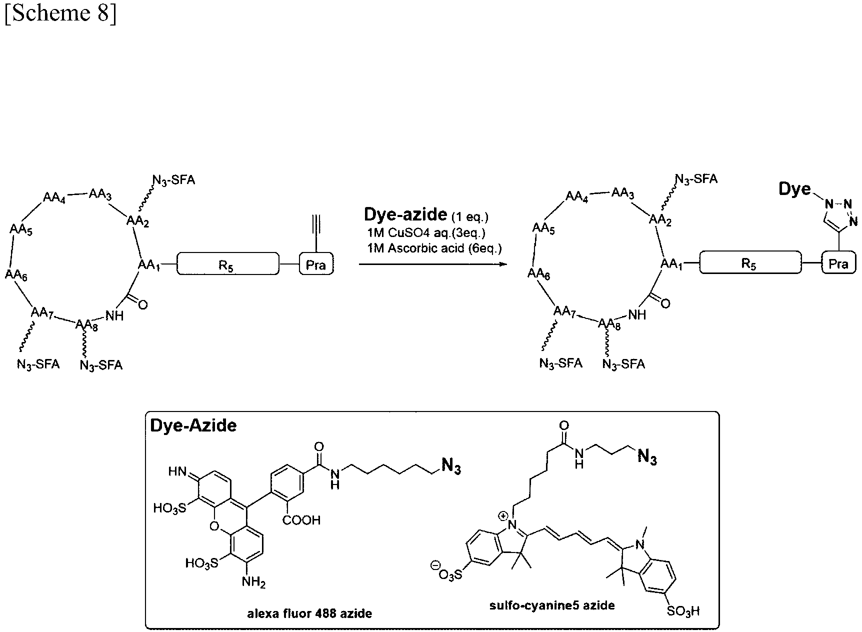

Peptide constructs bearing sulfonamide functional groups that specifically bind to human CAIX were obtained by the synthetic procedures described above. The HPLC conditions and retention times for each compound, as well as the molecular weights observed by electrospray ionization mass spectrometry (ESI-MS), are shown in Figs. 6a to 6d. The analytical instrument used was an LC-MS (1260 Infinity II, Infinity Lab LC/MSD, Agilent), and the data were analyzed using MNOVA software (v.14.2.0, Mestrelab Research, Spain). The stationary phase was an Agilent Poroshell 120 EC-C18 column (4.6 × 50 mm, 2.7 µm), with the column temperature maintained at 40 °C. As the mobile phase, a mixture of acetonitrile (ACN) containing 0.1% TFA and triple-distilled water containing 0.1% TFA was used, and the wavelengths were set at 214 and 254 nm with a flow rate maintained at 1 mL/min.

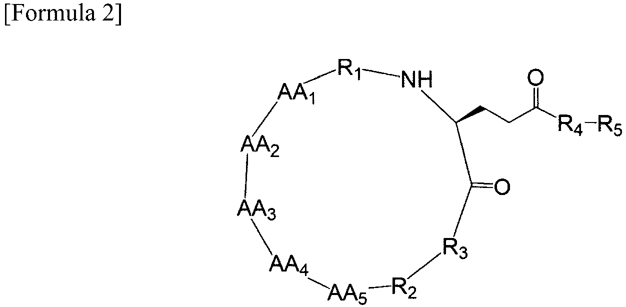

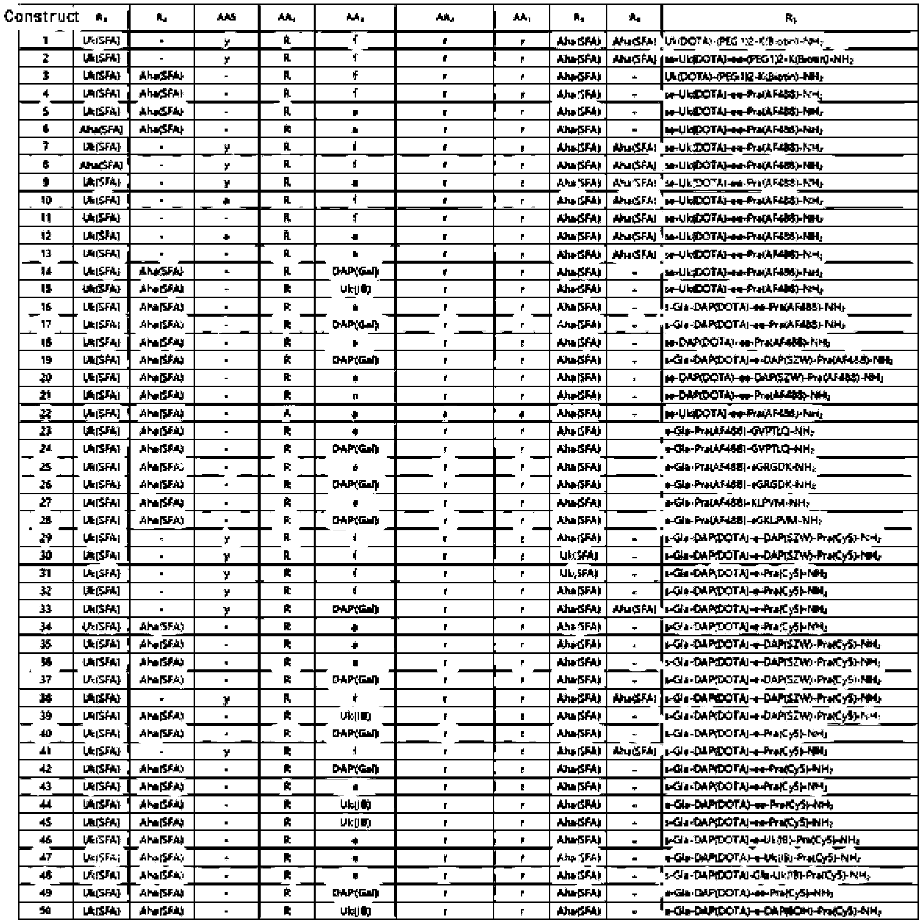

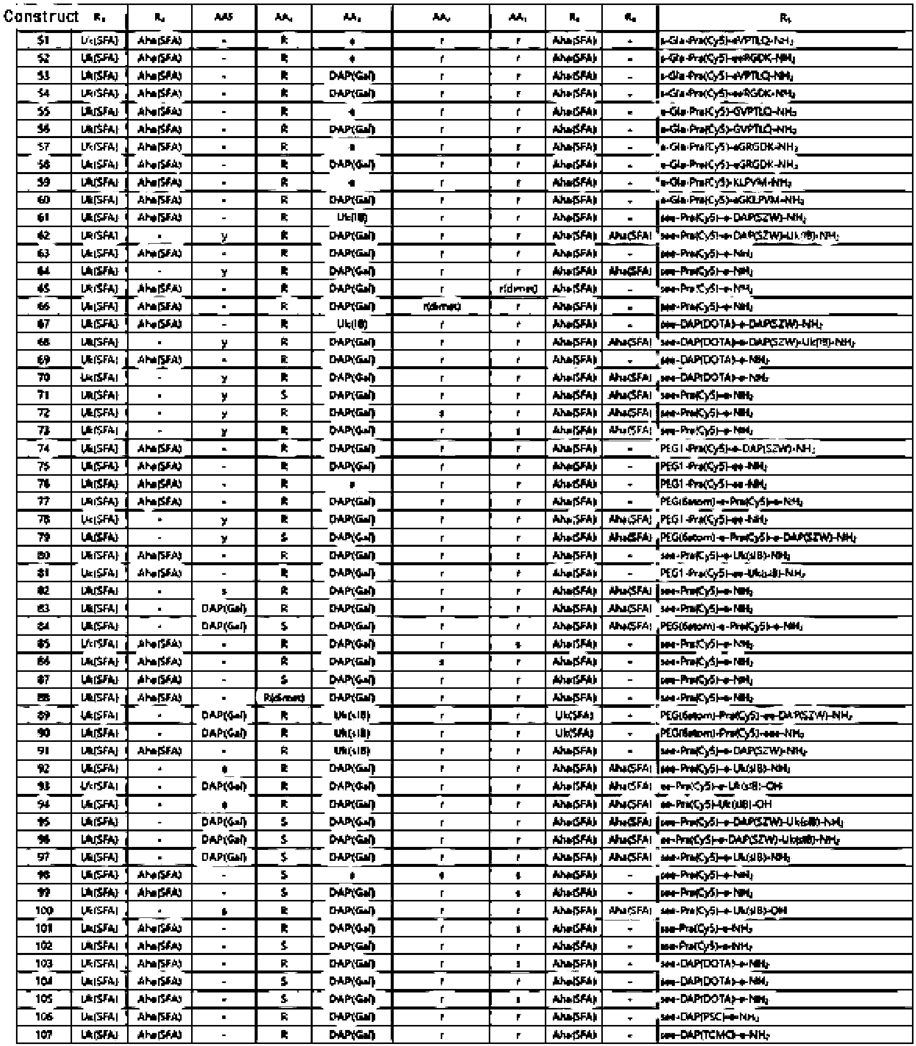

In the above formula, R₁ to R₄ may comprise a sulfonamide. AA₁ to AA₅ refer to amino acid residues comprising natural amino acid residues or amino acid residues comprising functional groups (Gal, IB, sIB, dimethyl). The lowercase letters represent D-amino acids, and the uppercase letters represent L-amino acids, respectively. U refer to a modified or unnatural D-amino acid. Formulas other than natural amino acid residues are defined as having the following structures herein.

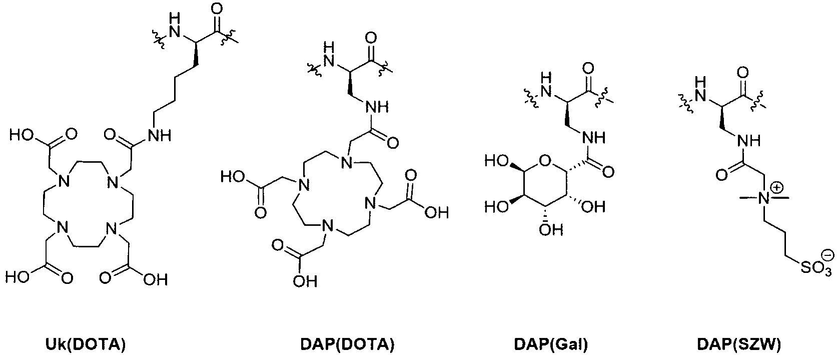

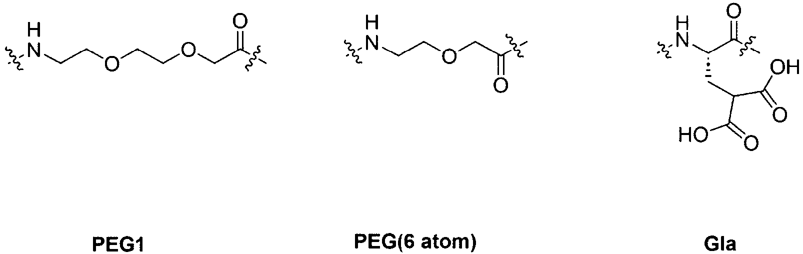

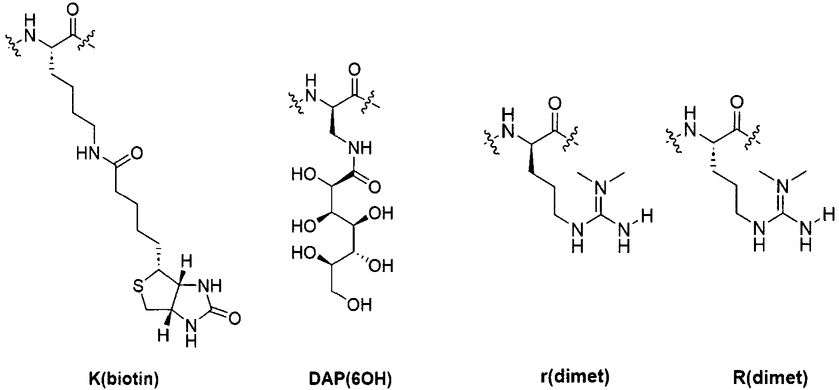

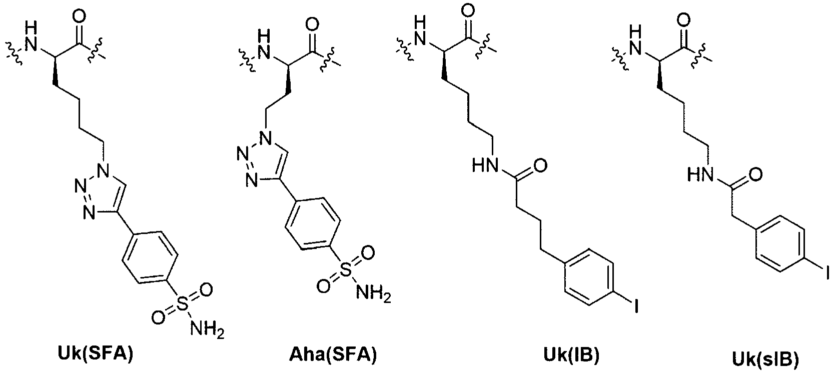

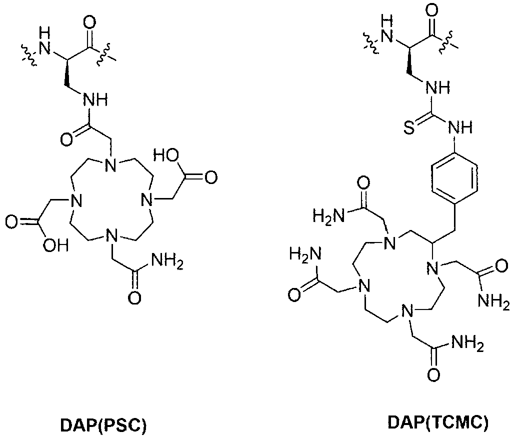

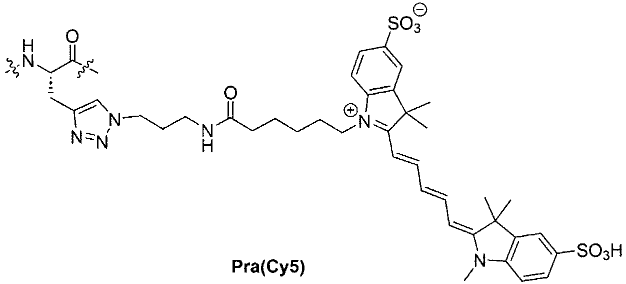

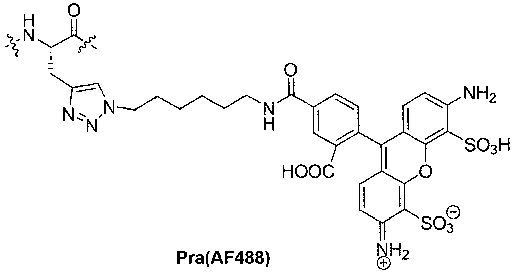

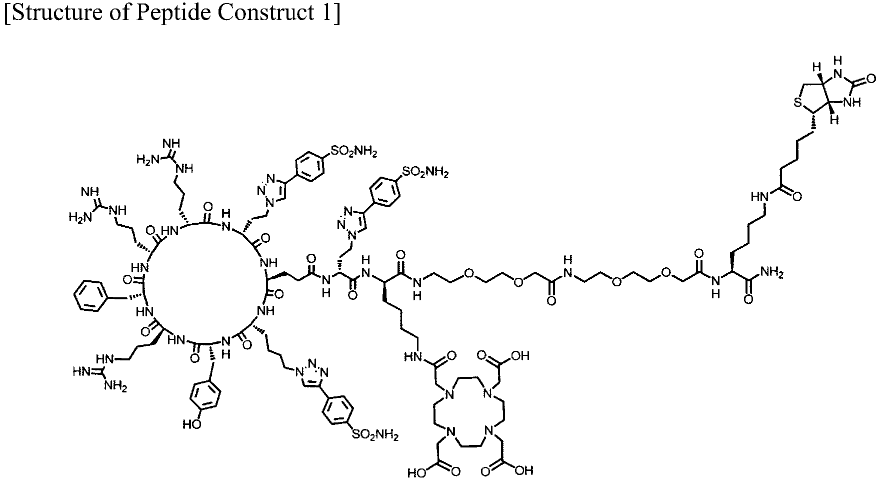

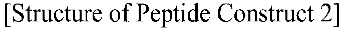

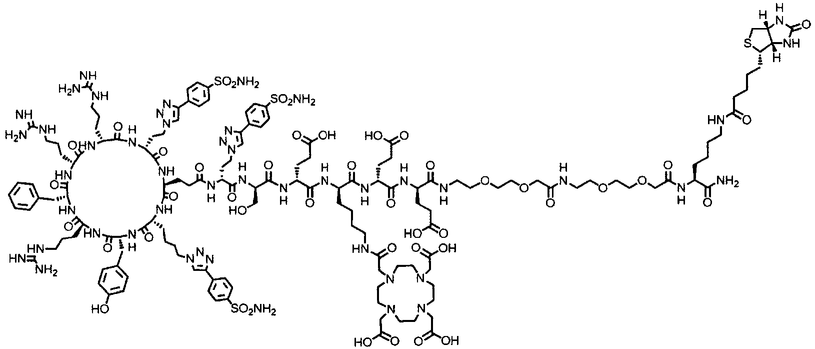

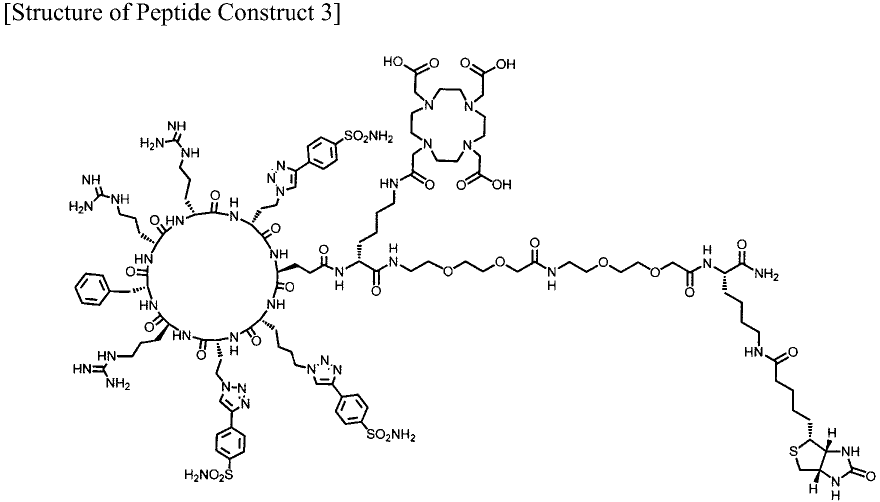

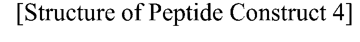

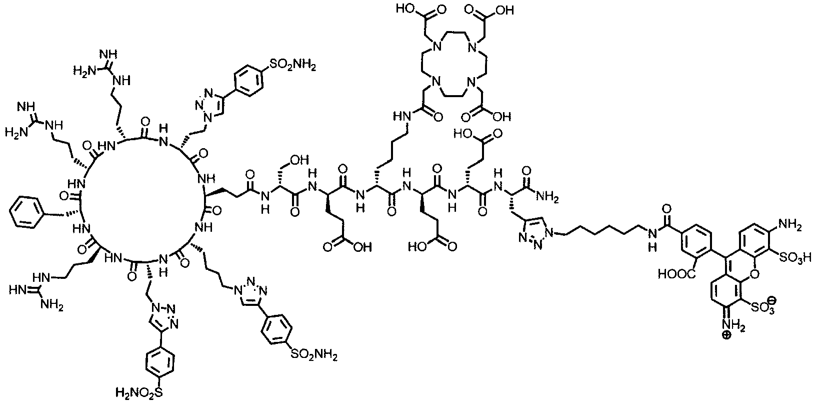

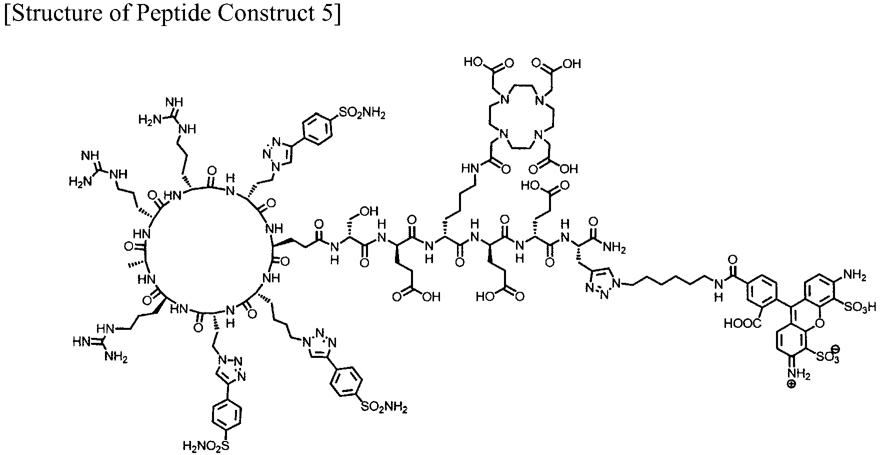

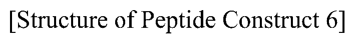

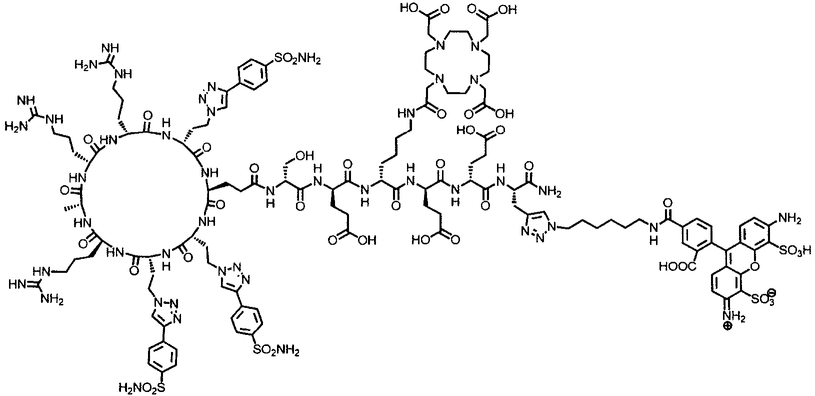

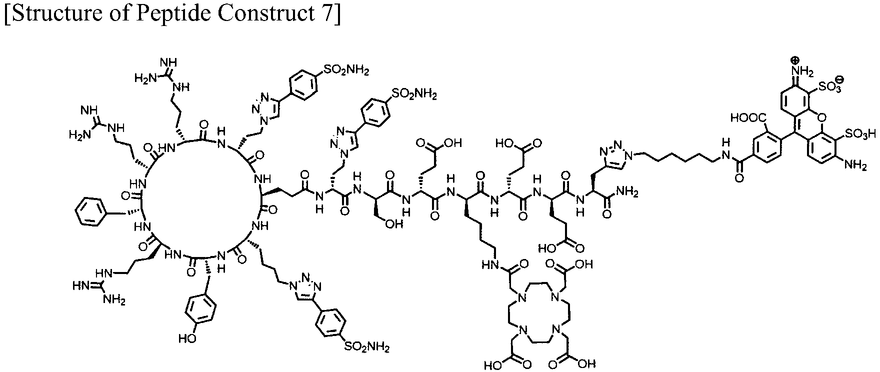

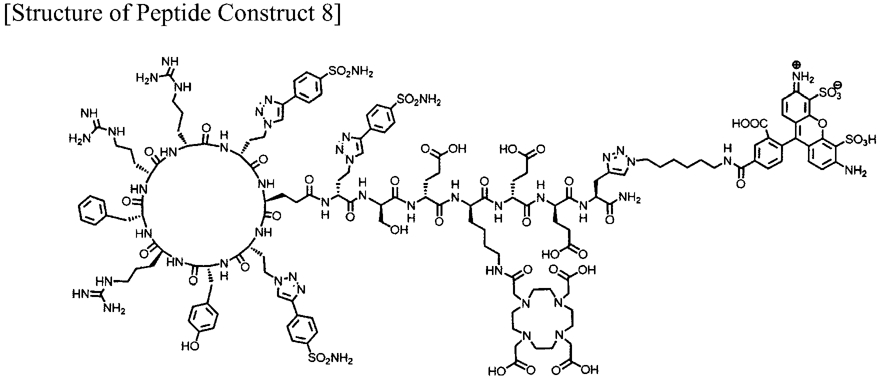

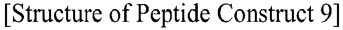

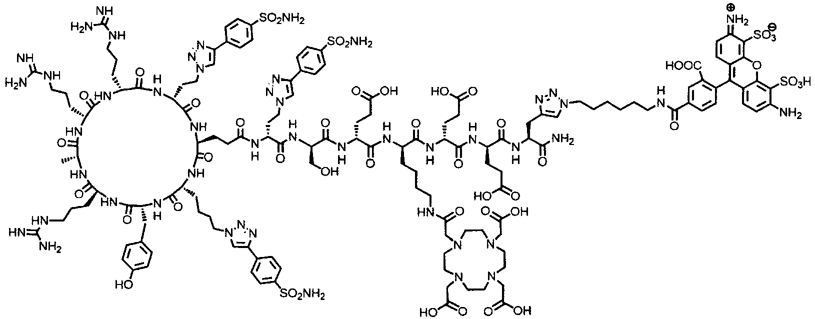

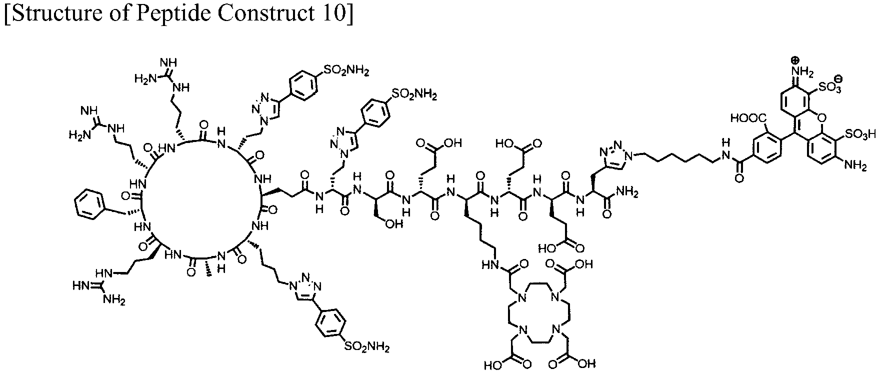

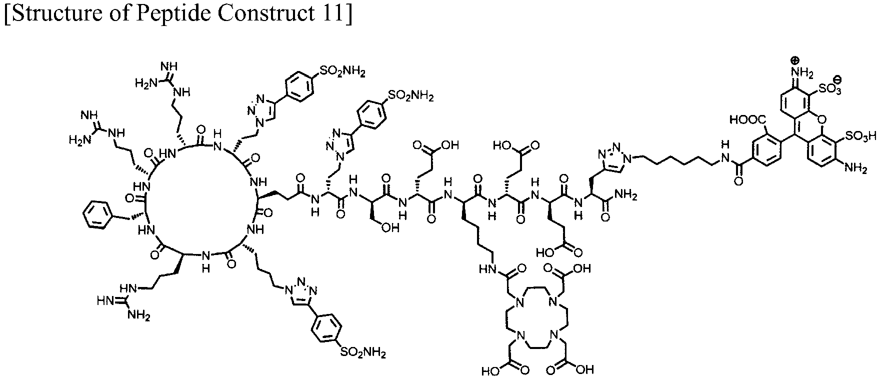

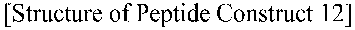

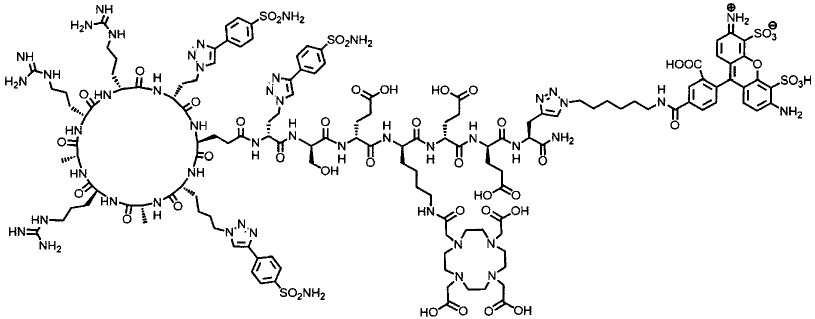

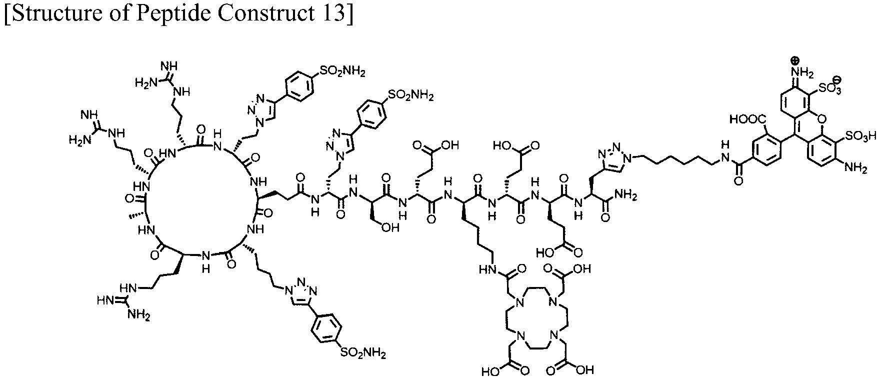

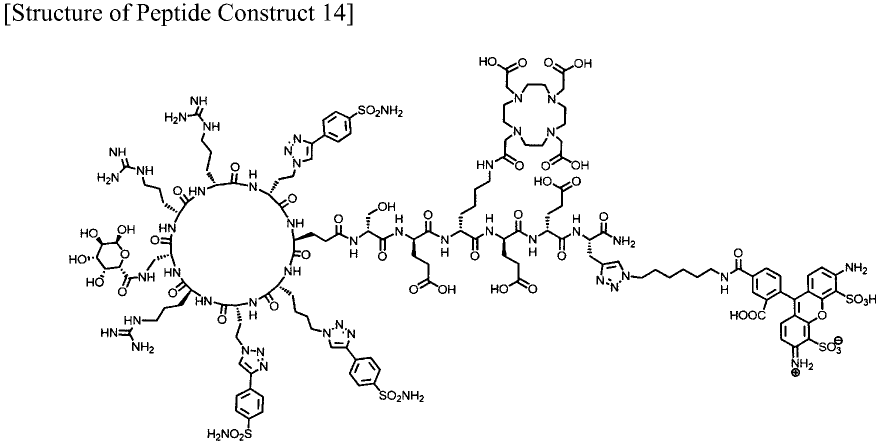

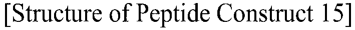

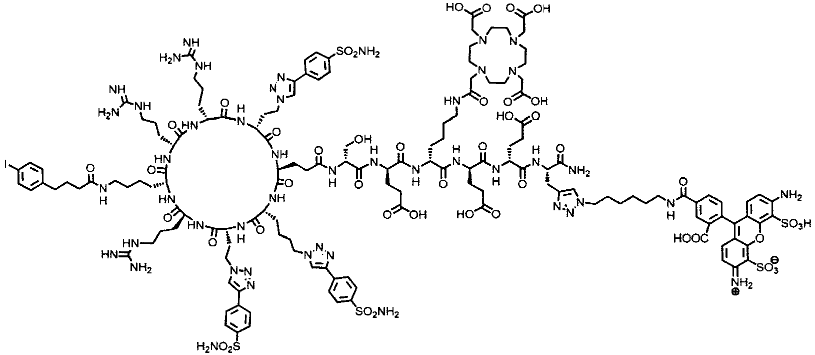

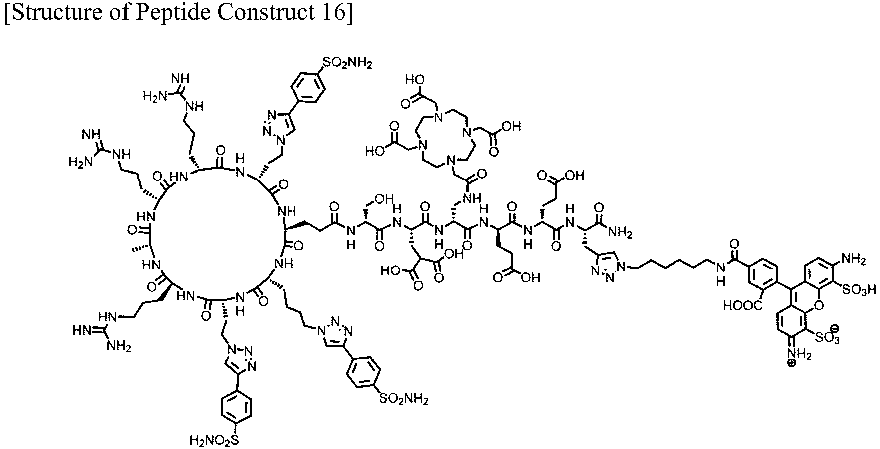

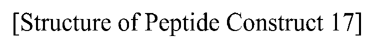

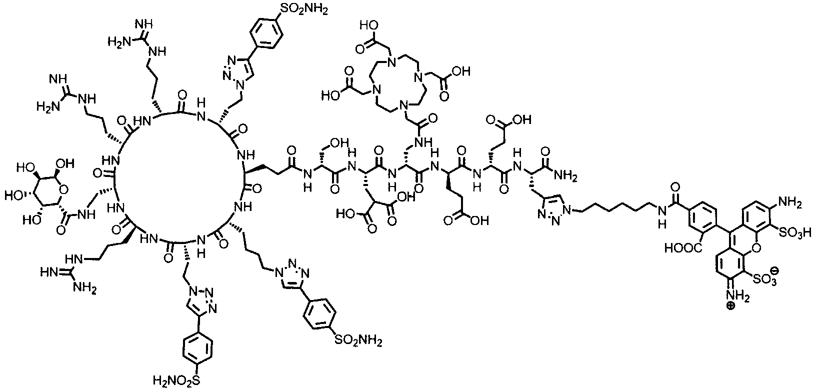

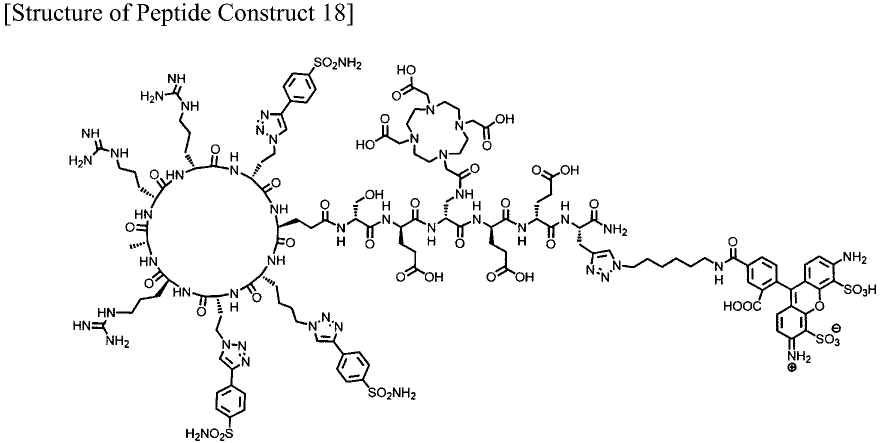

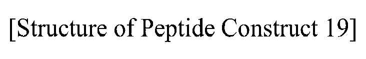

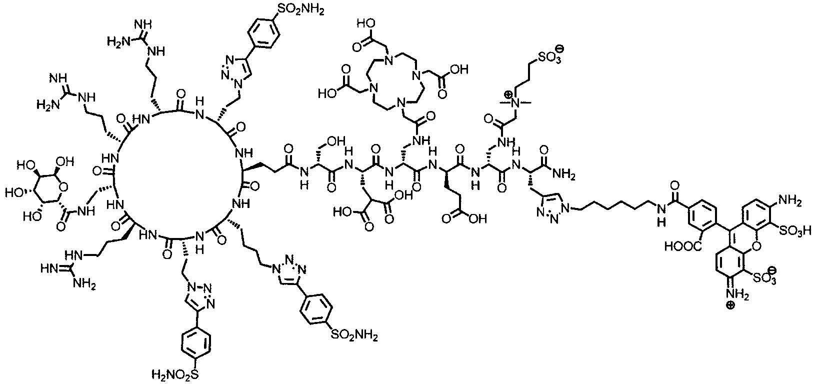

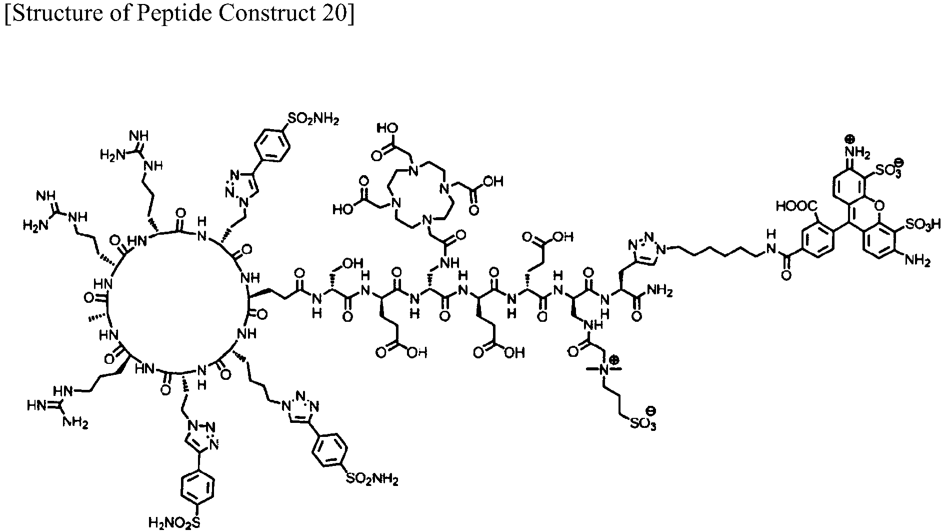

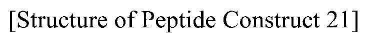

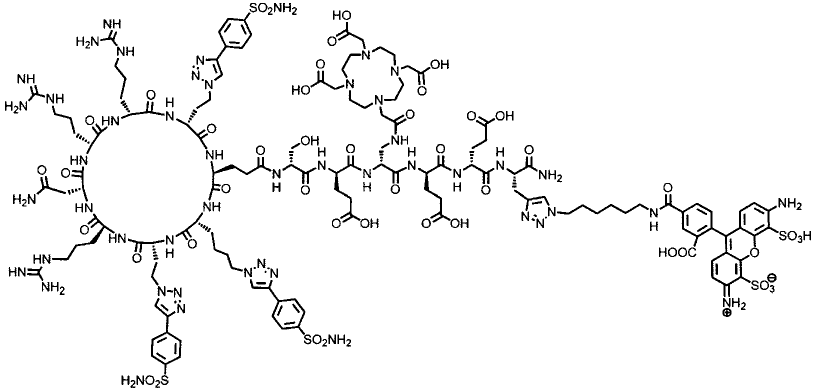

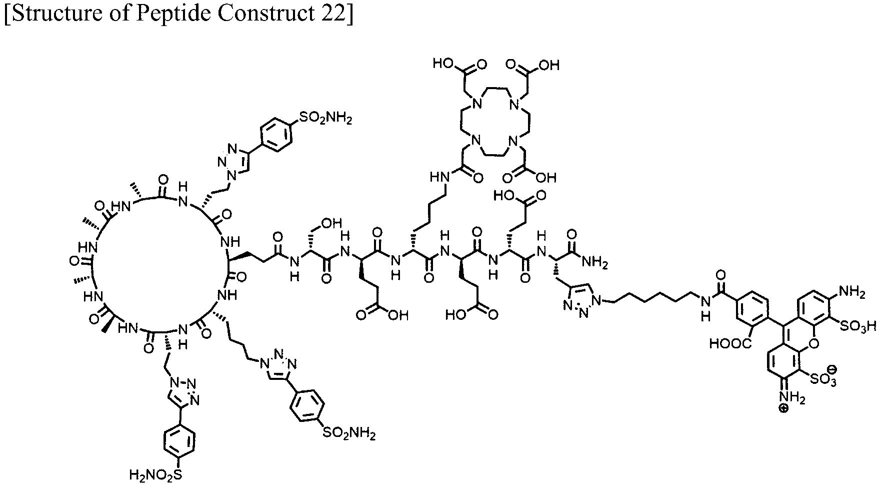

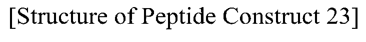

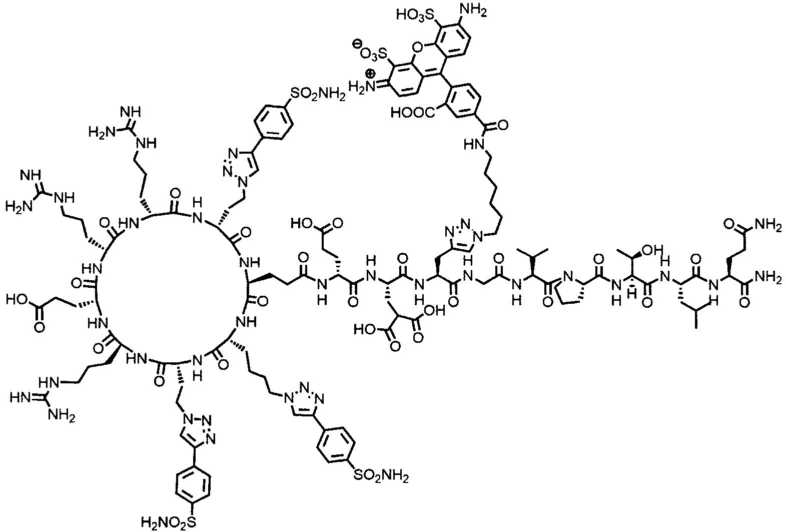

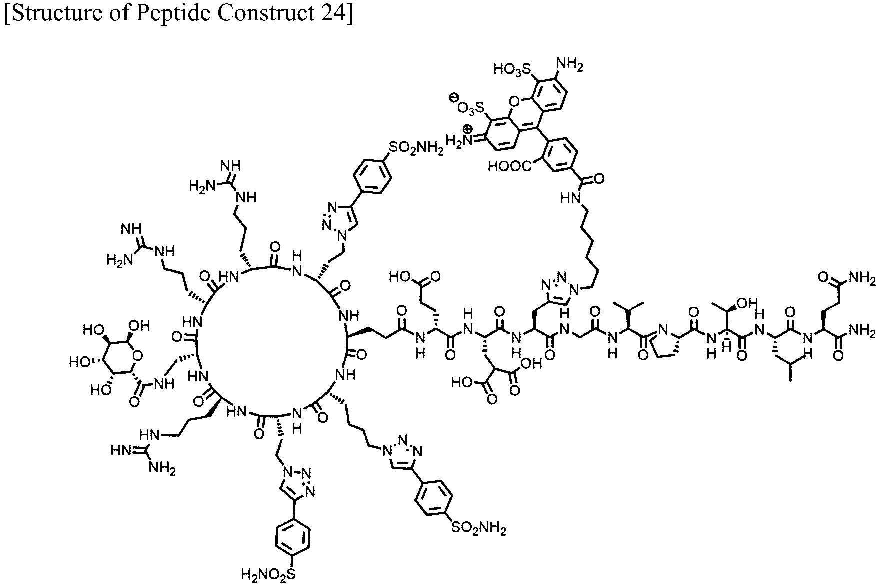

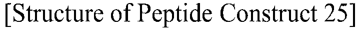

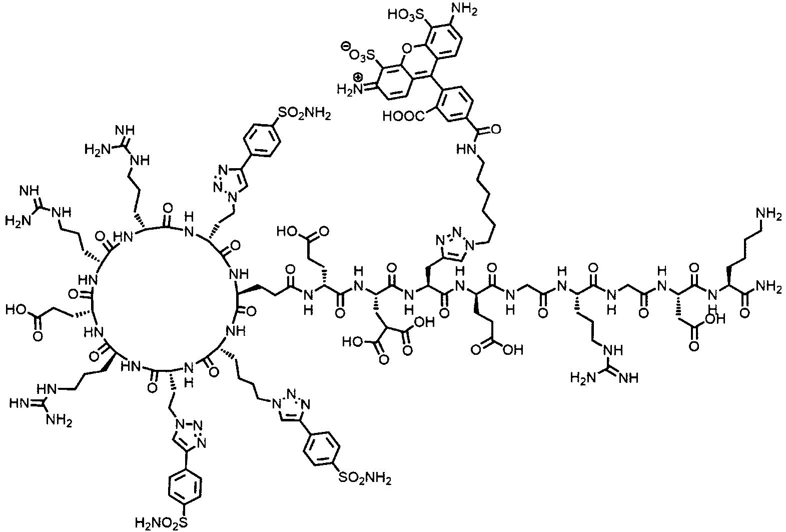

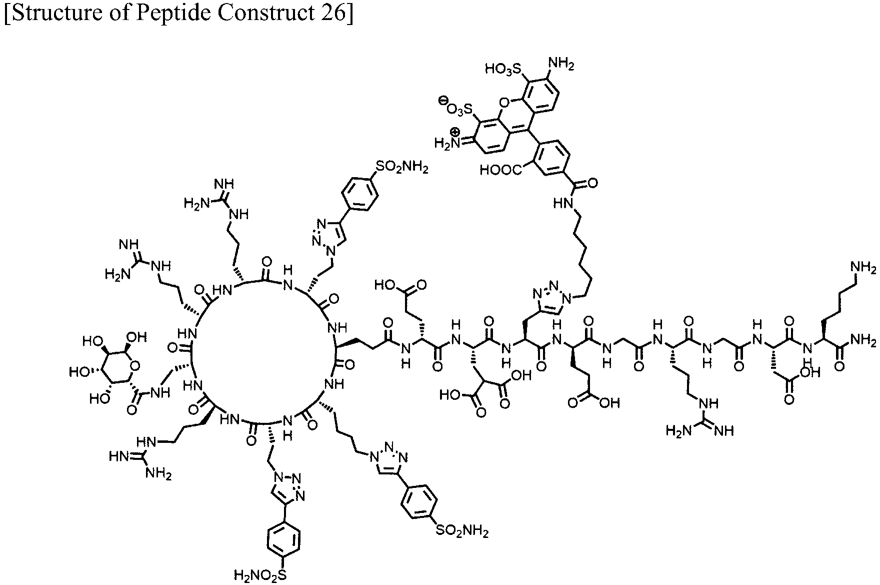

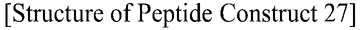

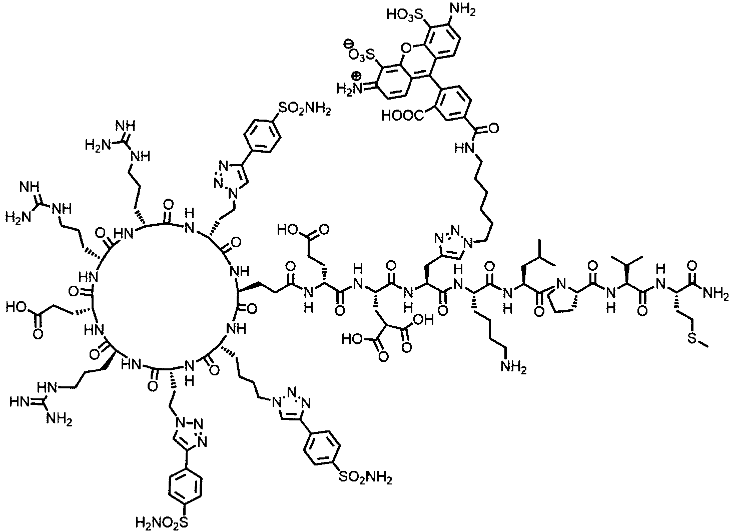

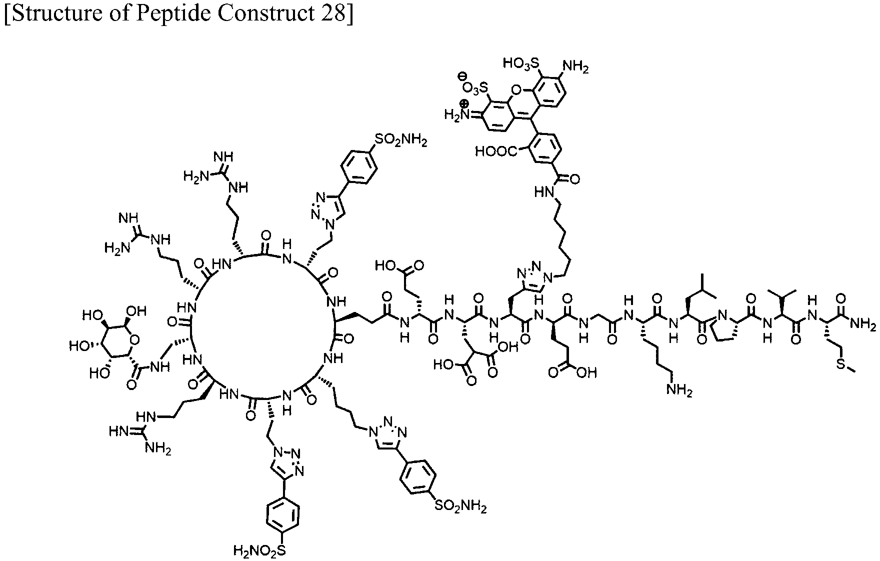

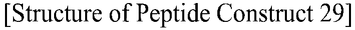

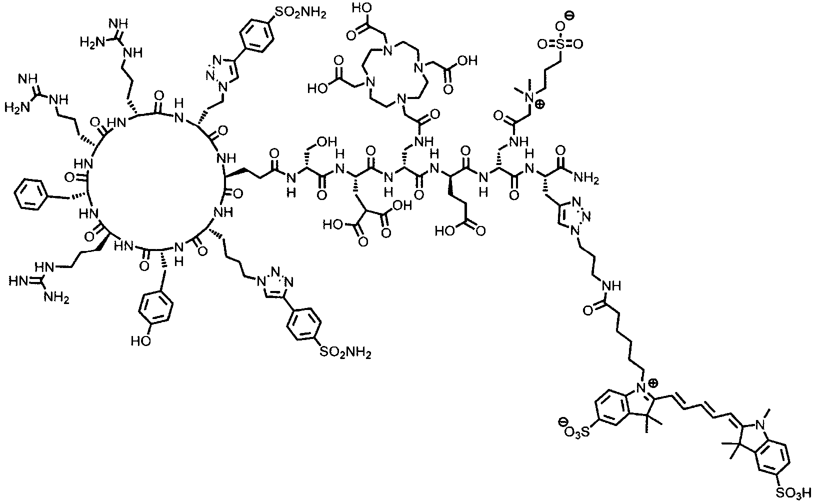

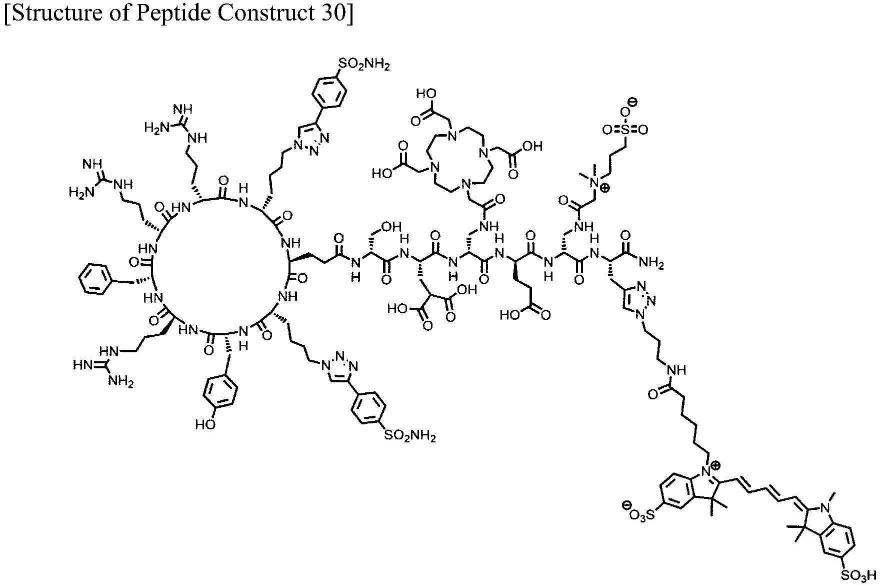

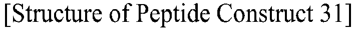

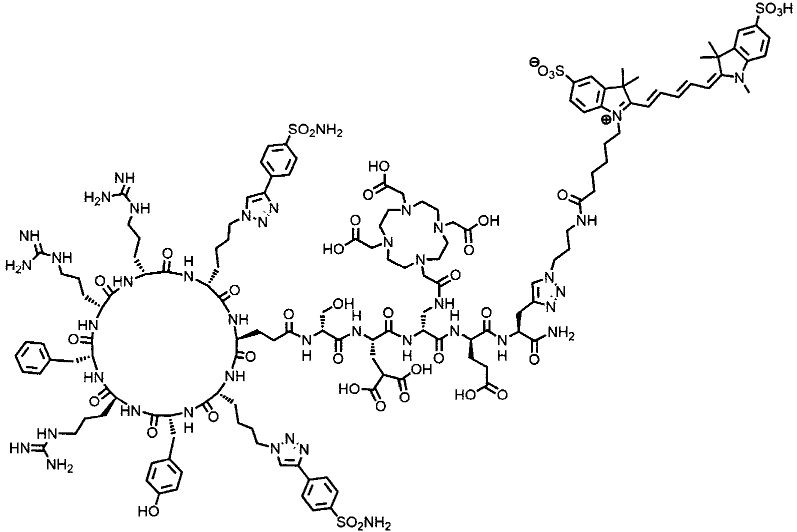

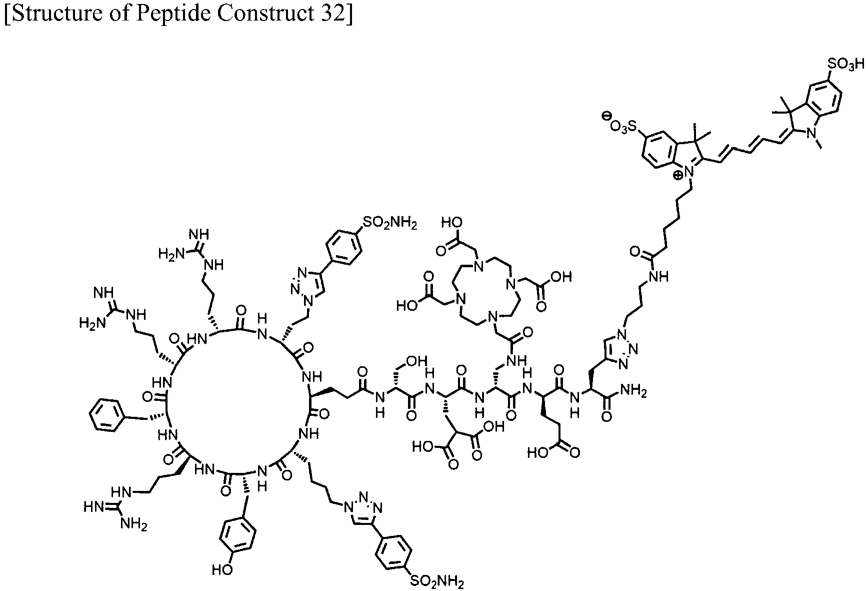

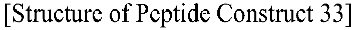

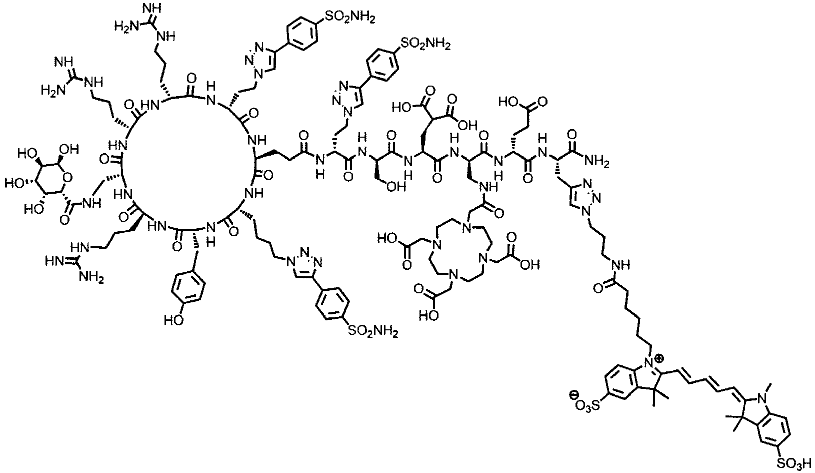

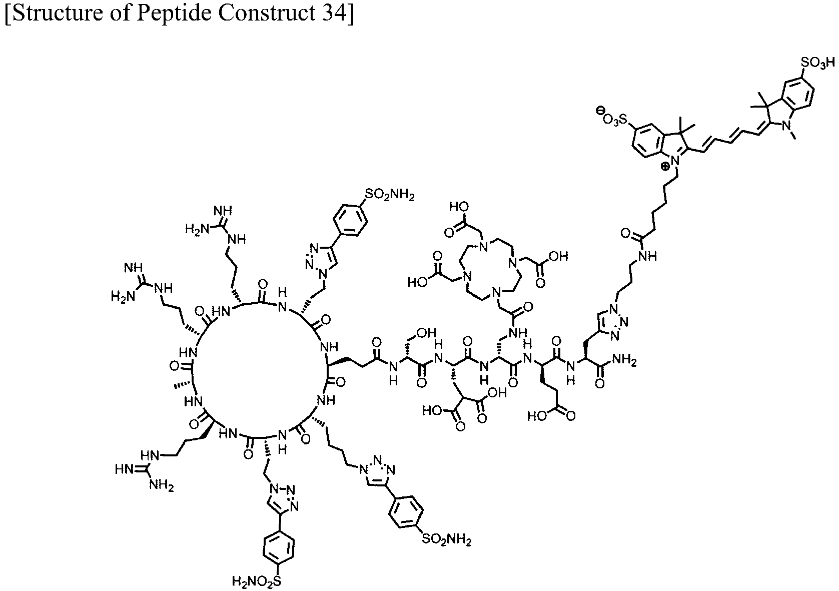

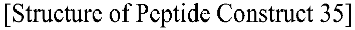

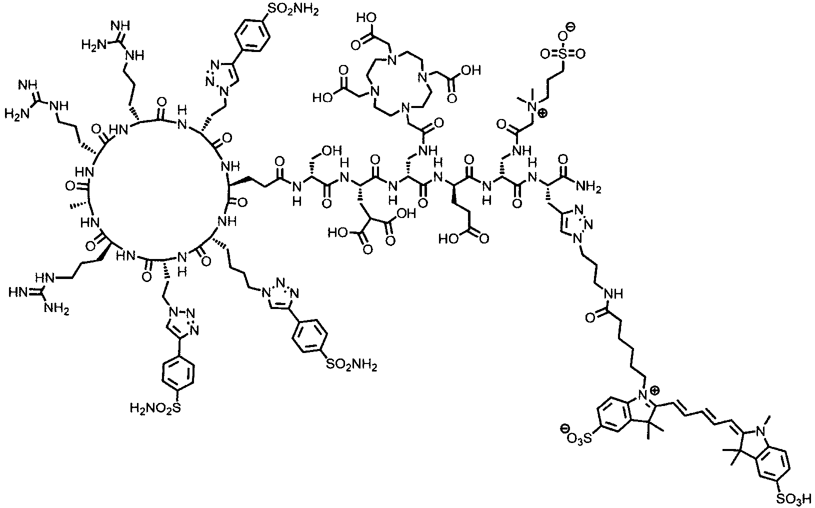

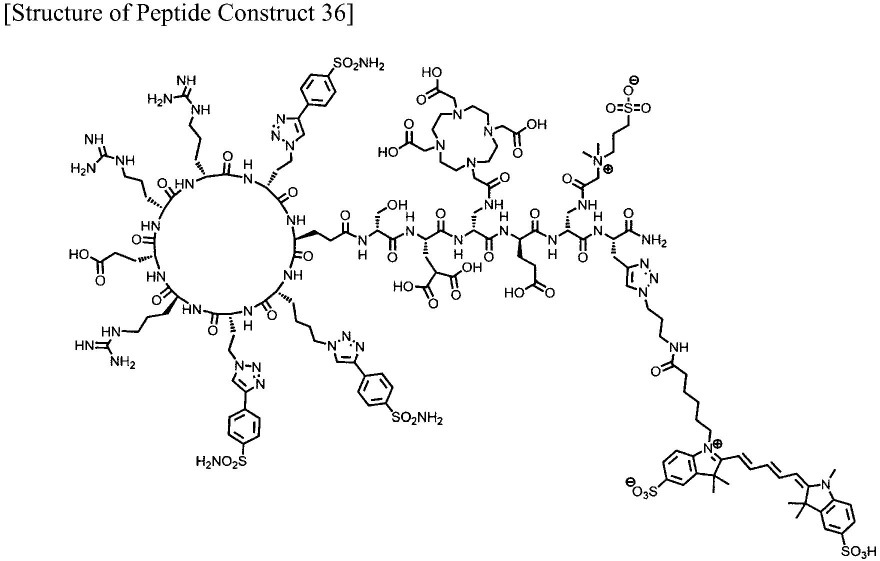

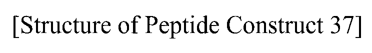

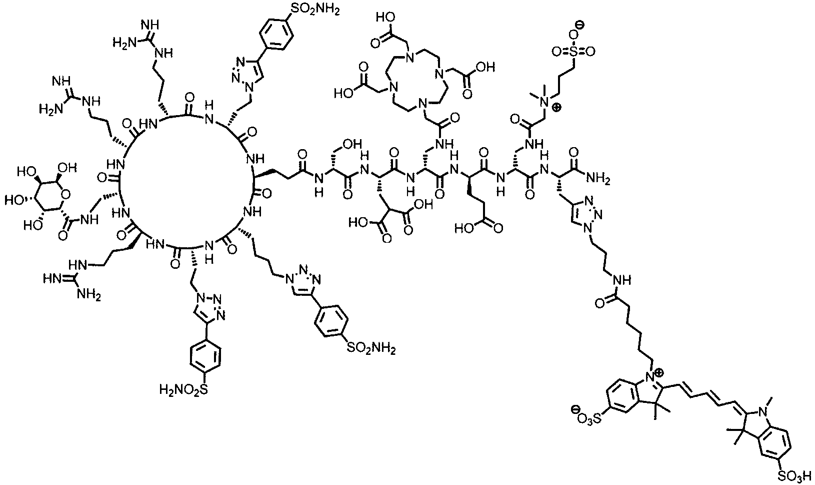

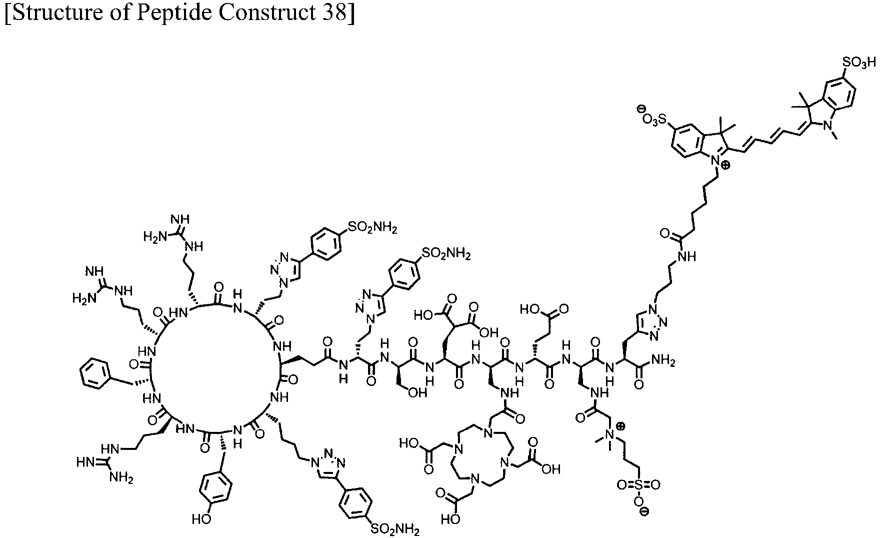

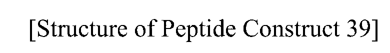

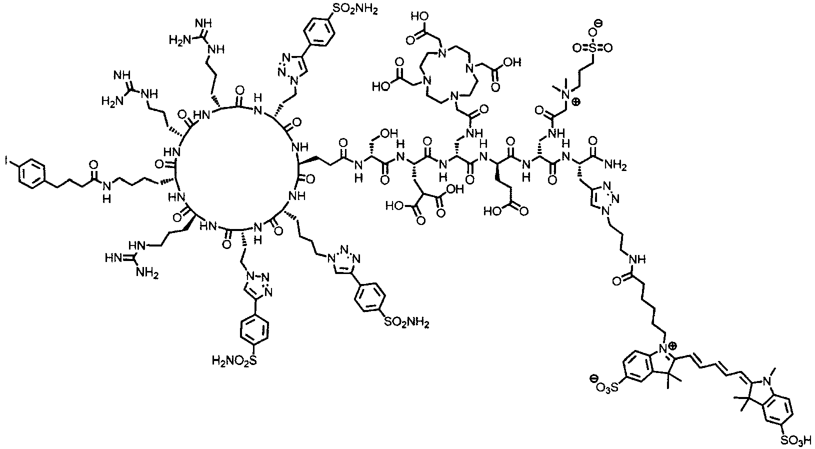

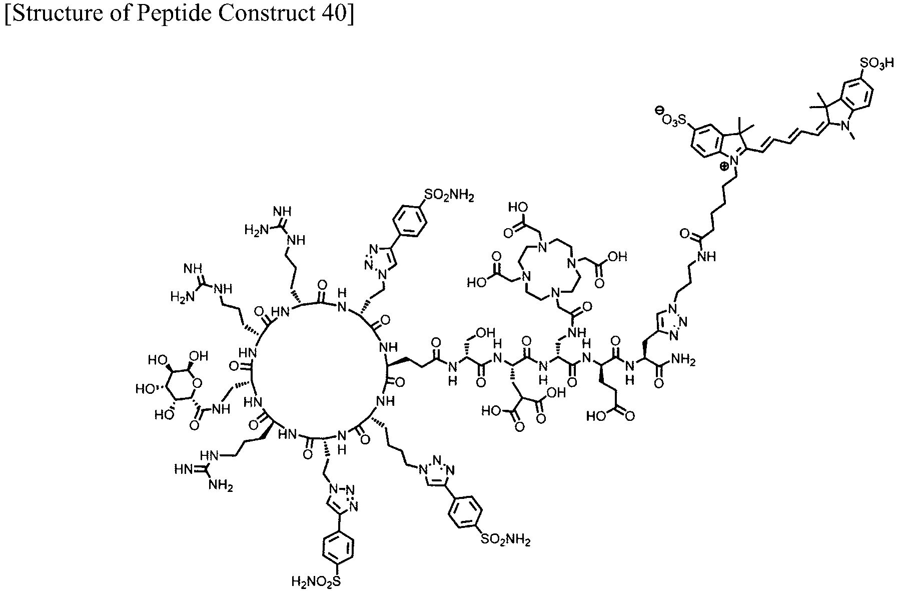

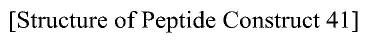

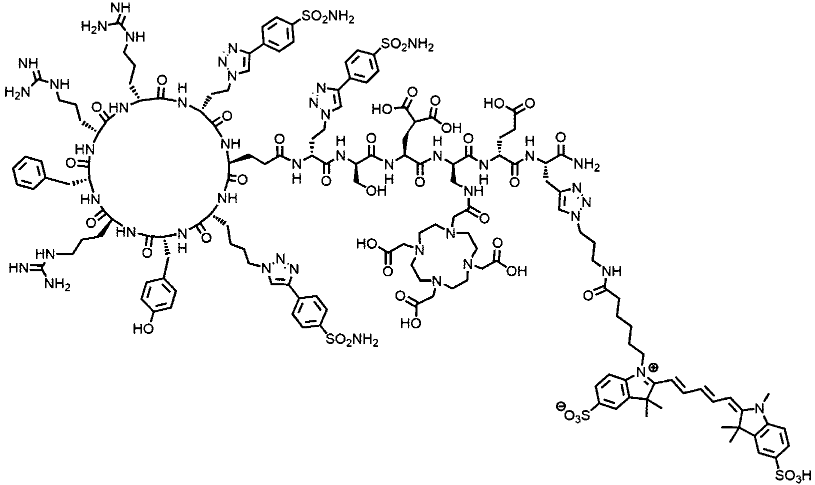

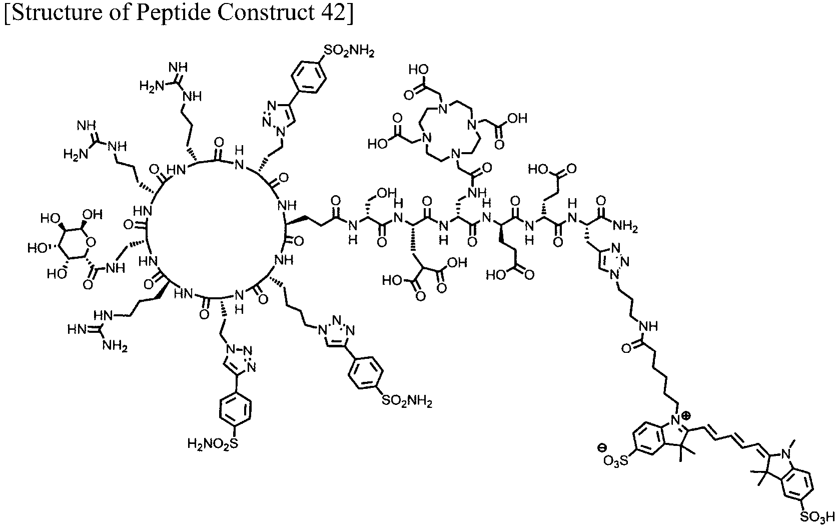

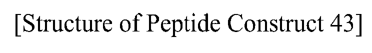

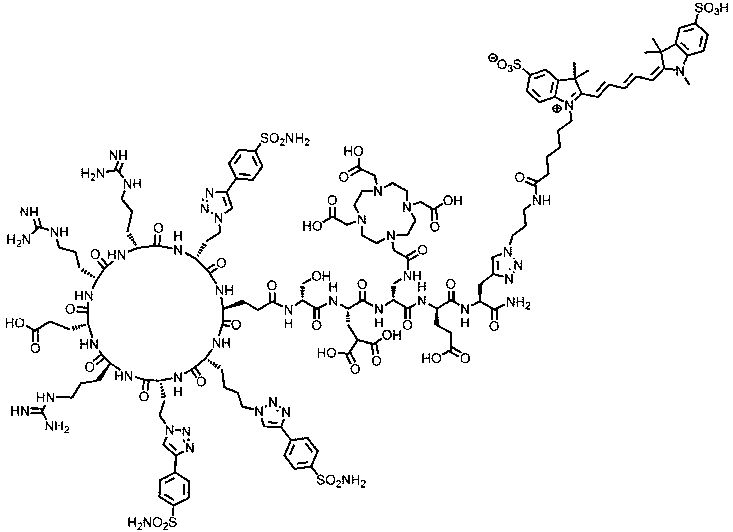

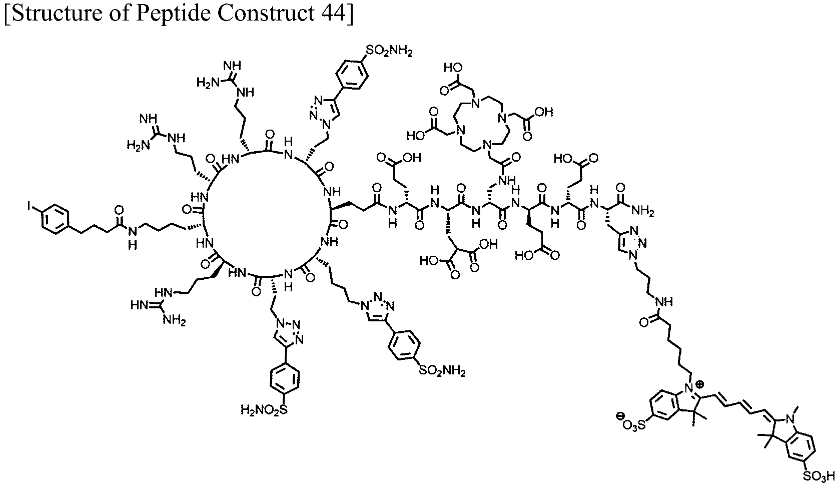

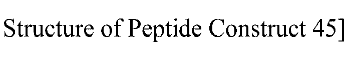

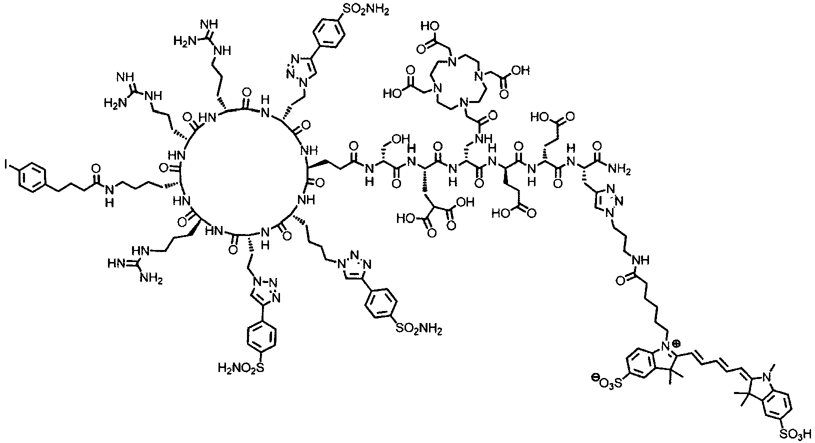

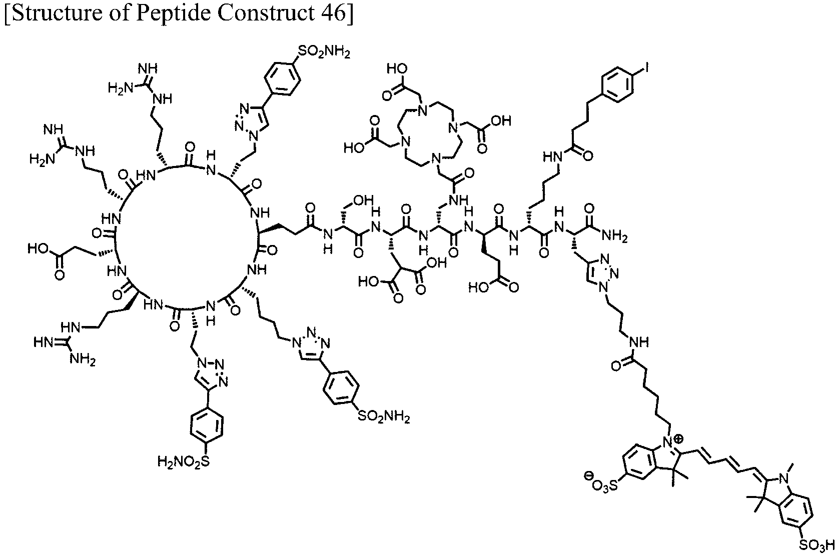

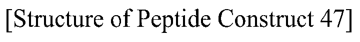

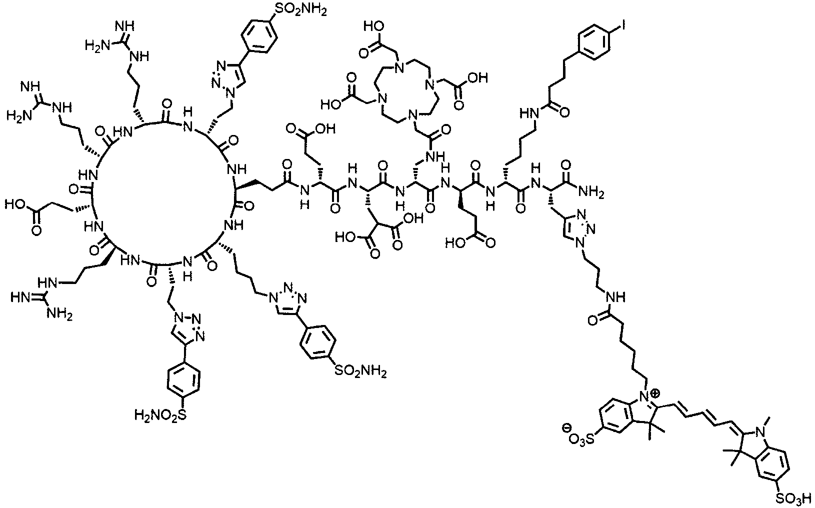

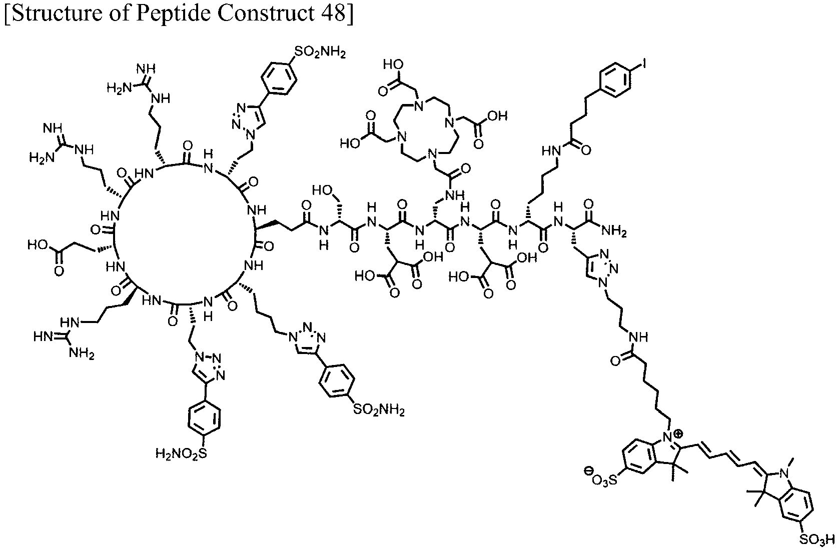

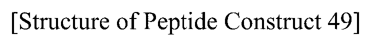

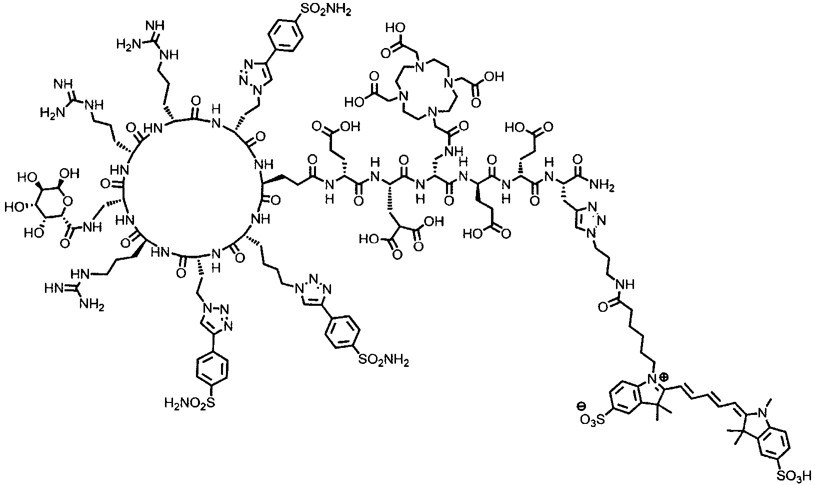

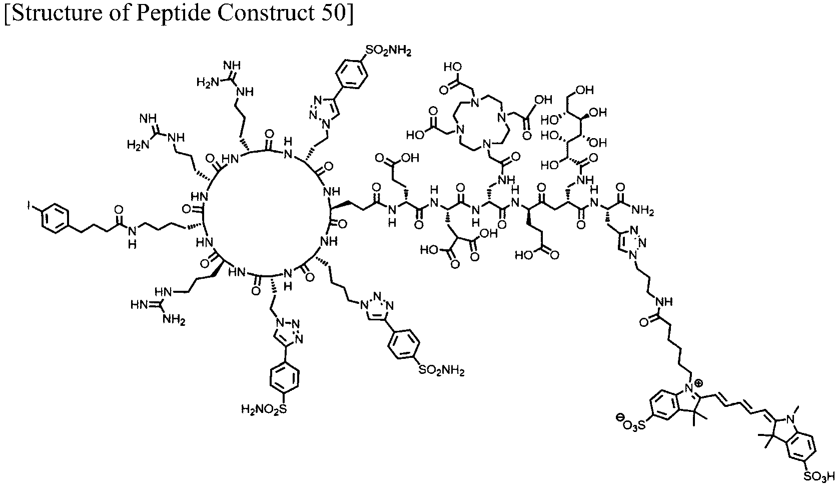

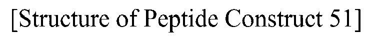

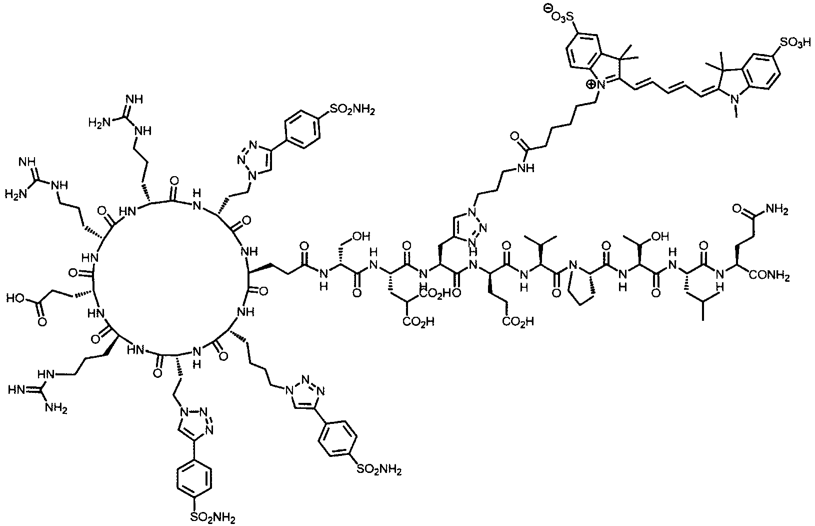

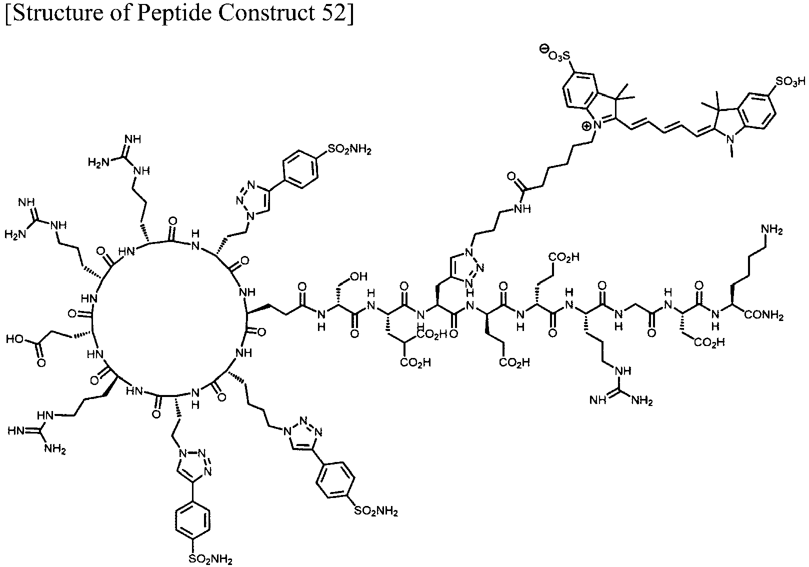

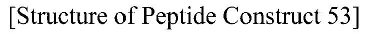

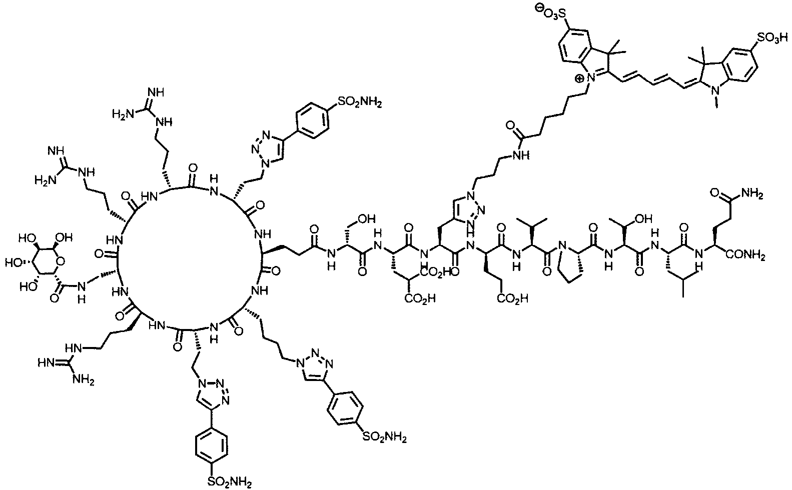

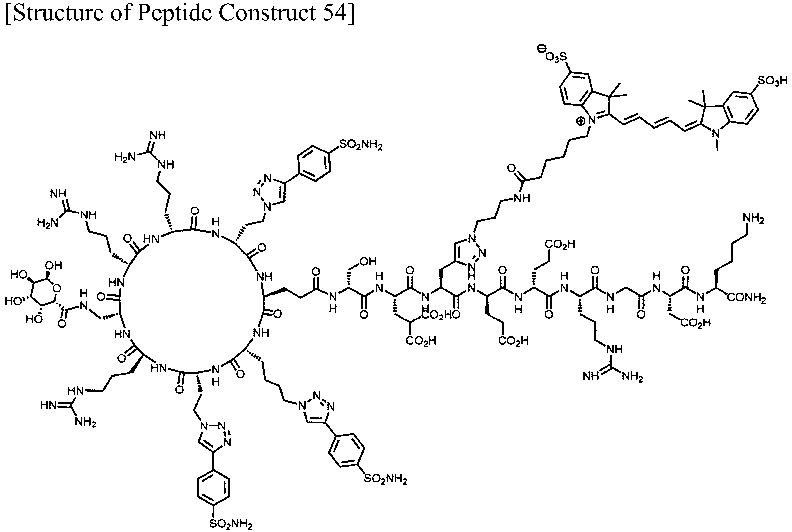

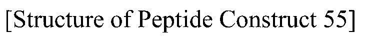

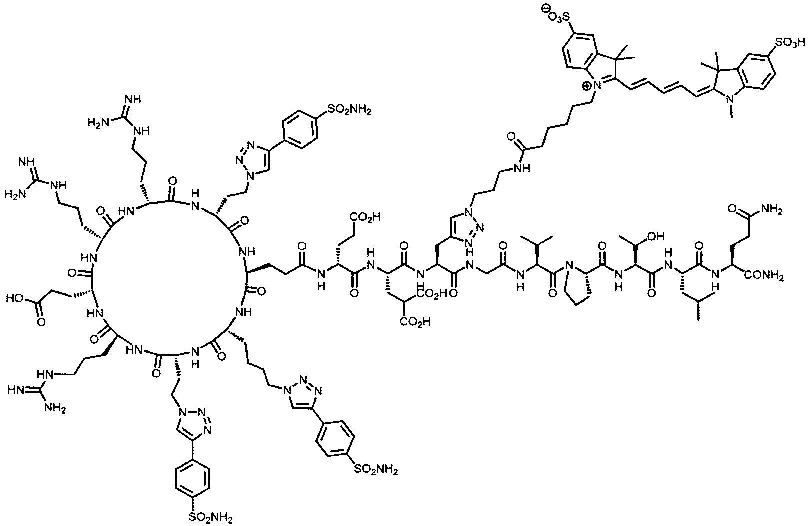

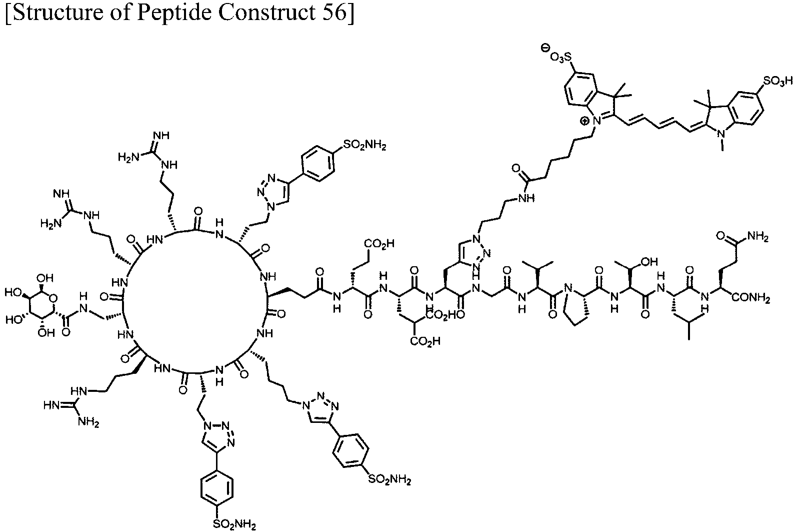

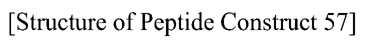

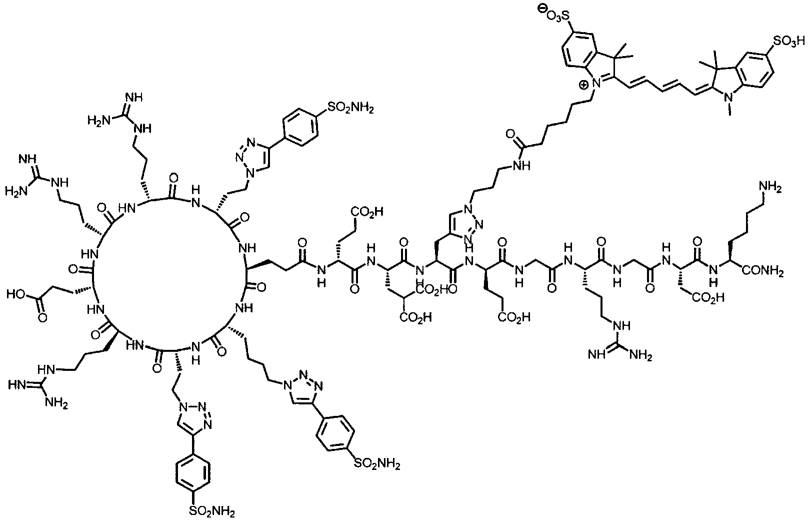

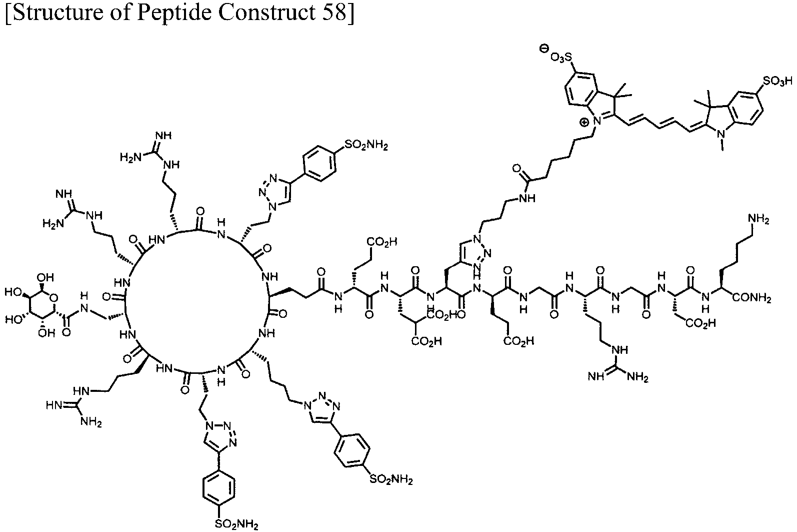

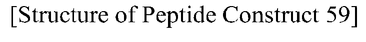

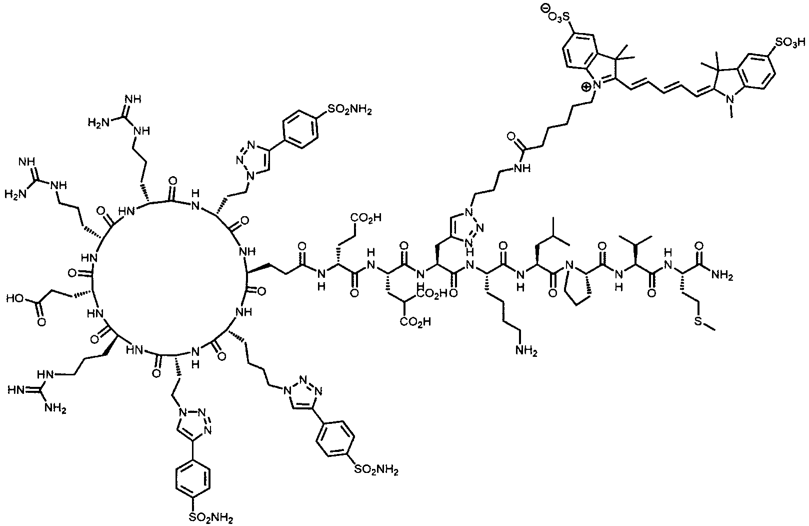

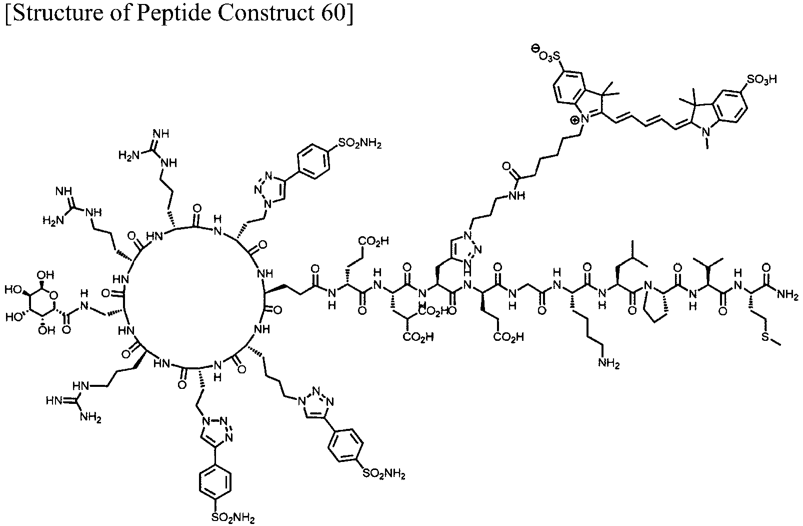

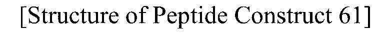

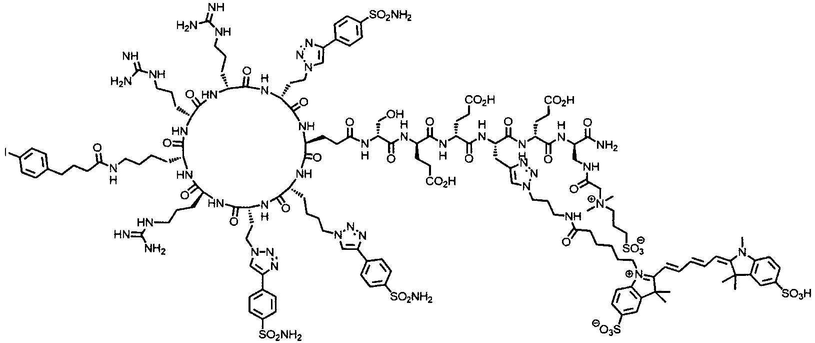

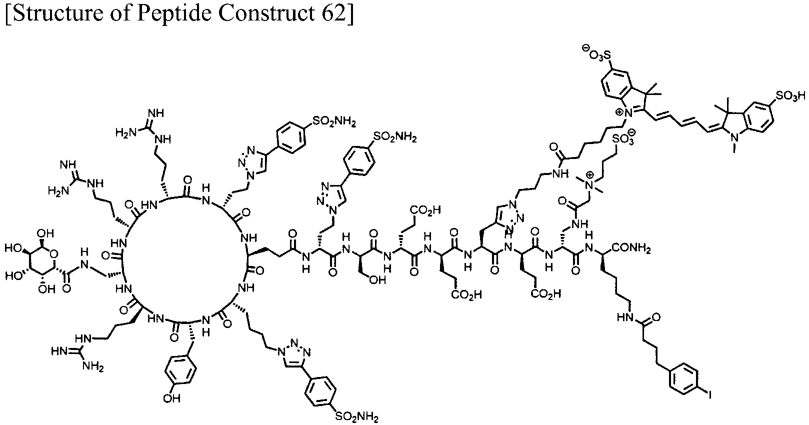

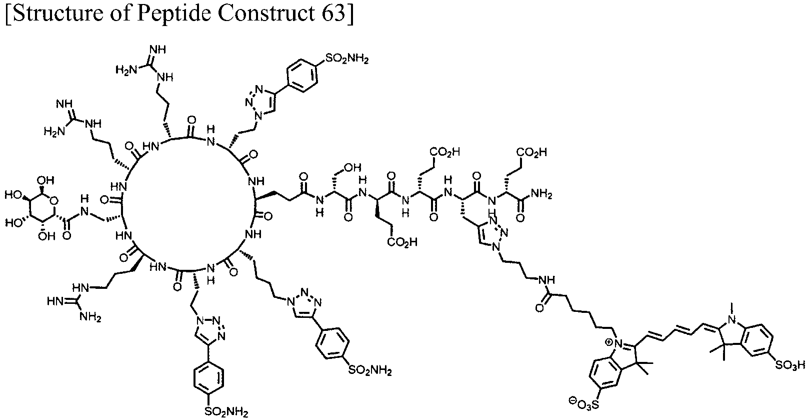

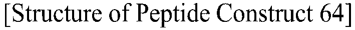

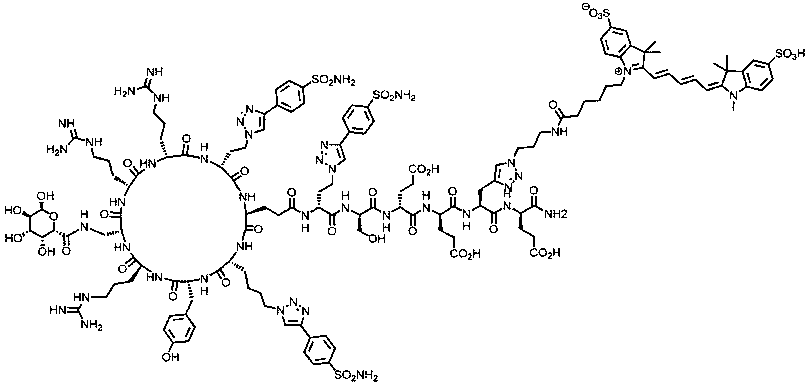

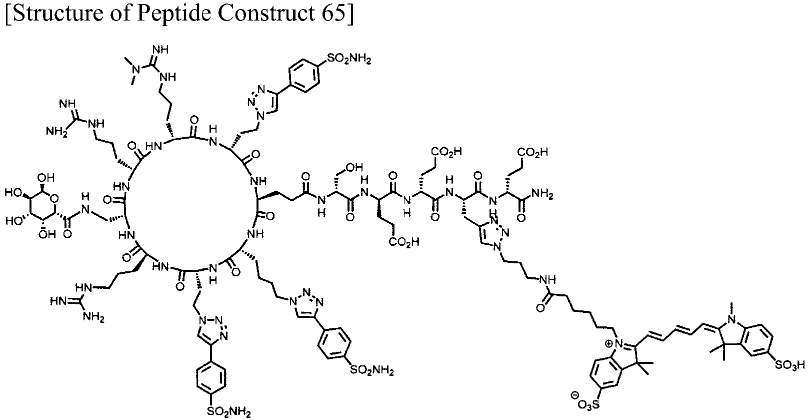

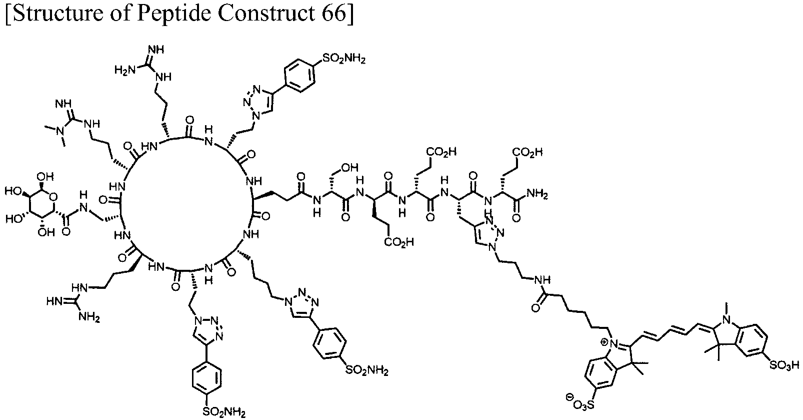

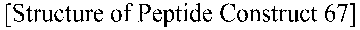

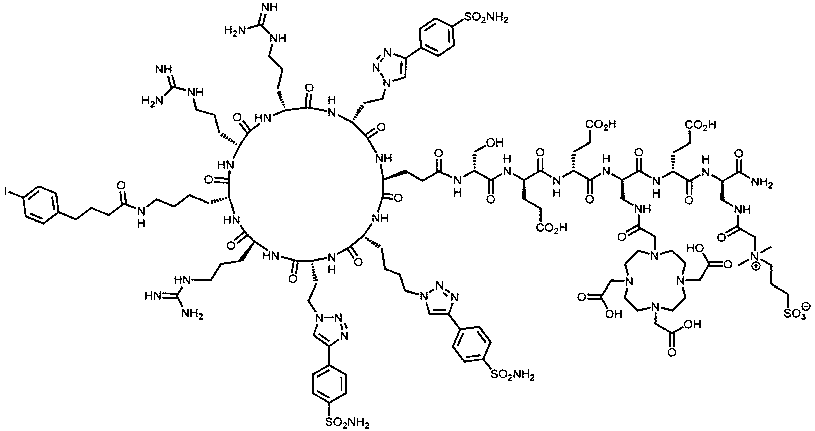

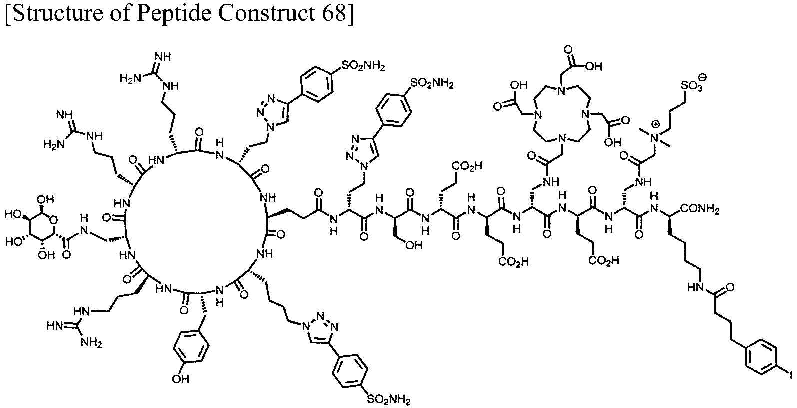

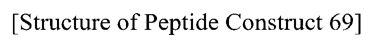

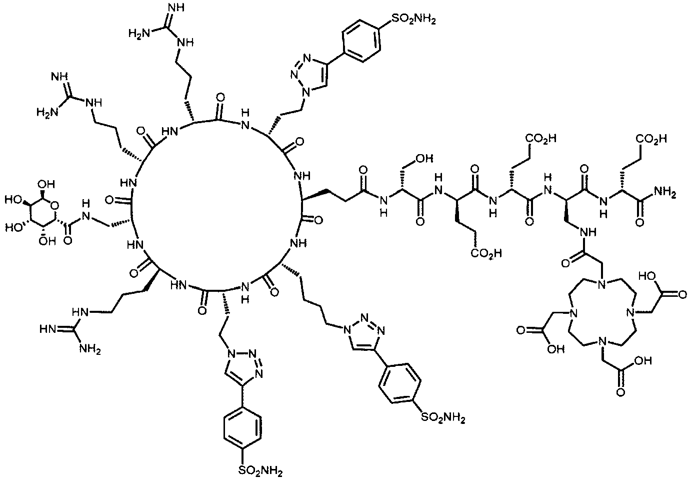

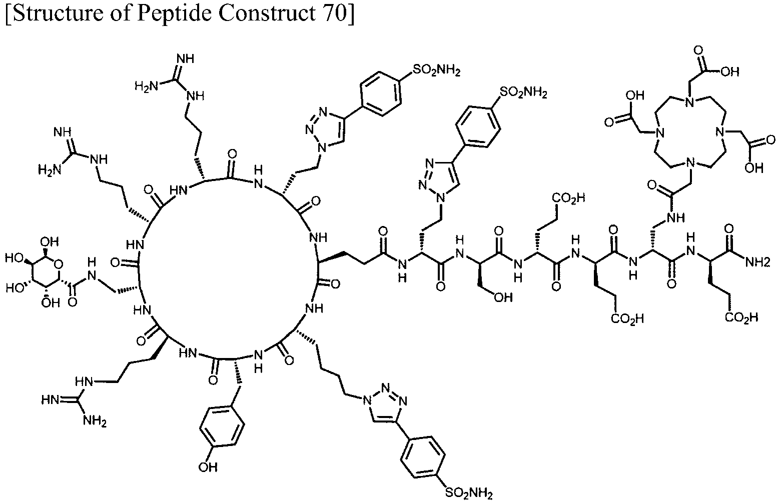

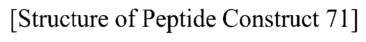

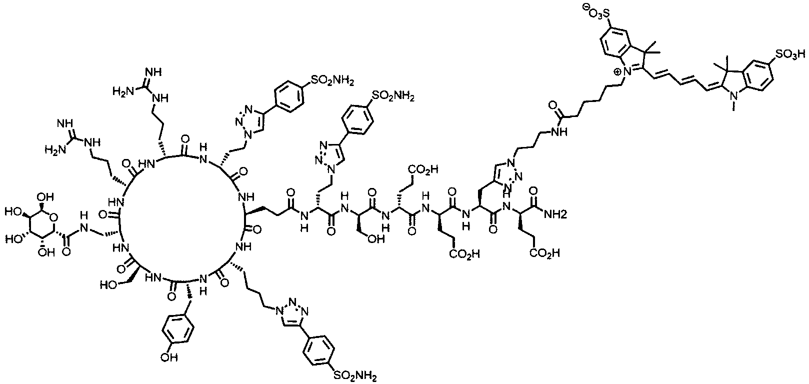

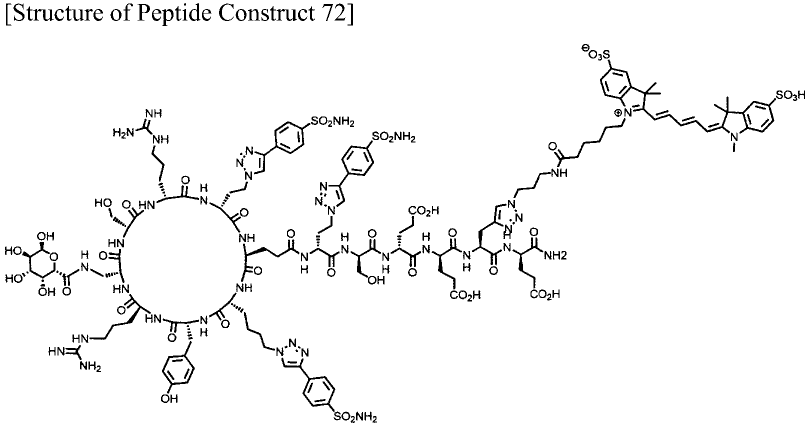

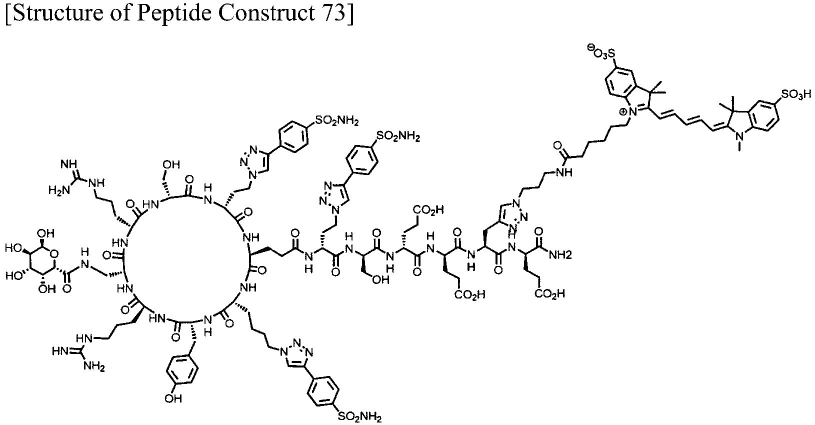

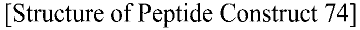

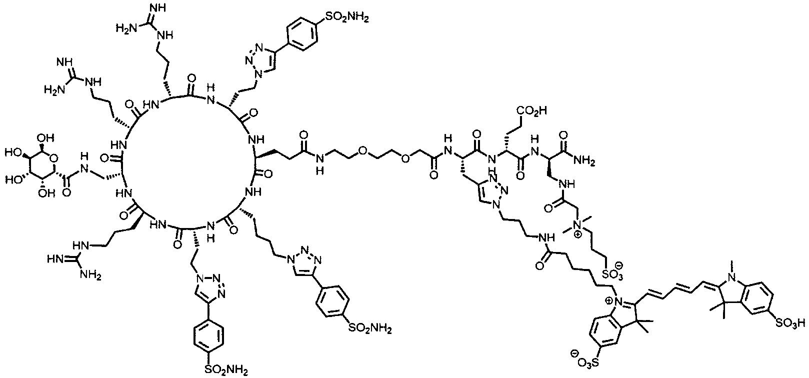

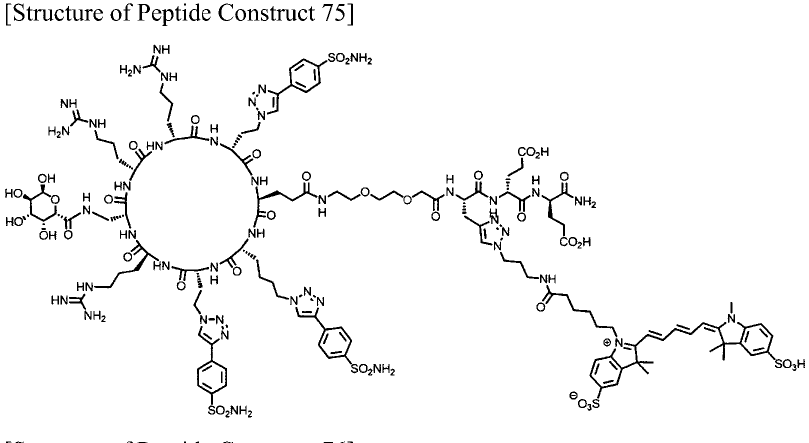

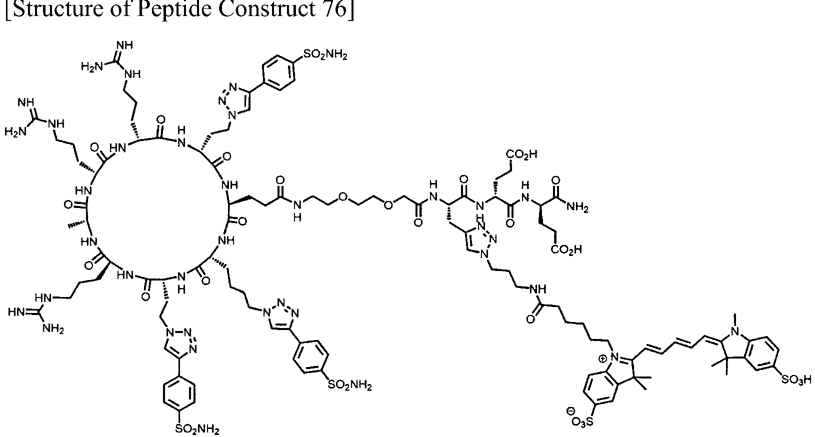

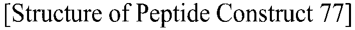

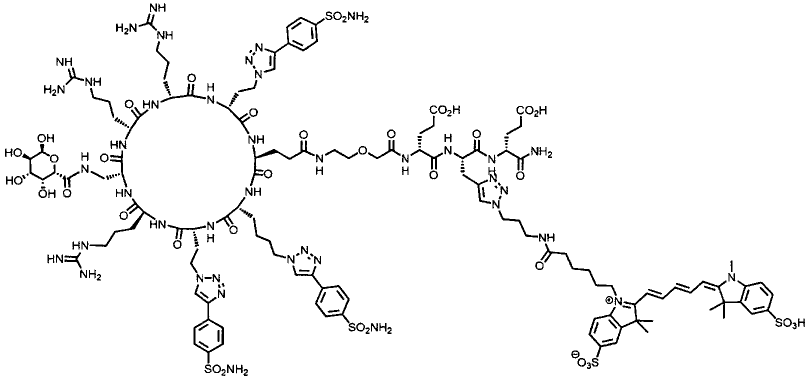

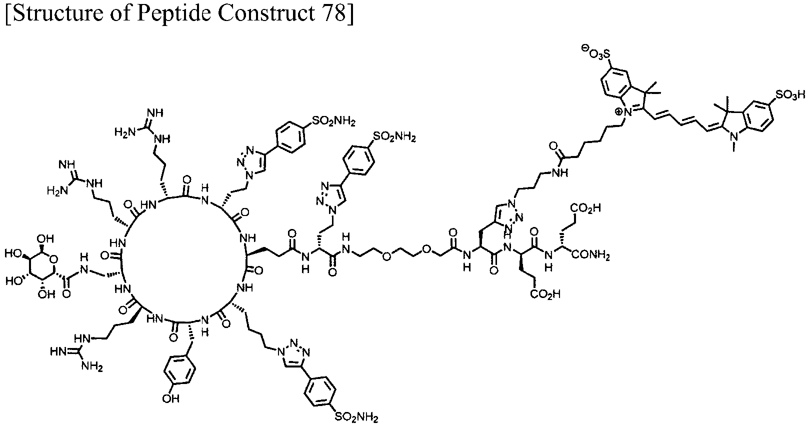

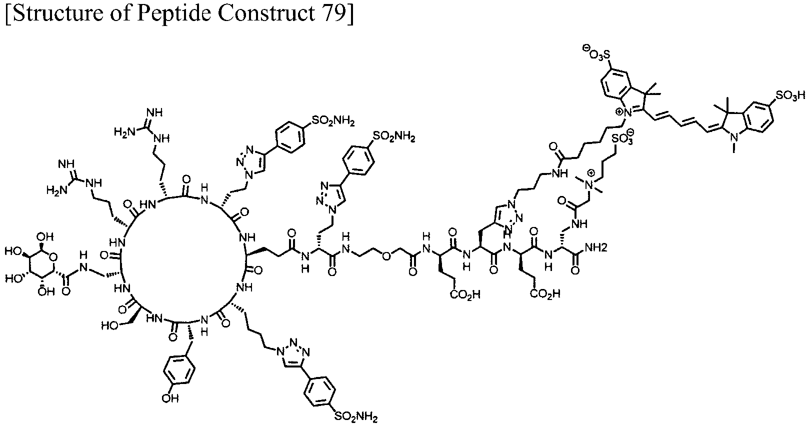

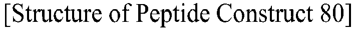

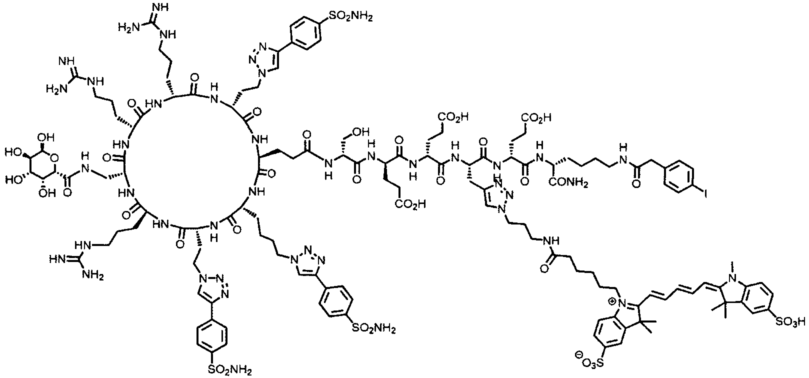

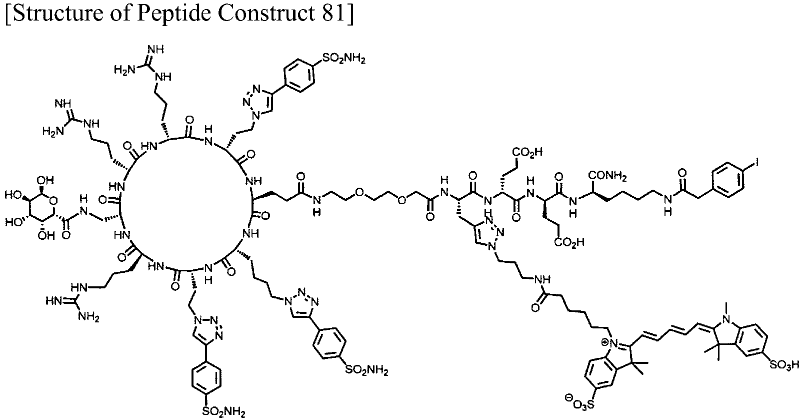

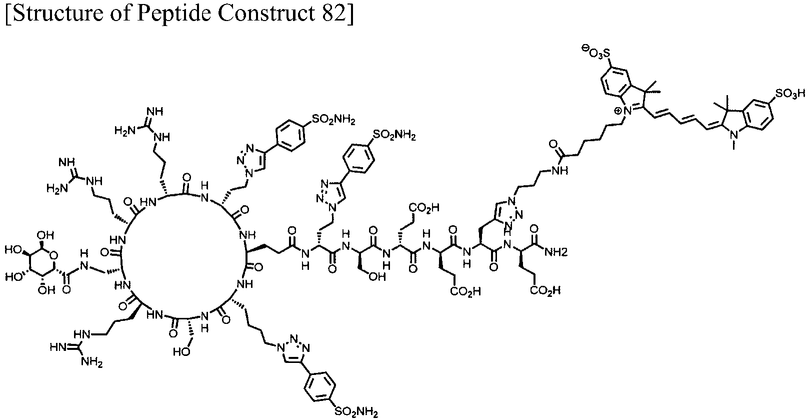

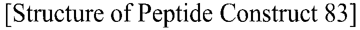

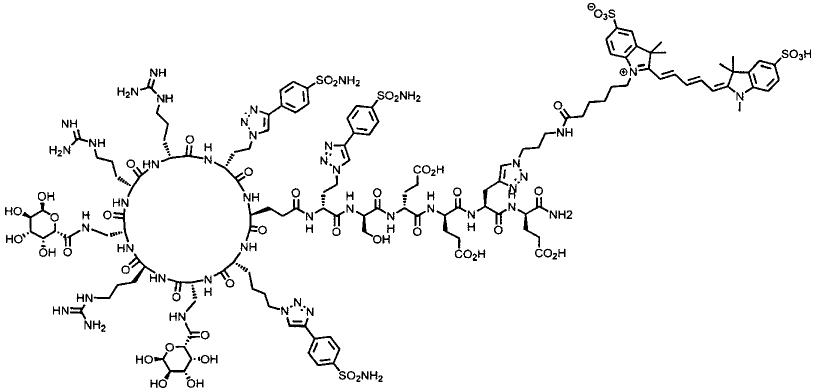

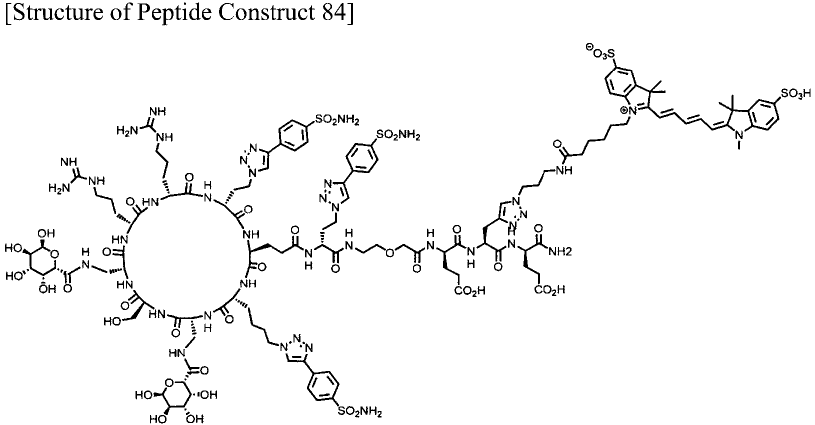

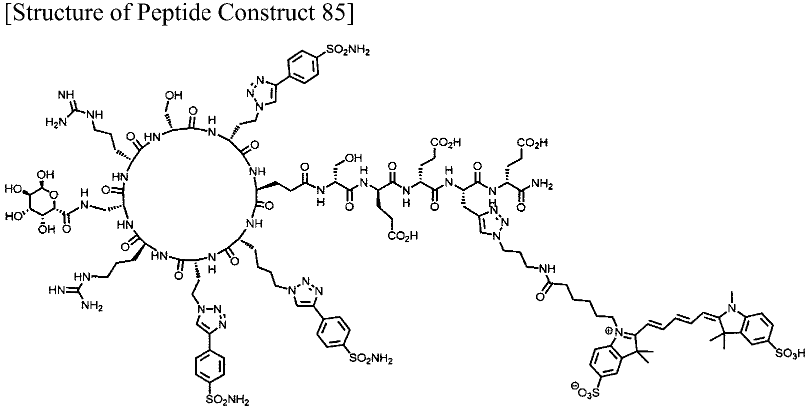

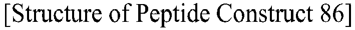

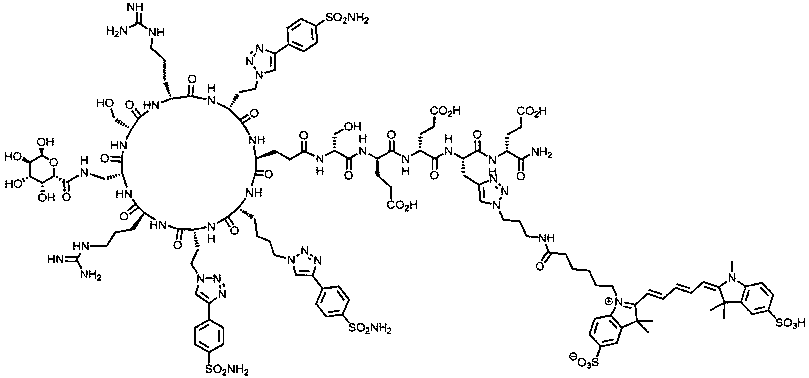

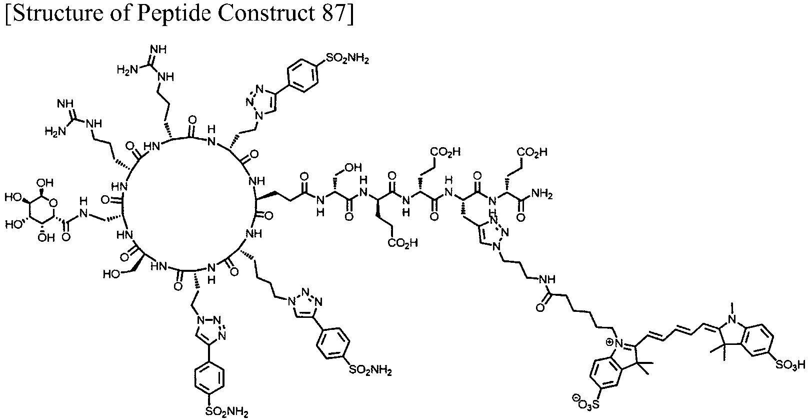

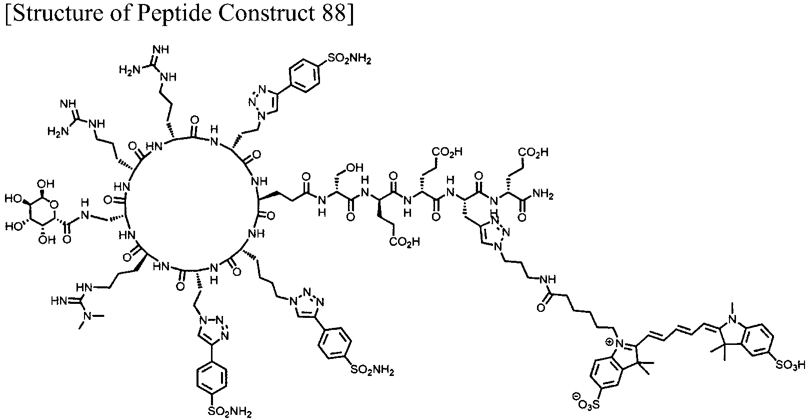

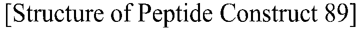

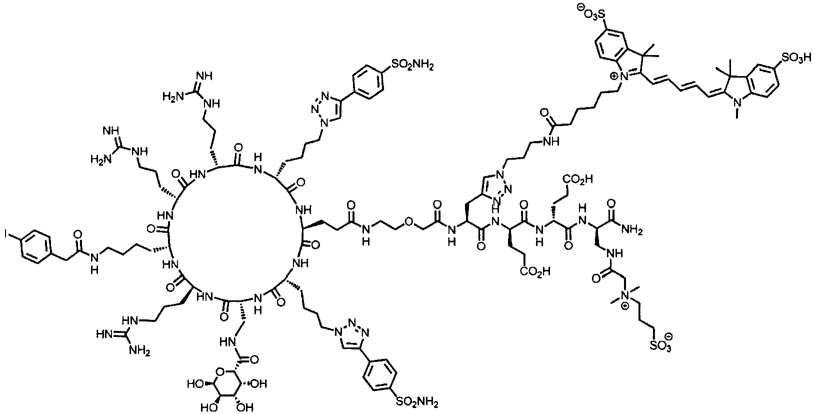

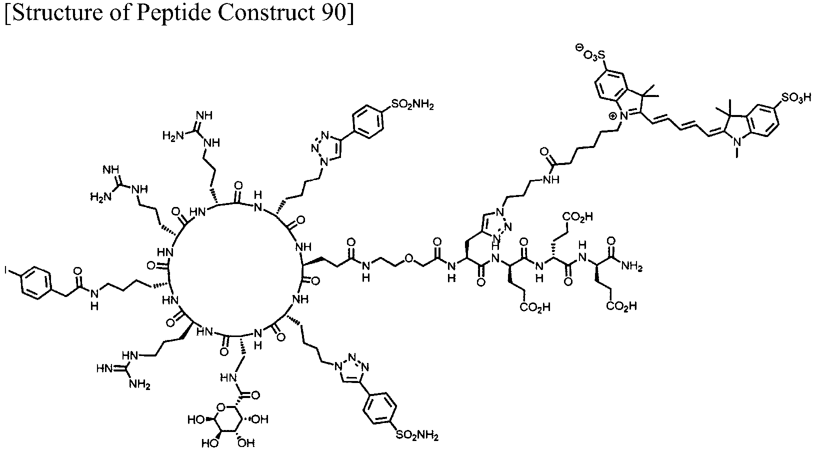

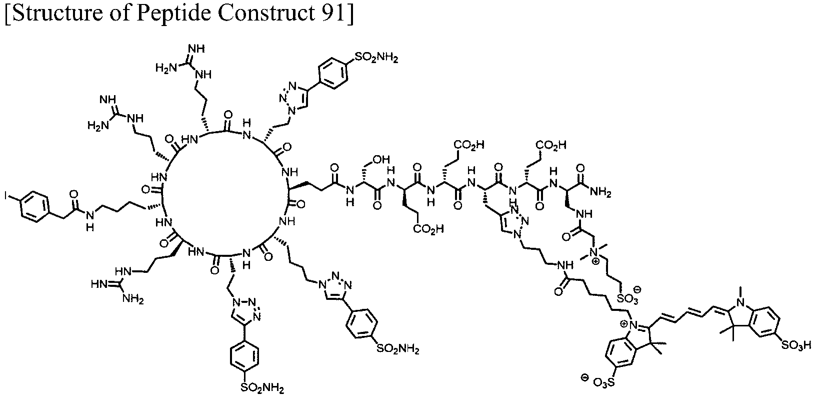

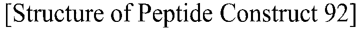

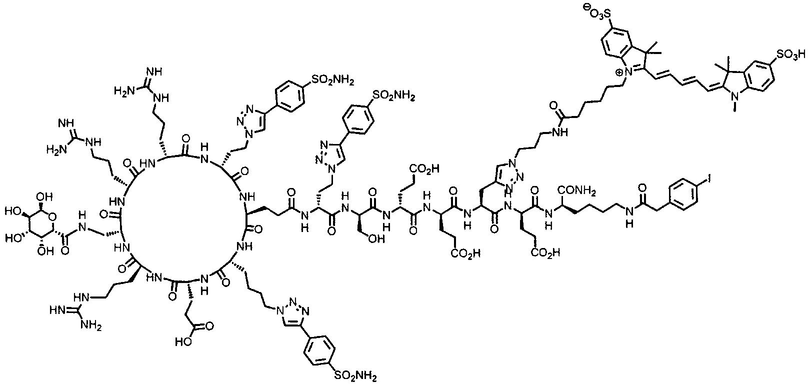

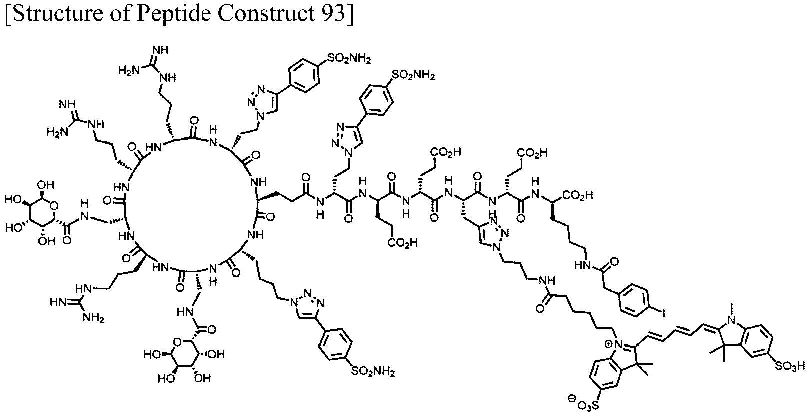

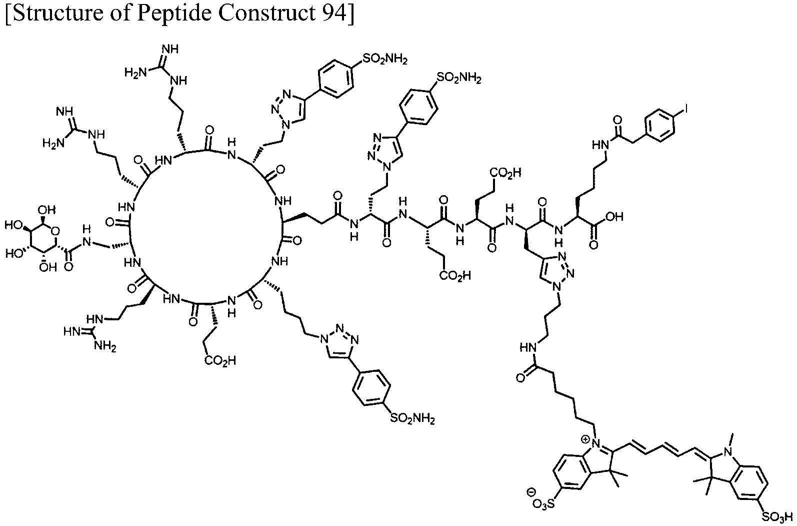

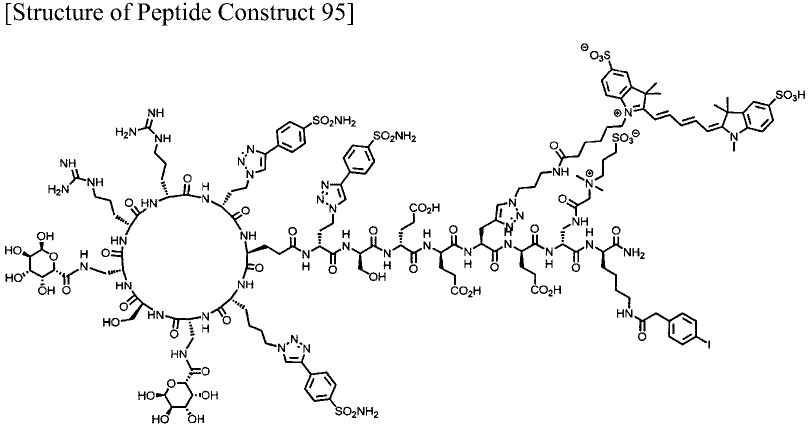

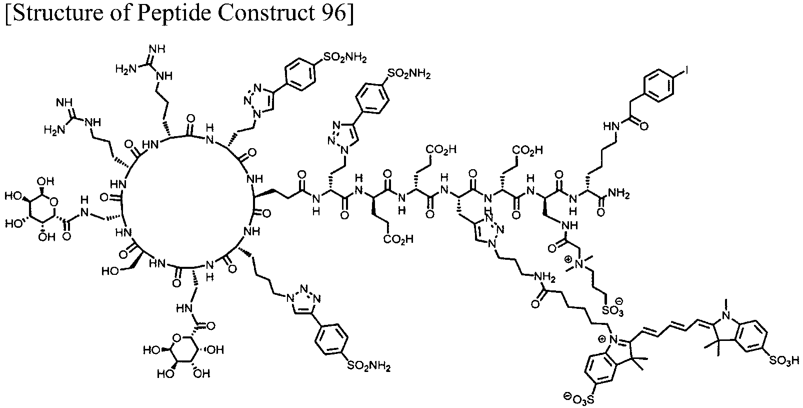

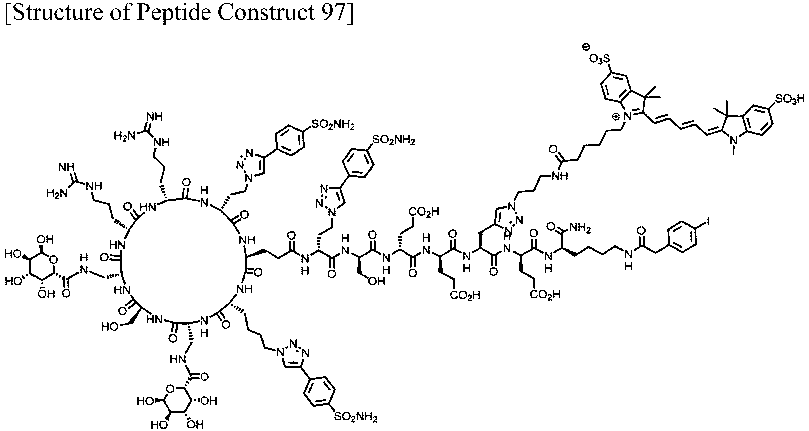

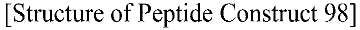

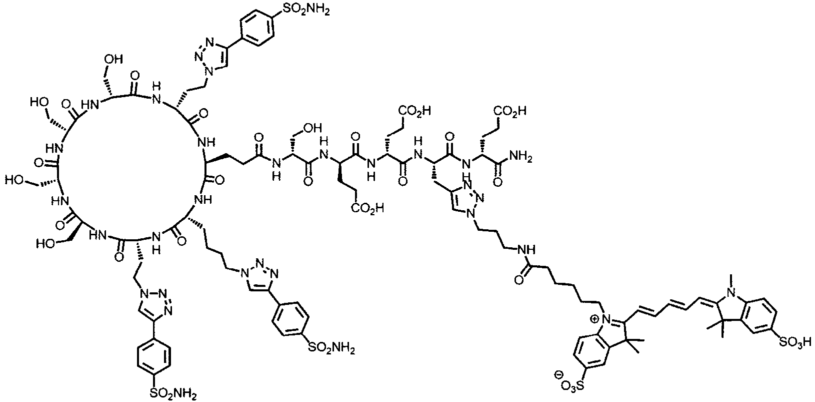

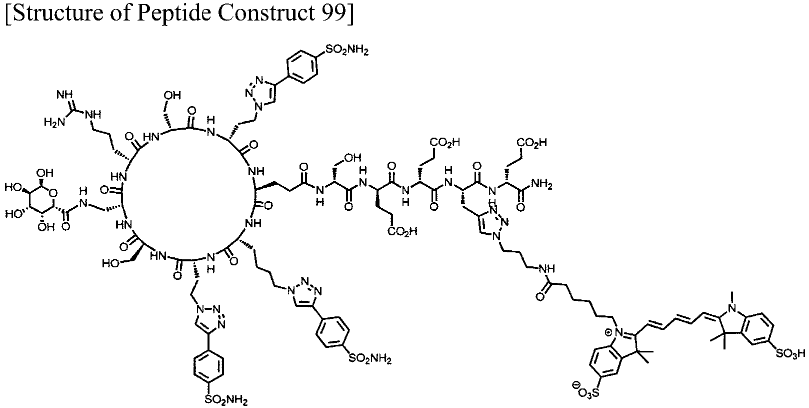

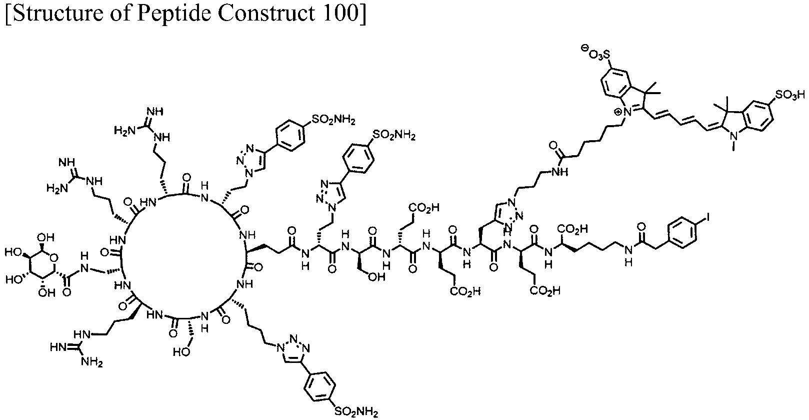

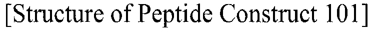

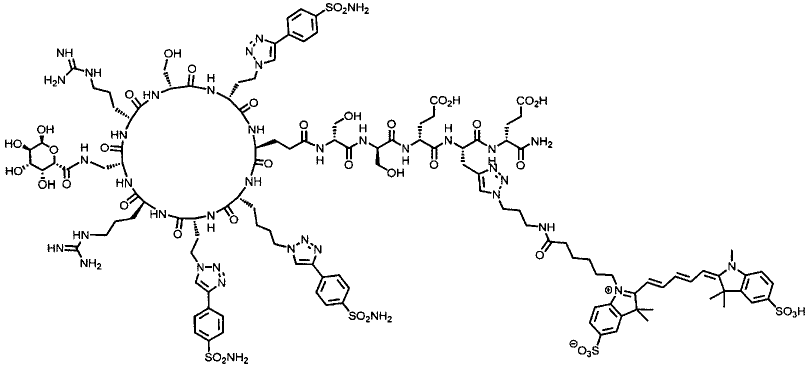

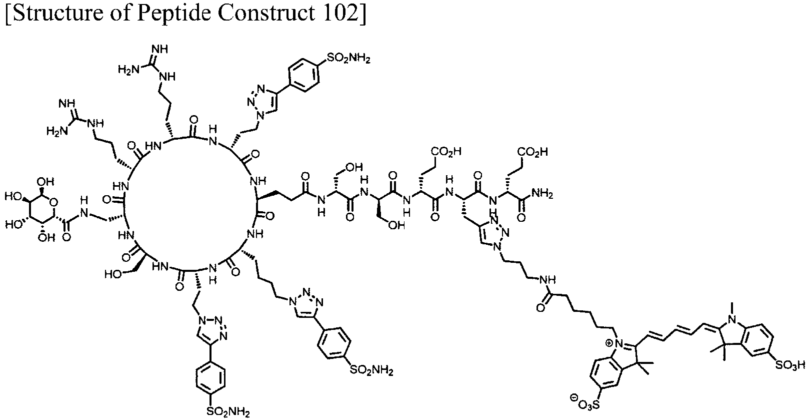

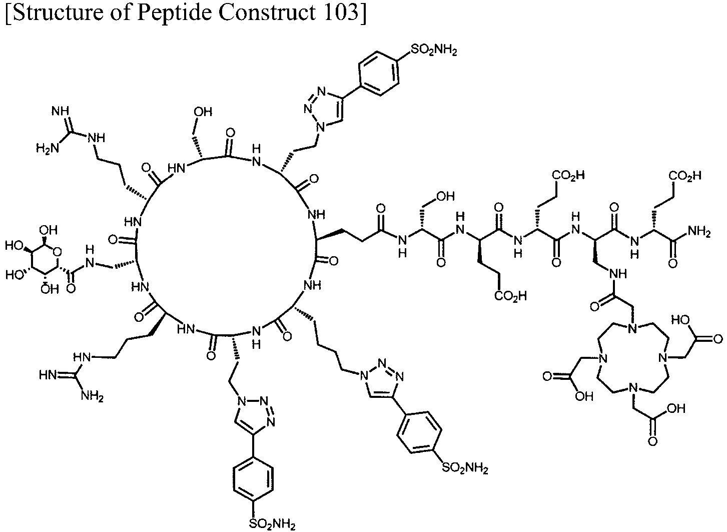

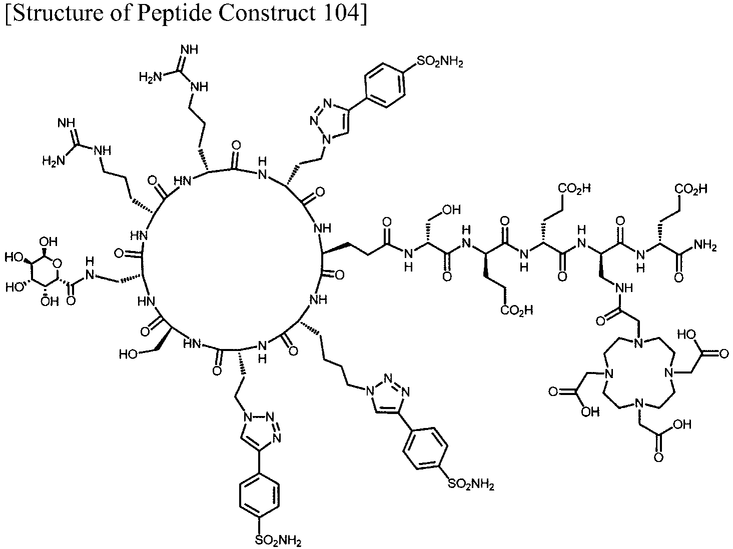

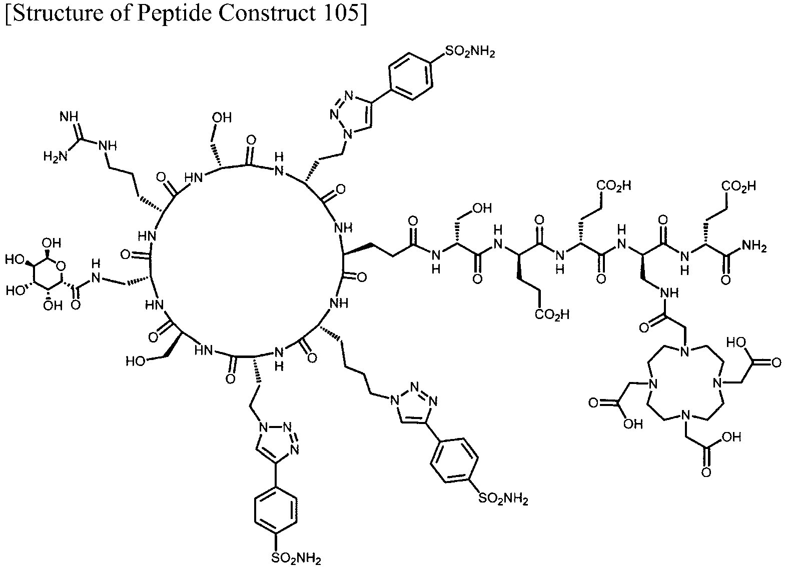

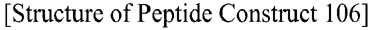

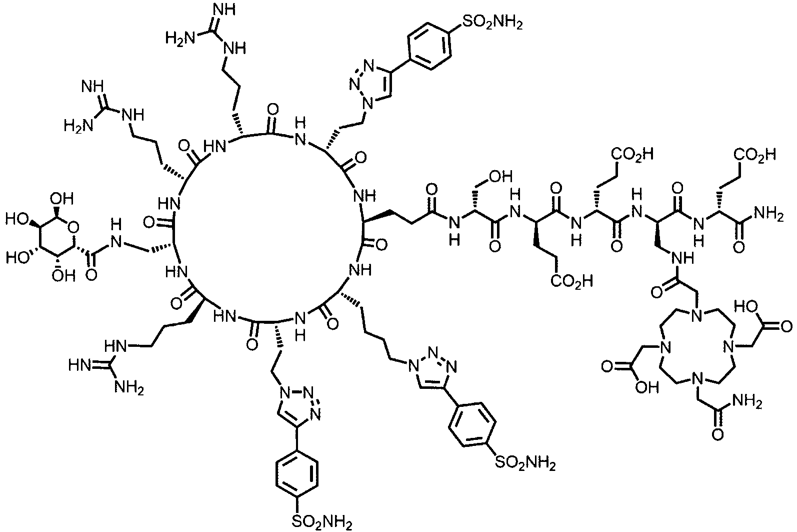

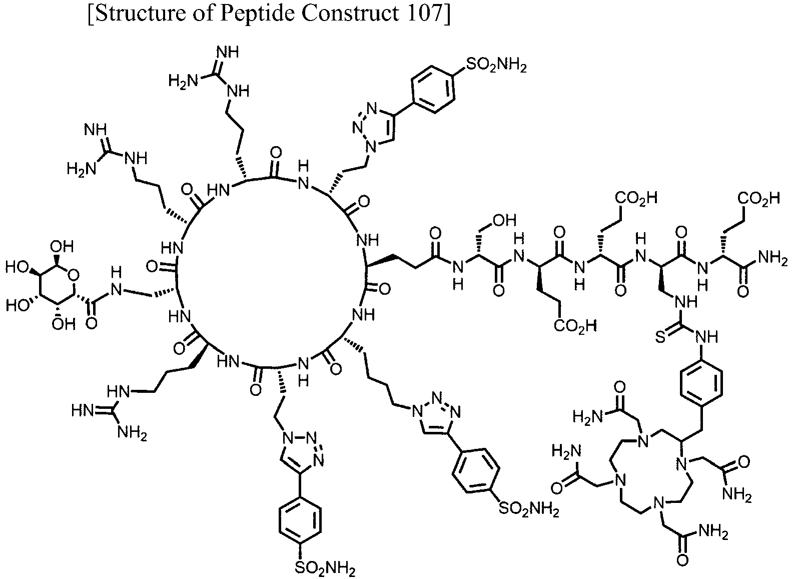

### 1-7. Labeling with Lutetium-175 (Lu-175)

For binding affinity experiments against hCAIX protein, some peptide constructs containing a DOTA linker were labeled with lutetium-175 (Lu-175) instead of isotope lutetium-177 (Lu-177).

The synthesized peptide constructs were dissolved in DMSO at a concentration of 2 mM, and lutetium-175 (LuCl₃, Cat# 450960, Sigma-Aldrich) was prepared in sodium acetate buffer (pH 5.5) at a concentration of 10 mM. To the peptide construct (1 equiv.) dissolved in DMSO, 3 equiv. of lutetium-175 was added, followed by the addition of 500 µL of sodium acetate buffer (pH 5.5), and the mixture was reacted at 90 °C for 30 min in a thermo block (Cat# B10-48, BLE). Upon completion of the reaction, the mixture was cooled to room temperature and purified using a 1260 Infinity II LC system (Agilent) under the following conditions: (1) stationary phase, Kromasil 100-5-C18 column (10 × 250 mm, 5 µm), (2) mobile phase, a mixture of ACN and triple-distilled water containing 0.1% acetic acid, (3) flow rate, 8 mL/min, (4) detection wavelengths, 214 and 254 nm. The purified peptide solution labeled with lutetium-175 was lyophilized to obtain the product in powder form.

### Example 2. Confirmation of Biological Properties of Peptide Constructs

### 2-1. Analysis of Binding Affinity and Binding Kinetics to Human Carbonic Anhydrase IX (hCAIX) Extracellular Domain (ECD)

The binding affinity and binding kinetics of the peptide constructs to the *h*CAIX ECD were analyzed. To confirm the specific binding selectivity to *h*CAIX ECD, the binding kinetics of the peptide constructs were also analyzed with respect to the extracellular domain of another isoform of carbonic anhydrase, human carbonic anhydrase XII (*h*CAXII),.

### 2-1-1. Analysis using BLITZ Instrument

Binding affinity was analyzed using the BLItz^{®} system (Cat# 45-5000, FortéBio) based on Bio-Layer Interferometry (BLI) technology, together with the advanced kinetics module of the BLItz Pro ver. 1.3 software. A streptavidin biosensor (Cat# 18-5019, Sartorius) was employed for all measurements. In addition, a protein concentration of 0 nM was used as the reference for nonspecific binding and background correction. First, 10X Octet kinetics buffer (Cat# 18-1105, Sartorius) was diluted to 1X with DPBS (Cat# L0615, Biowest) and used as the assay buffer. The assay buffer was then dispensed into a 96-well, black, and flat-bottom polypropylene (200 µL/well, Cat# 655209, Greiner Bio-One), and the streptavidin biosensors were hydrated for 10 min by immersion. The biosensors were then mounted on the instrument, and peptides were loaded at a concentration of 1 µM at 2200 rpm for 120 s. Subsequently, association and dissociation with proteins at various concentrations were performed at 2200 rpm for 120 s each. Kinetics data were analyzed using the global fitting function, and the K_{D} value was calculated as the ratio of kd to ka. Kinetic parameters were obtained by fitting baseline-corrected data to a 1:1 binding model.

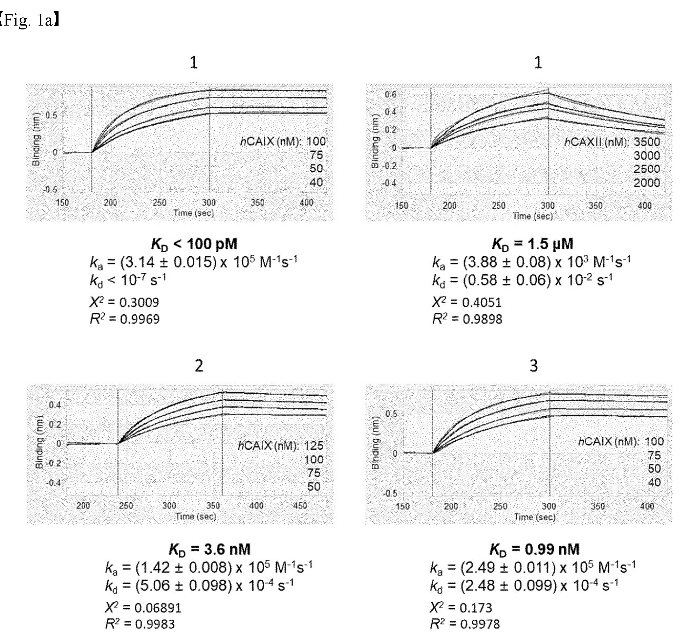

The results are shown in Fig. 1a. The curves in each graph represent experimental results obtained at different protein concentrations, with the corresponding protein concentrations indicated below each curve. For example, in Graph 2 of Fig. 1a, the topmost curve corresponds to an *h*CAIX concentration of 125 nM, while the bottommost curve corresponds to an *h*CAIX concentration of 50 nM. The peptide constructs exhibited significantly higher selectivity for *h*CAIX ECD compared with hCAXII ECD.

### 2-1-2. Analysis using Octet Instrument

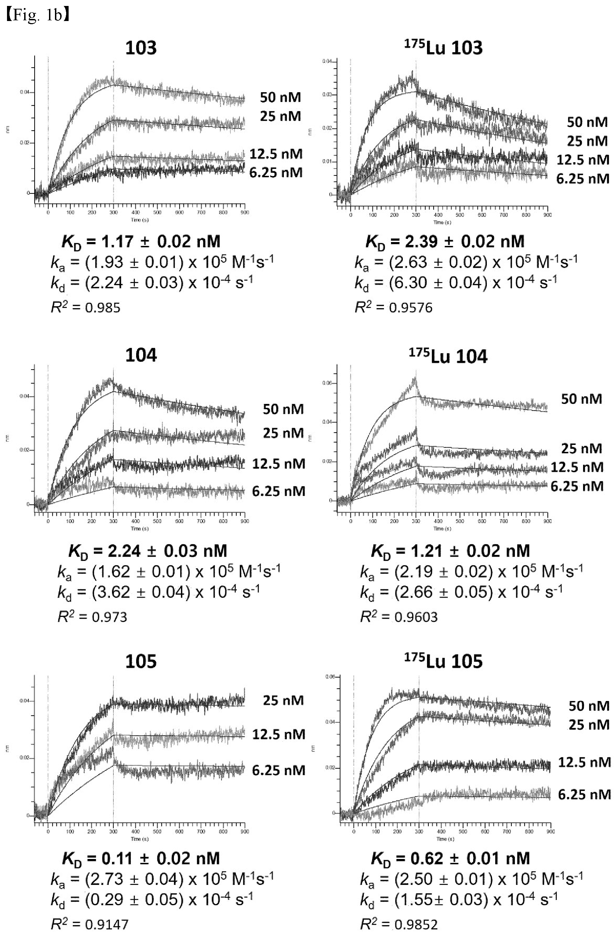

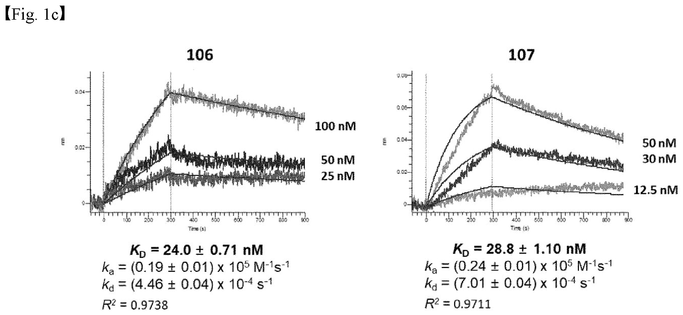

The Octet^{®}R8 system (Octet BLI Discovery 13 software), which operates on the same principle as the BLItz^{®} system, was used for analysis by attaching proteins (Cat# CA9-H82E3, ACRO Biosystems) to a streptavidin biosensor (Cat# 18-5019, Sartorius). The protein was diluted to 300 nM in DPBS (Cat# L001-02, WELGENE) and loaded for 600 s at 1000 rpm, followed by association for 300 s and dissociation for 600 s at 1000 rpm with peptides (0.5% DMSO) diluted in DPBS at each concentration. Non-specific binding of the peptides to the biosensor was confirmed to be absent at 100 nM of each peptide, and a peptide concentration of 0 nM was used as a reference for background correction. K_{D} values were obtained by the same method as in the BLItz^{®} system, and the results are shown in Figs. 1b and 1c.

### 2-2. Fluorescence-activated Cell Sorting (FACS) Analysis

In order to confirm the specific binding of the peptide constructs to target cells expressing CAIX, their *in vitro* cell-binding properties were analyzed. The human renal carcinoma cell line SK-RC-52, which expresses CAIX, was obtained from the Memorial Sloan Kettering Cancer Center (MSK, USA).

Peptide constructs labeled with the fluorescent dyes AF488 and Cy5 were quantified by measuring absorbance with a NanoPhotometer^{®} (N60, IMPLEN), and concentrations were calculated according to Lambert-Beer's law. Lyophilized peptide constructs were dissolved in dimethyl sulfoxide (DMSO) to prepare millimolar (mM) solutions, and 1.5 µL of a 100-fold dilution in PBS was measured at 495 nm for AF488 and 650 nm for Cy5 using the NanoPhotometer^{®} instrument. The molar extinction coefficient used for AF488 was 73,000 L·mol⁻¹·cm⁻¹, and for Cy5 was 271,000 L·mol⁻¹·cm⁻¹. The quantified peptide constructs were dissolved in DMSO to prepare a 1 mM solution and diluted to 5 nM or 10 nM using a mixture of Dulbecco's Modified Eagle Medium (DMEM) supplemented with 1% fetal bovine serum (FBS) (FACS assay buffer).

The SK-RC-52 cell line was seeded into T75 flasks (1.0-1.5 × 10⁶ cells) or T175 flasks (5.0-8.0 × 10⁶ cells) two to three days prior to FACS analysis. On the day of the experiment, when the SK-RC-52 cells had reached approximately 80% confluency, the culture medium was removed from the flasks, and the cells were washed twice with 10 mL of PBS buffer. The cells were treated with 1 mL of Trypsin-EDTA for 5 min in a humidified incubator (37 °C, 5% CO₂, Galaxy 170 S), after which the Trypsin-EDTA was neutralized with 9 mL of culture medium. The neutralized cell mixture, together with an additional 10 mL of culture medium, was transferred to a 50 mL tube and centrifuged at 1000 rpm for 3 min using a VARISPIN 15R centrifuge. The supernatant of the centrifuged cell mixture was removed, and the cells were diluted with fresh culture medium to 5 × 10⁵ cells per 200 µL. Then, 200 µL of the mixture was dispensed into each well of a 96-well round-bottom plate (Cat# 34096, SPL Life Science). The plate was centrifuged at 1000 rpm for 3 min using a VARISPIN 15R centrifuge, after which the supernatant was removed from each well. A 200 µL of the pre-prepared peptide construct solution (5 or 10 nM) was then added, and the plate was then incubated in a humidified incubator. After 1 h, the plates were centrifuged at 1000 rpm for 3 min using a VARISPIN 15R centrifuge, the supernatant was removed, and the cells were washed with 200 µL of FACS buffer. Following washing, 200 µL of FACS buffer was added to each well, mixed with the cells, and transferred to 5 mL round-bottom tubes equipped with cell strainers (Cat# 352235, CORNING). Fluorescence intensity was measured for each cell using a BD Accuri C6 Plus (BD Biosciences), and the acquired data were analyzed with FlowJo 10.7.1 (BD Biosciences).

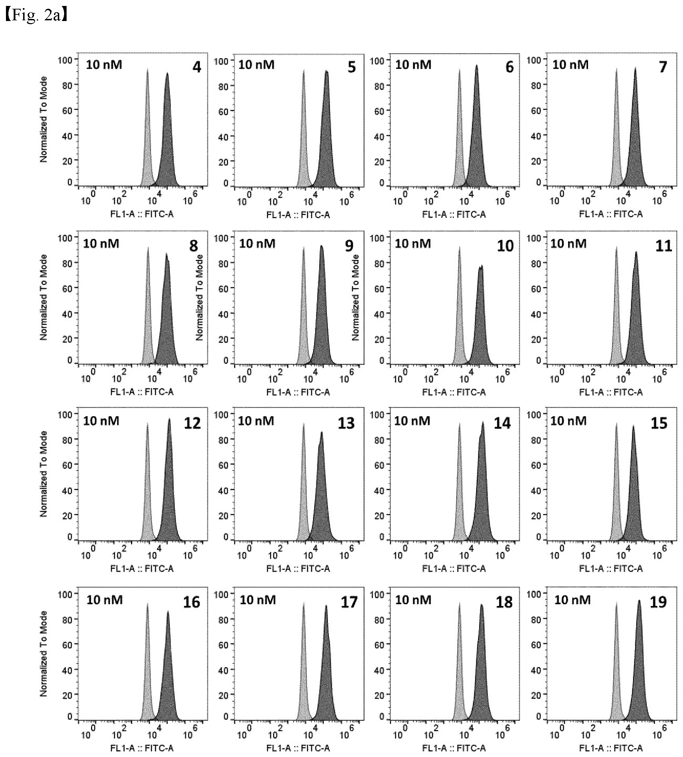

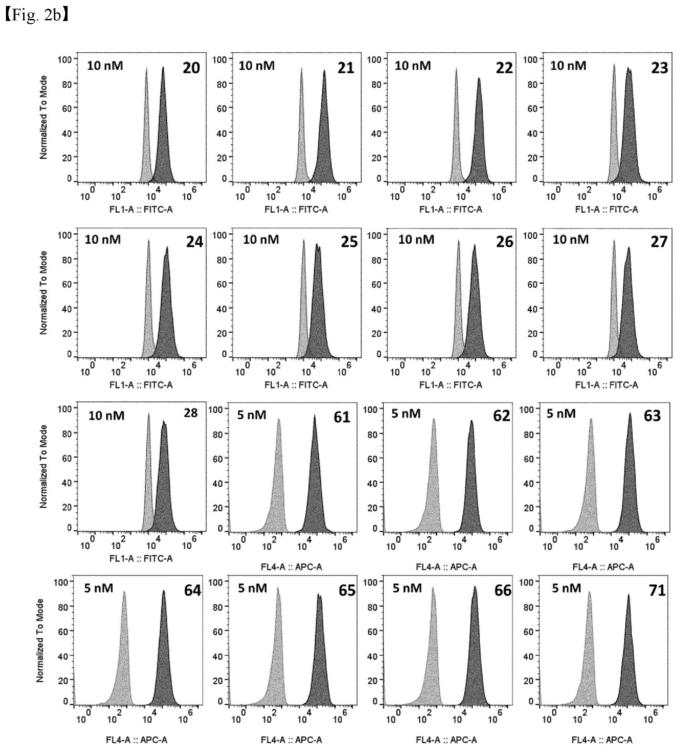

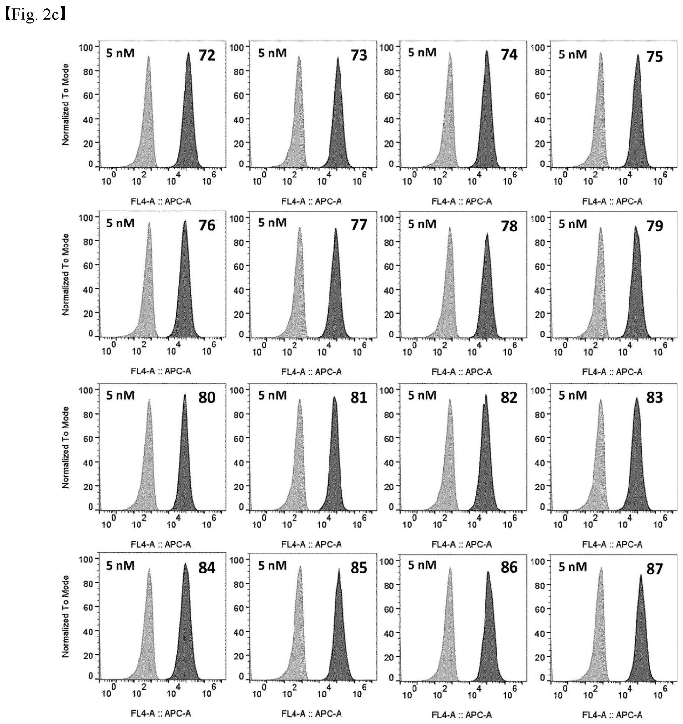

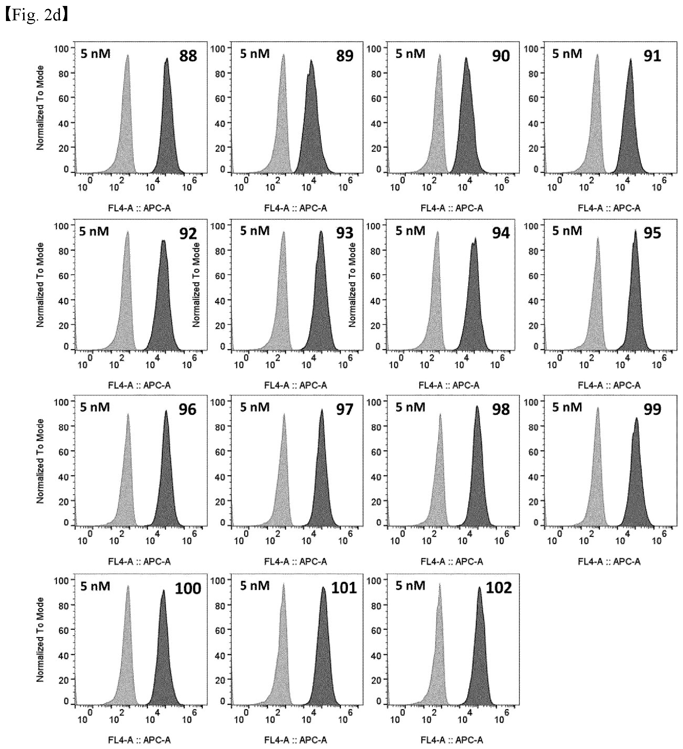

The results of FACS analysis using the SK-RC-52 cell line are shown in Figs. 2a-2d. Based on the FACS results, it was confirmed that the peptide constructs of the present invention exhibit high affinity for target cells expressing CAIX.

### 2-3. Biodistribution Analysis Using Animal Models

Six-week-old female BALB/c nude mice (OrientBio or HanaBio, Korea) were used. SK-RC-52 cells (2 × 10⁶ or 5 × 10⁶) were subcutaneously injected into the upper region of the right forelimb. Tumor size was measured two to three times per week beginning 5 days after injection, and tumor volume was calculated using a caliper according to the formula [volume = (width² × length)/2].

When the tumors of SK-RC-52 xenografts reached 90-120 mm³, peptide constructs (10 µM, 200 µL; 1% DMSO in PBS) were administered via tail vein injection, and *ex vivo* imaging was performed 24 h after injection(Figs. 3a-3c). Each peptide construct was tested in two mice, and those administered to more than two mice are shown in Table 1.

**[Table 1]**

| | | | | | | | |
|---|---|---|---|---|---|---|---|
| Peptide No. | 33 | 35 | 36 | 37 | 40 | 42 | 43 |
| The number of mice used | 6 | 3 | 4 | 3 | 6 | 4 | 4 |

For repeated experimentations, seven Peptide Constructs (31, 33, 39, 40, 43, 45, and 46) that showed high tumor signals in Figs. 3a-3c were each administered to three mice, and *ex vivo* imaging was performed 24 h after injection (Fig. 3d). In addition, three Peptide Constructs (33, 39, and 40) were each administered to four mice, and *ex vivo* imaging was performed 72 h after injection (Fig. 3e).

6 Peptide Constructs (55, 56, 57, 58, 59, and 60) were each administered to 2 mice by tail vein injection at a dose of 5 µM in a volume of 200 µL (1% DMSO in PBS). *ex vivo* imaging was performed 72 h after injection to confirm biodistribution (Fig. 3f). Among these, Peptide Constructs 58 and 60, which exhibited high fluorescence intensity, were each administered to three mice by tail vein injection at a dose of 10 µM in a volume of 200 µL (1% DMSO in PBS), and *ex vivo* imaging was performed on day 5 to confirm biodistribution (Fig. 3g).

4 Peptide Constructs (61, 62, 63, and 64) were each administered to groups of 5 mice by tail vein injection at a dose of 5 µM in a volume of 200 µL (5% DMSO in PBS). Whole-body fluorescence imaging was performed 24 h after injection, followed by sacrifice of the mice and *ex vivo* imaging to confirm biodistribution (Figs. 3h and 3i).

14 Peptide Constructs (65, 75, 80, 83, 84, 85, 87, 89, 90, 91, 92, 97, 98, and 99), derived from Constructs 63 and 64 that exhibited high fluorescence intensity in the tumor and low fluorescence intensity in the kidney, were each administered to groups of five mice by tail vein injection at a dose of 5 µM in a volume of 200 µL (5% DMSO in PBS). Whole-body fluorescence imaging was performed 24 or 72 h after injection, followed by sacrifice of the mice and *ex vivo* imaging to confirm biodistribution (Figs. 3j to 3m).

For biodistribution confirmation, the mice were sacrificed by carbon dioxide inhalation, followed by dissection, and the tumors and organs (spleen, lungs, heart, liver, kidneys, stomach, pancreas, small intestine, and large intestine) were excised and subjected to *ex vivo* imaging using the *in vivo* imaging system AMI HTX (Spectral Instruments Imaging). The results were obtained by applying the same area (0.08 cm²), corresponding to the smallest tumor size, to the regions of highest fluorescence intensity in the tumor, kidney, liver, spleen, lung, and heart. This analysis was performed using Aura imaging software (Spectral Imaging, USA), and the fluorescence intensity was expressed in units of photons/s/cm²/sr. It was confirmed that some constructs specifically binding to tumors overexpressing CAIX remained localized in the tumors even after 72 h, compared to other organs.

### 2-4. In vivo SPECT/CT Imaging Experiments of Peptide Constructs Labeled with the Isotope ¹⁷⁷Lu

To confirm imaging diagnostic and therapeutic functionalities of Peptide Constructs 67, 68, 69, 70, 103, 104, and 105, each construct was radiolabeled with as follows. A 1 mM stock solution of each peptide construct was prepared by dissolving it in DMSO. 30 mCi of isotope (¹⁷⁷LuCl₃, ITM Medical Isotopes GmbH) diluted in 0.04 M HCl was evaporated to dryness under a nitrogen stream. The residue was dissolved in 200 µL of 0.2 M sodium acetate buffer (pH 5.5), and 7 mCi (approximately 50 µL) of this isotope solution was then mixed with 7 µL of the peptide stock solution. The mixture was incubated at 90 °C for 30 min to achieve radiolabeling. After completion, the reaction mixture was cooled to room temperature and purified using an Agilent 1200 Infinity LC system under the following conditions: (1) stationary phase: Agilent Poroshell 120 EC-C18 column (4.6 × 50 mm, 2.7 µm); (2) mobile phase: a mixture of triple-distilled water and ACN; (3) flow rate: 1 mL/min; and (4) detection wavelengths: 254 nm and 214 nm.

The purified ¹⁷⁷Lu-labeled peptide constructs were processed to remove organic solvents using a Sep-Pak C18 column (Cat# 186005125, Waters). The purified peptides were diluted fivefold with distilled water, loaded onto an activated Sep-Pak column (5 mL ethanol and 5 mL distilled water), washed with 5 mL distilled water to remove residual organic solvents, eluted with 300 µL of 60% ethanol, and diluted with saline for subsequent use.

When tumors derived from implantation of the human renal carcinoma cell line SK-RC-52 reached an average volume of approximately 120 mm³, 200 µL of ¹⁷⁷Lu-labeled peptide constructs corresponding to a radioactivity of 500 µCi was administered once via tail vein injection in mice. SPECT/CT (Siemens Inveon, software: Inveon Acquisition Workplace) imaging was performed at 1.5 h, 1, 2, 3, 7 or 8, and 10 days for constructs 67, 68, 69, and 70, and at 1.5 h, 1, 2, and 3 days for constructs 103, 104, and 105. For image acquisition, mice were anesthetized by inhalation with 0.2% isoflurane in oxygen for 3-5 min, placed in the instrument, and the anesthetic was adjusted to be continuously administered at 1.5 L/min during 40 min of SPECT and 5 min of CT measurements. The images were acquired using Inveon Research Workplace 4.2.

The SPECT/CT images obtained as described above are in Figures 4a and 5a. It was confirmed that the peptide construct labeled with the radioisotope ¹⁷⁷Lu binds to the carcinoma cell SK-RC-52.

### 2-5. In vivo SPECT/CT Imaging Experiments of Peptide Constructs Labeled with the Isotope ¹⁷⁷Lu

To evaluate the anticancer effect of Peptide Constructs 67, 68, 69, 70, 103, 104, and 105 with labeled with the isotope ¹⁷⁷Lu, six-week-old female BALB/c nude mice (OrientBio or HanaBio, Korea) were used. SK-RC-52 cells (5 × 10⁶) were subcutaneously injected into the upper right forelimb of the nude mice. After cell injection, grouping was carried out with at least 4 animals per group such that the average tumor volume of each group corresponded to approximately 120 mm³. Tumor volume was measured using a caliper according to the formula: [volume = (width)² × (length)/2].

After the SK-RC-52 cell lines were xenotransplanted and grouping was carried out, a control group was administered saline solution, and each of the Peptide Constructs (67, 68, 69, 70, 103, 104, and 105) labeled with ¹⁷⁷Lu was intravenously injected via the tail vein at a dose of about 500 µCi. Body weight and tumor size were measured two to three times per week.

The results are shown in Fig. 4b, Fig. 4c, Fig. 5b, and Fig. 5c, respectively. As shown in these figures, the ¹⁷⁷Lu-labeled Peptide Constructs 67, 68, 69, 70, 103, 104, and 105 exhibited anticancer effects that significantly reduced the size of CAIX-overexpressing tumors without causing substantial changes in the body weight of the mice.

The foregoing description of the present invention is provided for illustrative purposes only, and it will be understood by those skilled in the art that the scope of the present invention is not limited to the disclosed embodiments and accompanying drawings, but may be variously modified into other specific forms without departing from the spirit and scope of the present invention.

### Sequence List

**[Table 2]**

| Seq ID No. | Amino Acid Sequences |
|---|---|
| 1 | YRFRR |
| 2 | RFRR |
| 3 | RARR |
| 4 | YRARR |
| 5 | ARFRR |
| 6 | ARARR |
| 7 | RXRR (wherein, X=DAP(Gal)) |
| 8 | RXRR (wherein, X=Uk(IB)) |
| 9 | RNRR |
| 10 | AAAA |
| 11 | RERR |
| 12 | YRXRR (wherein, X=DAP(Gal)) |
| 13 | RX1RX2 (wherein, X1=DAP(Gal), X2=r(dimet)) |
| 14 | RX1X2R (wherein, X1=DAP(Gal), X2=r(dimet)) |
| 15 | YSXRR (wherein, X=DAP(Gal)) |
| 16 | YRXSR (wherein, X=DAP(Gal)) |
| 17 | YRXRS (wherein, X=DAP(Gal)) |
| 18 | SRXRR (wherein, X=DAP(Gal)) |
| 19 | XRXRR (wherein, X=DAP(Gal)) |
| 20 | XSXRR (wherein, X=DAP(Gal)) |
| 21 | RXRS (wherein, X=DAP(Gal)) |
| 22 | RXSR (wherein, X=DAP(Gal)) |
| 23 | SXRR (wherein, X=DAP(Gal)) |
| 24 | X1X2RR (wherein, X1=R(dimet), X2=DAP(Gal)) |
| 25 | X1RX2RR (wherein, X1=DAP(Gal), X2=Uk(sIB)) |
| 26 | RXRR (wherein, X=Uk(sIB)) |
| 27 | ERXRR (wherein, X=DAP(Gal)) |
| 28 | SSSS |
| 29 | SXRS (wherein, X=DAP(Gal)) |
| 30 | SEX1EEX2 (wherein X1=Uk(DOTA), X2=K(Biotin)) |
| 31 | SEX1EEX2 (wherein, X1=Uk(DOTA), X2=Pra(AF488)) |
| 32 | SX1X2EEX3 (wherein, X1=Gla, X2=DAP(DOTA), X3=Pra(AF488)) |
| 33 | SEX1EEX2 (wherein, X1=DAP(DOTA), X2=Pra(AF488)) |
| 34 | SX1X2EX3X4 (wherein, X1=Gla, X2=DAP(DOTA), X3=DAP(SZW), X4=Pra(AF488)) |
| 35 | SEX1EEX2X3 (wherein, X1=DAP(DOTA), X2=DAP(SZW), X3=Pra(AF488)) |
| 36 | EX1X2GVPTLQ (wherein, X1=Gla, X2=Pra(AF488)) |
| 37 | EX1X2EGRGDK (wherein, X1=Gla, X2=Pra(AF488)) |
| 38 | EX1X2KLPVM (wherein, X1=Gla, X2=Pra(AF488)) |
| 39 | EX1X2EGKLPVM (wherein, X1=Gla, X2=Pra(AF488)) |
| 40 | SX1X2EX3X4 (wherein, X1=Gla, X2=DAP(DOTA), X3=DAP(SZW), X4=Pra(Cy5)) |
| 41 | SX1X2EX3 (wherein, X1=Gla, X2=DAP(DOTA), X3=Pra(Cy5)) |
| 42 | SX1X2EEX3 (wherein, X1=Gla, X2=DAP(DOTA), X3=Pra(Cy5)) |
| 43 | EX1X2EEX3 (wherein, X1=Gla, X2=DAP(DOTA), X3=Pra(Cy5)) |
| 44 | SX1X2EX3X4 (wherein, X1=Gla, X2=DAP(DOTA), X3=Uk(IB), X4=Pra(Cy5)) |
| 45 | EX1X2EX3X4 (wherein, X1=Gla, X2=DAP(DOTA), X3=Uk(IB), X4=Pra(Cy5)) |
| 46 | SX1X2X1X3X4 (wherein, X1=Gla, X2=DAP(DOTA), X3=Uk(IB), X4=Pra(Cy5)) |
| 47 | EX1X2EX3X4 (wherein, X1=Gla, X2=DAP(DOTA), X3=DAP(6OH), X4=Pra(Cy5)) |
| 48 | SX1X2EVPTLQ (wherein, X1=Gla, X2=Pra(Cy5)) |
| 49 | SX1X2EERGDK (wherein, X1=Gla, X2=Pra(Cy5)) |
| 50 | EX1X2GVPTLQ (wherein, X1=Gla, X2=Pra(Cy5)) |
| 51 | EX1X2EGRGDK (wherein, X1=Gla, X2=Pra(Cy5)) |
| 52 | EX1X2KLPVM (wherein, X1=Gla, X2=Pra(Cy5)) |
| 53 | EX1X2EGKLPVM (wherein, X1=Gla, X2=Pra(Cy5)) |
| 54 | SEEX1EX2 (wherein, X1=Pra(Cy5), X2=DAP(SZW)) |
| 55 | SEEX1EX2X3 (wherein, X1=Pra(Cy5), X2=DAP(SZW), X3=Uk(IB)) |
| 56 | SEEXE (wherein, X=Pra(Cy5)) |
| 57 | SEEX1EX2 (wherein, X1=DAP(DOTA), X2=DAP(SZW)) |
| 58 | SEEX1EX2X3 (wherein, X1=DAP(DOTA), X2=DAP(SZW), X3=Uk(IB)) |
| 59 | SEEXE (wherein, X1=DAP(DOTA)) |
| 60 | EX1EX2 (wherein, X1=Pra(Cy5), X2=DAP(SZW)) |
| 61 | SEEX1EX2 (wherein, X1=Pra(Cy5), X2=Uk(sIB)) |
| 62 | X1EEX2 (wherein, X1=Pra(Cy5), X2=Uk(sIB)) |
| 63 | X1EEX2 (wherein, X1=Pra(Cy5), X2=DAP(SZW)) |
| 64 | X1EEE (wherein, X1=Pra(Cy5)) |
| 65 | EEX1EX2* (wherein, X1=Pra(Cy5), X2=Uk(sIB)) |
| 66 | EEX1X2* (wherein, X1=Pra(Cy5), X2=Uk(sIB)) |
| 67 | SEEX1EX2X3 (wherein, X1=Pra(Cy5), X2=DAP(SZW), X3=Uk(sIB)) |
| 68 | EEX1EX2X3 (wherein, X1=Pra(Cy5), X2=DAP(SZW), X3=Uk(sIB)) |
| 69 | SEEX1EX2* (wherein, X1=Pra(Cy5), X2=Uk(sIB)) |
| 70 | SSEXE (wherein, X=Pra(Cy5)) |
| 71 | SEEXE (wherein, X=DAP(PSC)) |
| 72 | SEEXE (wherein, X=DAP(TCMC)) |
| 73 | X1YRFRRX2EX2 (wherein, X1=Uk(SFA), X2=Aha(SFA)) |
| 74 | X1X2RFRRX2E (wherein, X1=Uk(SFA), X2=Aha(SFA)) |
| 75 | X1X2RARRX2E (wherein, X1=Uk(SFA), X2=Aha(SFA)) |
| 76 | XXRARRXE (wherein, X=Aha(SFA)) |
| 77 | XYRFRRXEX (wherein, X=Aha(SFA)) |
| 78 | X1YRARRX2EX2 (wherein, X1=Uk(SFA), X2=Aha(SFA)) |
| 79 | X1ARFRRX2EX2 (wherein, X1=Uk(SFA), X2=Aha(SFA)) |
| 80 | X1RFRRX2EX2 (wherein, X1=Uk(SFA), X2=Aha(SFA)) |
| 81 | X1ARARRX2EX2 (wherein, X1=Uk(SFA), X2=Aha(SFA)) |
| 82 | X1RARRX2EX2 (wherein, X1=Uk(SFA), X2=Aha(SFA)) |
| 83 | X1X2RX3RRX2E (wherein, X1=Uk(SFA), X2=Aha(SFA), X3=DAP(Gal))) |
| 84 | X1X2RX3RRX2E (wherein, X1=Uk(SFA), X2=Aha(SFA), X3=Uk(IB)) |
| 85 | X1X2RNRRX2E (wherein, X1=Uk(SFA), X2=Aha(SFA)) |
| 86 | X1X2AAAAX2E (wherein, X1=Uk(SFA), X2=Aha(SFA)) |
| 87 | X1X2RERRX2E (wherein, X1=Uk(SFA), X2=Aha(SFA)) |
| 88 | X1YRFRRX2E (wherein, X1=Uk(SFA), X2=Aha(SFA)) |
| 89 | XYRFRRXE (wherein, X=Uk(SFA)) |
| 90 | X1YRX2RRX3EX3 (wherein, X1=Uk(SFA), X2=DAP(Gal), X3=Aha(SFA)) |
| 91 | X1X2RX3RX4X2E (wherein, X1=Uk(SFA), X2=Aha(SFA), X3= DAP(Gal), X4=r(dimet)) |
| 92 | X1X2RX3X4RX2E (wherein, X1=Uk(SFA), X2=Aha(SFA), X3= DAP(Gal), X4=r(dimet)) |
| 93 | X1YSX2RRX3EX3 (wherein, X1=Uk(SFA), X2= DAP(Gal), X3=Aha(SFA)) |
| 94 | X1YRX2SRX3EX3 (wherein X1=Uk(SFA), X2= DAP(Gal), X3=Aha(SFA)) |
| 95 | X1YRX2RSX3EX3 (wherein X1=Uk(SFA), X2= DAP(Gal), X3=Aha(SFA)) |
| 96 | XISRX2RRX3EX3 (wherein X1=Uk(SFA), X2= DAP(Gal), X3=Aha(SFA)) |
| 97 | X1X2RX2RRX3EX3 (wherein, X1=Uk(SFA), X2= DAP(Gal), X3=Aha(SFA)) |
| 98 | X1X2SX2RRX3EX3 (wherein, X1=Uk(SFA), X2=DAP(Gal), X3=Aha(SFA)) |
| 99 | X1X2RX3RSX2E (wherein, X1=Uk(SFA), X2=Aha(SFA), X3= DAP(Gal)) |
| 100 | X1X2RX3SRX2E (wherein, X1=Uk(SFA), X2=Aha(SFA), X3= DAP(Gal)) |
| 101 | X1X2SX3RRX2E (wherein, X1=Uk(SFA), X2=Aha(SFA), X3= DAP(Gal)) |
| 102 | X1X2X3X4RRX2E (wherein, X1=Uk(SFA), X2=Aha(SFA), X3=R(dimet), X4=DAP(Gal)) |
| 103 | X1X2RX3RRX1E (wherein, X1=Uk(SFA), X2= DAP(Gal), X3=Uk(sIB)) |
| 104 | X1X2RX3RRX2E (wherein, X1=Uk(SFA), X2=Aha(SFA), X3=Uk(sIB)) |
| 105 | X1ERX2RRX3EX3 (wherein, X1=Uk(SFA), X2=DAP(Gal), X3=Aha(SFA)) |
| 106 | X1X2SSSSX2E (wherein, X1=Uk(SFA), X2=Aha(SFA)) |
| 107 | X1X2SX3RSX2E (wherein, X1=Uk(SFA), X2=Aha(SFA), X3=DAP(Gal)) |
| | * Trityl resin-synthesized compounds have a C-terminal -COOH group. |
| In the above sequences, the amino acids may be D-amino acids or L-amino acids. In addition, the chemical structures of the abbreviations used in each sequence are as follows. | |

| Abbreviation | Chemical formula |
|---|---|
| DAP(Gal) | 2-amino-3-((2S,3R,4S,5R,6S)-3,4,5,6-tetrahydroxytetrahydro-2H-pyran-2-carboxamido)propanoic acid |
| Uk(IB) | N⁶-(4-(4-iodophenyl)butanoyl)-lysine |
| Uk(DOTA) | 2,2',2"-(10-(2-((5,6-diamino-6-oxohexyl)amino)-2-oxoethyl)-1,4,7,10-tetraazacyclododecane-1,4,7-triyl)triacetic acid |
| Pra(AF488) | 5-((6-(4-(2-amino-2-carboxyethyl)-1H-1,2,3-triazol-1-yl)hexyl)carbamoyl)-2-(6-amino-3-imino-4,5-disulfo-3H-xanthen-9-yl)benzoic acid |
| DAP(DOTA) | 2,2',2"-(10-(2-((2-amino-2-carboxyethyl)amino)-2-oxoethyl)-1,4,7,10-tetraazacyclododecane-1,4,7-triyl)triacetic acid |
| DAP(SZW) | 3-((2-((2-amino-2-carboxyethyl)amino)-2-oxoethyl)(metheyliumyl)(methyl)-λ⁴-azaneyl)propane-1-sulfonate |
| Gla | γ-carboxyglutamic acid |
| Pra(Cy5) | 1-(6-((3-(4-(2-amino-2-carboxyethyl)-1H-1,2,3-triazol-1-yl)propyl)amino)-6-oxohexyl)-3,3-dimethyl-2-((1E,3E)-5-((E)-1,3,3-trimethyl-5-sulfoindolin-2-ylidene)penta-1,3-dien-1-yl)-3H-indol-1-ium-5-sulfonate |
| Uk(sIB) | N⁶-(2-(4-iodophenyl)acetyl)-D-lysine |
| DAP(6OH) | (R)-2-amino-3-((2R,3R,4S,5R,6R)-2,3,4,5,6,7-hexahydroxyheptanamido)propanoic acid |
| DAP(PSC) | (R)-2,2'-(4-(2-((2-amino-2-carboxyethyl)amino)-2-oxoethyl)-10-(2-amino-2-oxoethyl)-1,4,7,10-tetraazacyclododecane-1,7-diyl)diacetic acid |
| DAP(TCMC) | (2R)-2-amino-3-(3-(4-((1,4,7,10-tetrakis(2-amino-2-oxoethyl)-1,4,7,10-tetraazacyclododecan-2-yl)methyl)phenyl)thioureido)propanoic acid |
| r(dimet) | (R)-2-amino-5-((amino(dimethyl-14-azaneylidene)methyl)amino)pentanoic acid |
| R(dimet) | (S)-2-amino-5-((amino(dimethyl-14-azaneylidene)methyl)amino)pentanoic acid |
| PEG1 | 2-(2-(2-aminoethoxy)ethoxy)acetic acid |
| PEG(6atom) | 2-(2-aminoethoxy)acetic acid |
| K(biotin) | N6-(5-((3aR,4R,6aS)-2-oxohexahydro-1H-thieno[3,4-d]imidazol-4-yl)pentanoyl)-L-lysine |
| UK(SFA) | (R)-2-amino-6-(4-(4-sulfamoylphenyl)-1H-1,2,3-triazol-1-yl)hexanoic acid |
| Aha(SFA) | (R)-2-amino-4-(4-(4-sulfamoylphenyl)-1H-1,2,3-triazol-1-yl)butanoic acid |

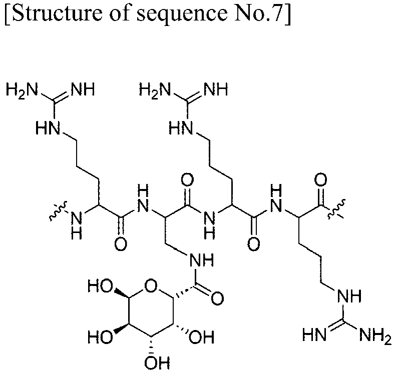

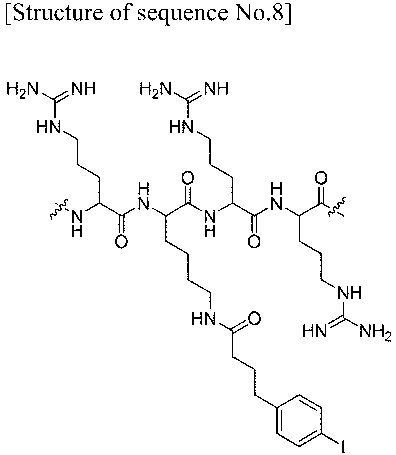

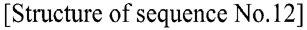

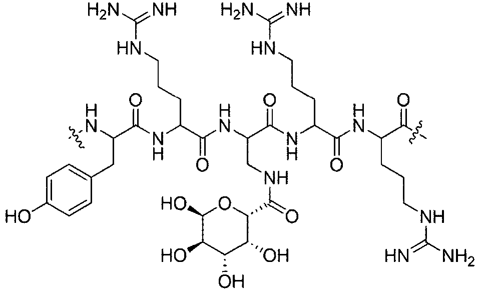

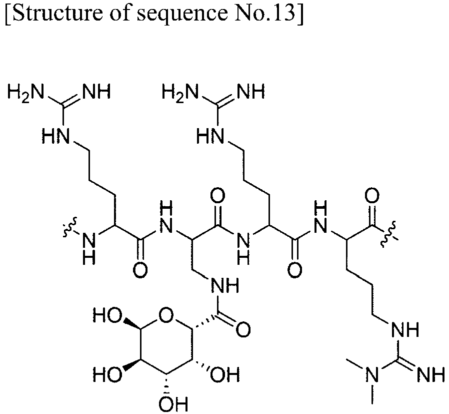

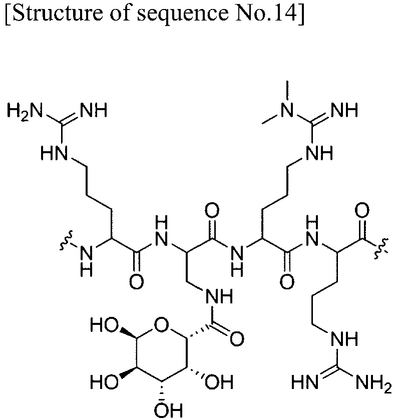

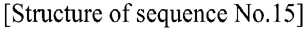

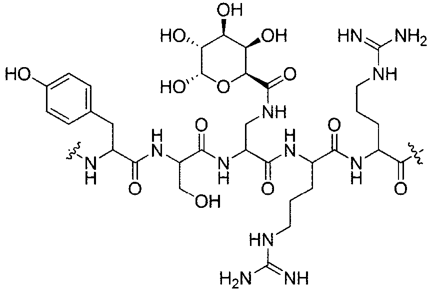

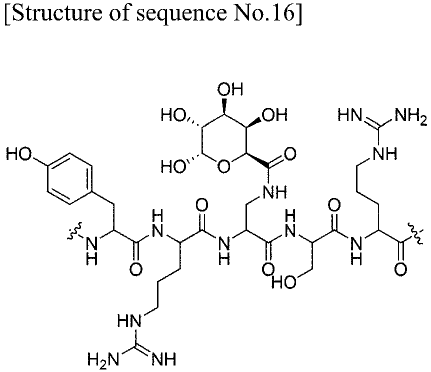

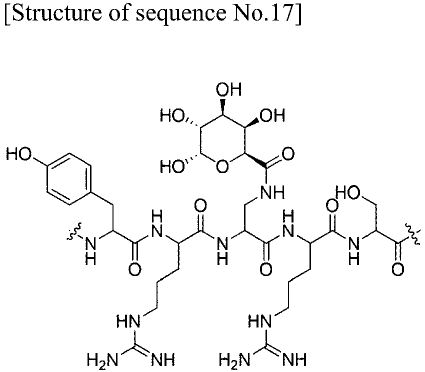

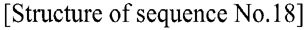

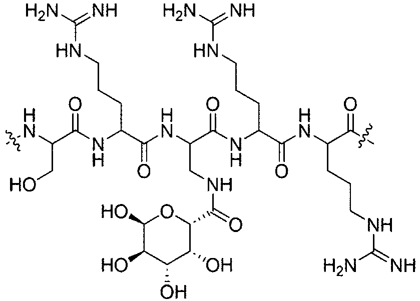

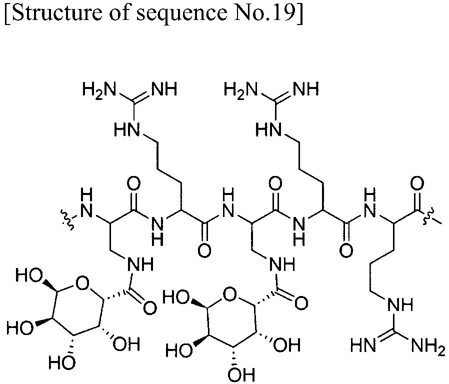

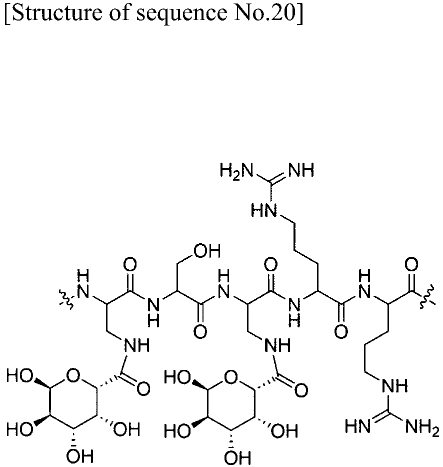

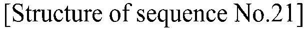

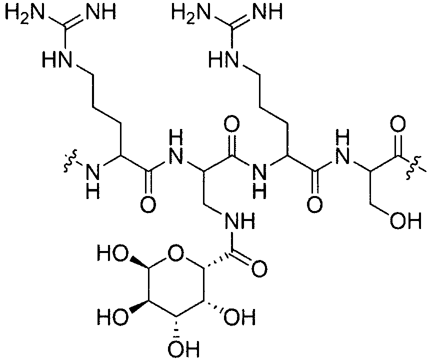

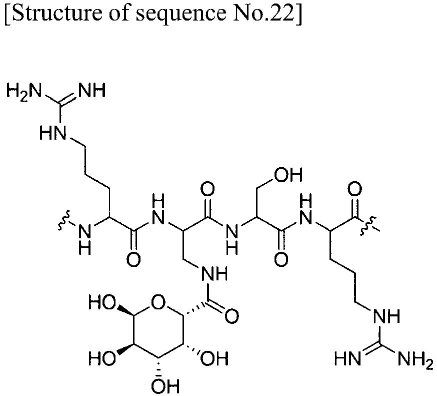

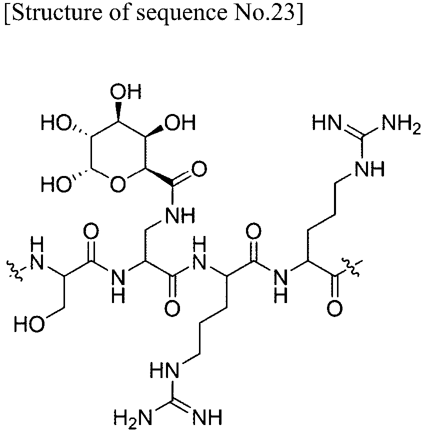

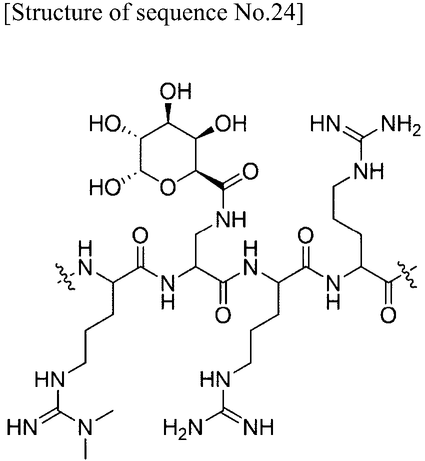

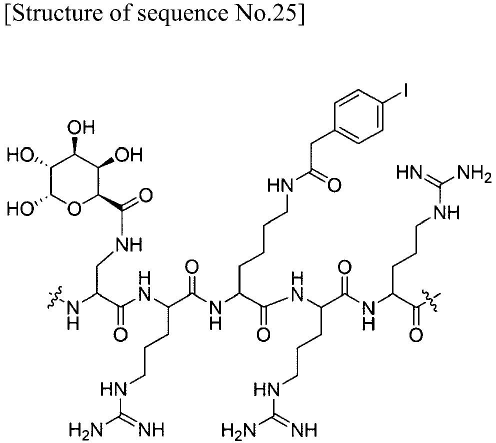

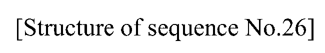

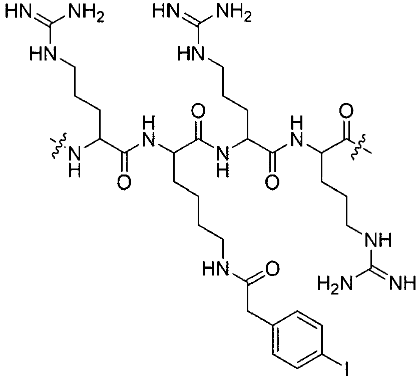

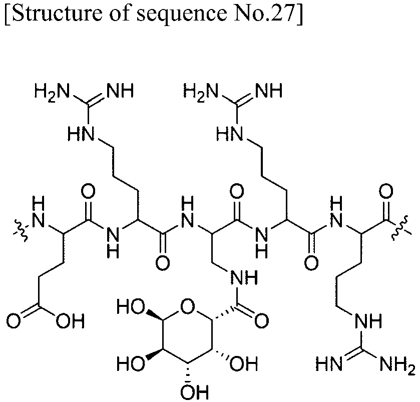

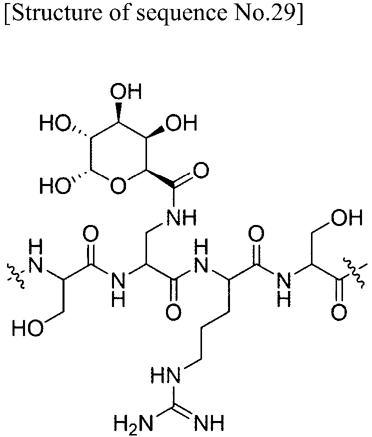

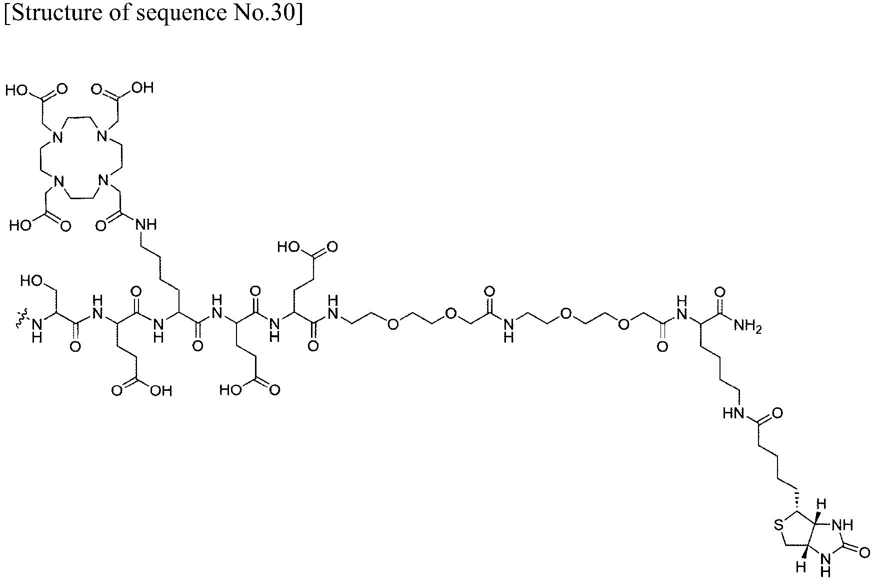

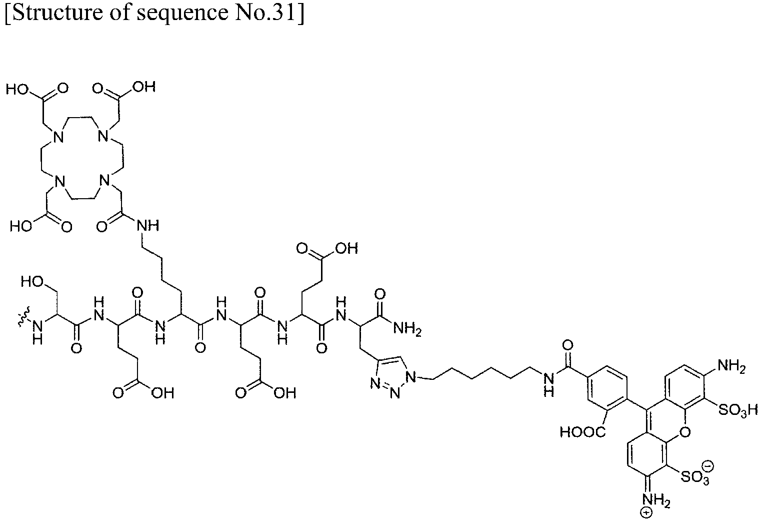

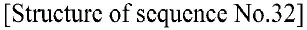

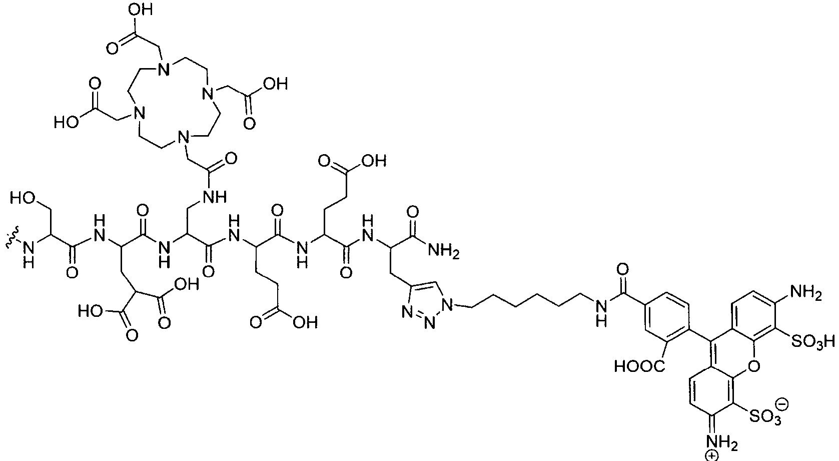

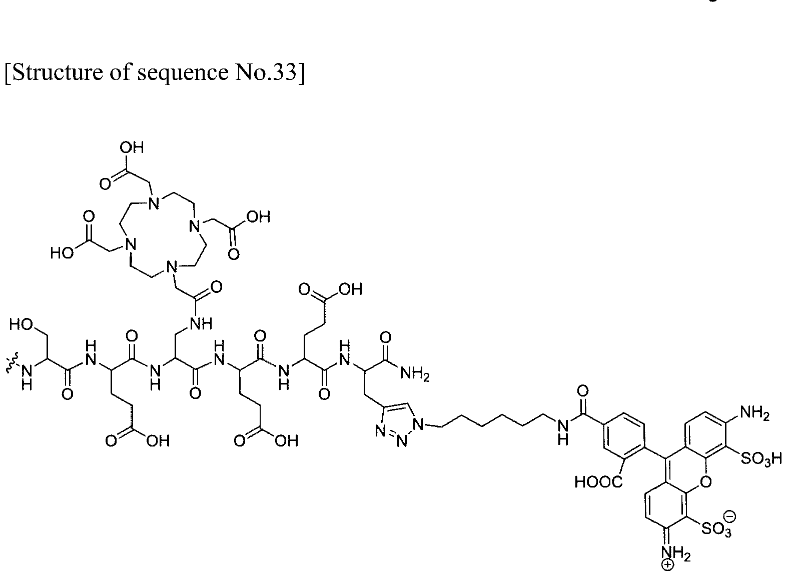

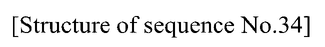

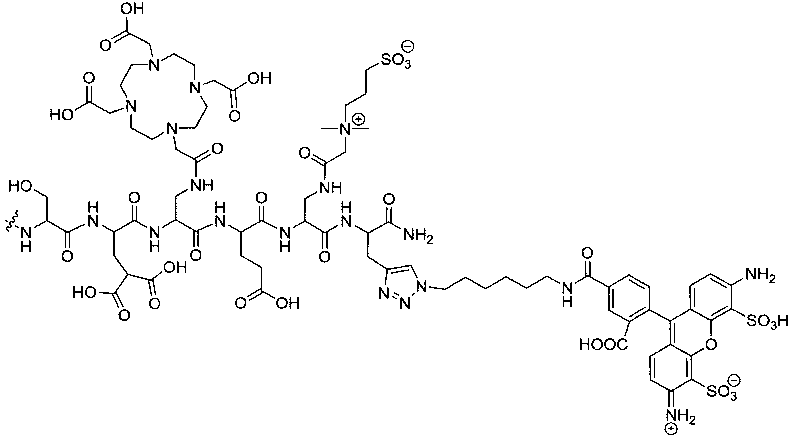

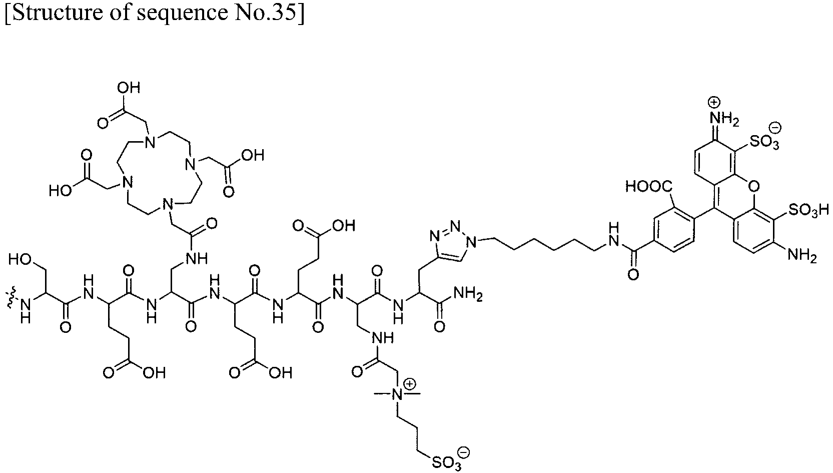

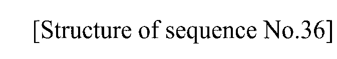

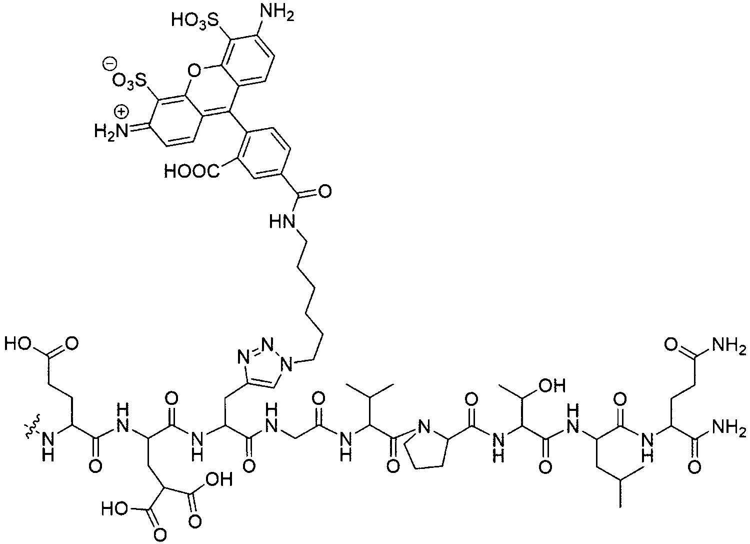

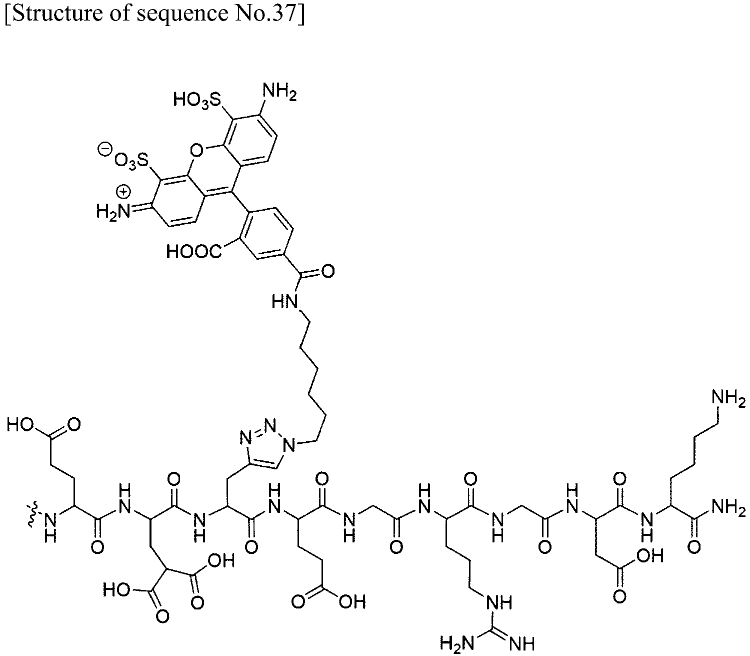

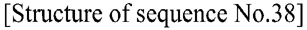

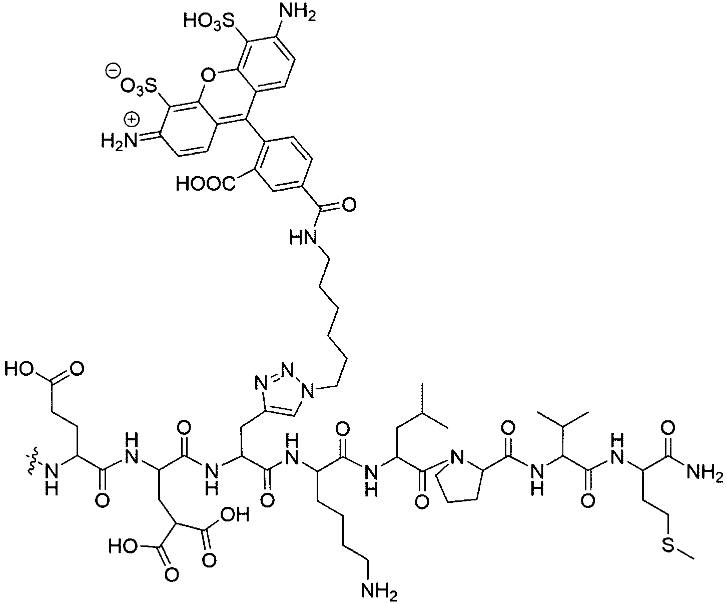

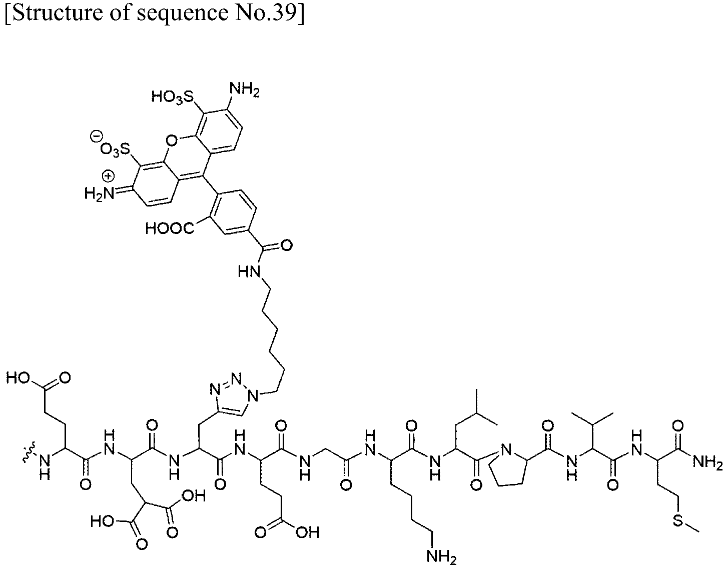

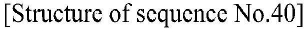

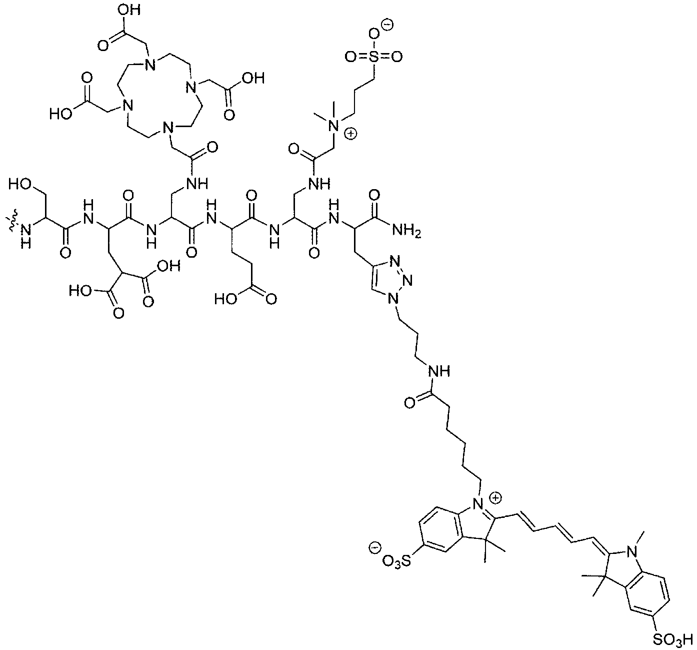

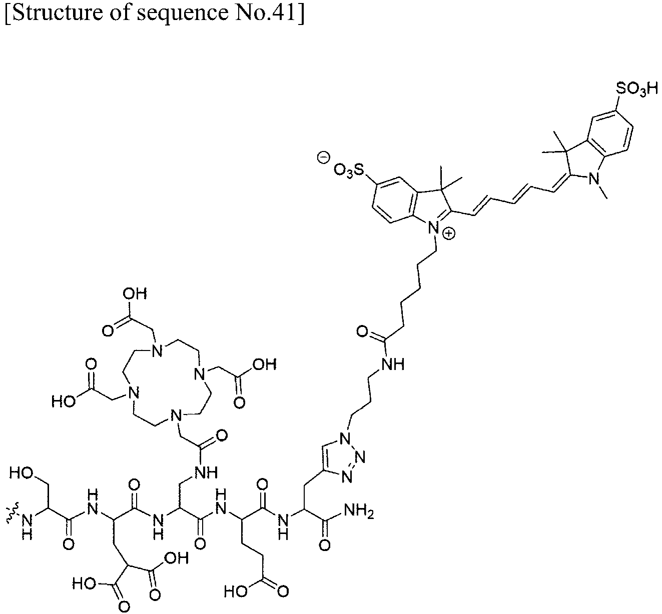

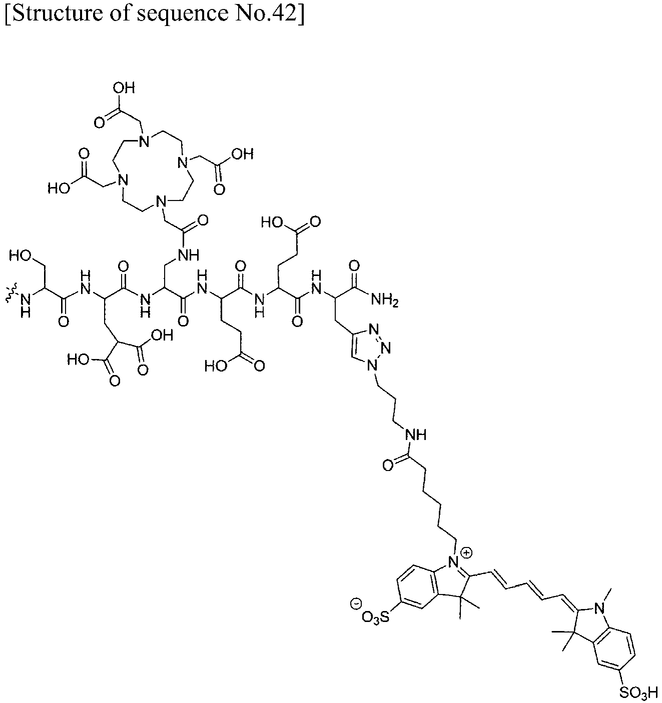

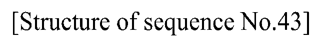

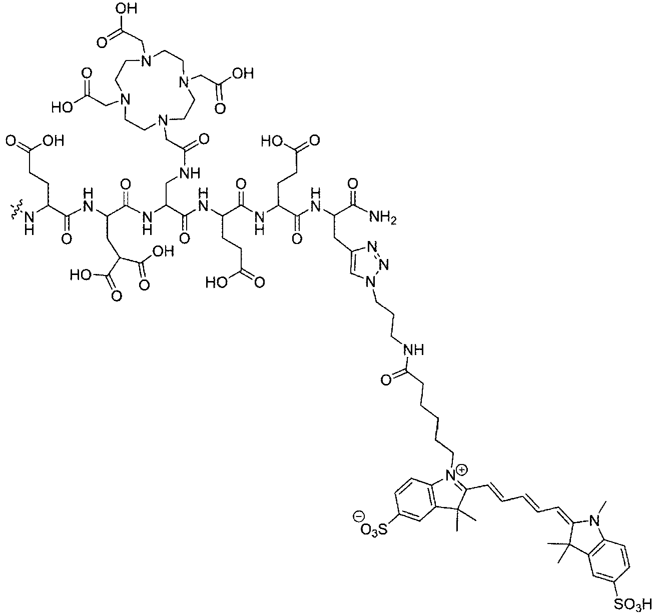

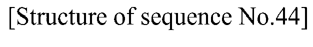

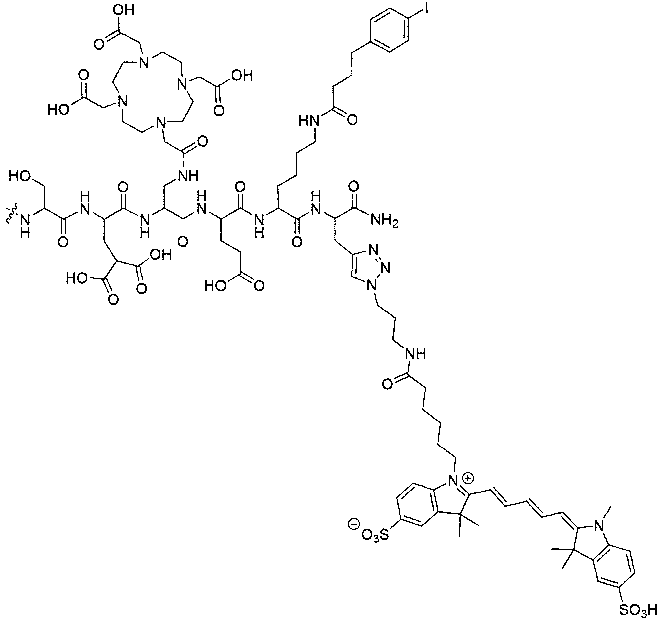

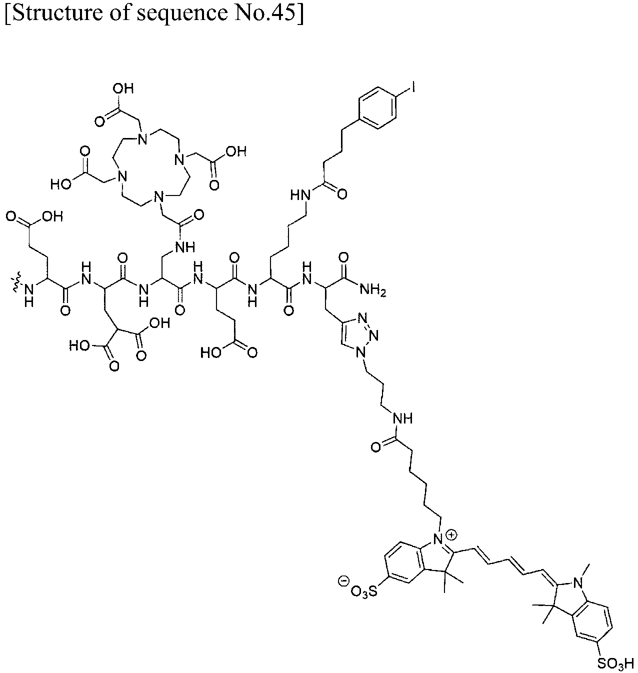

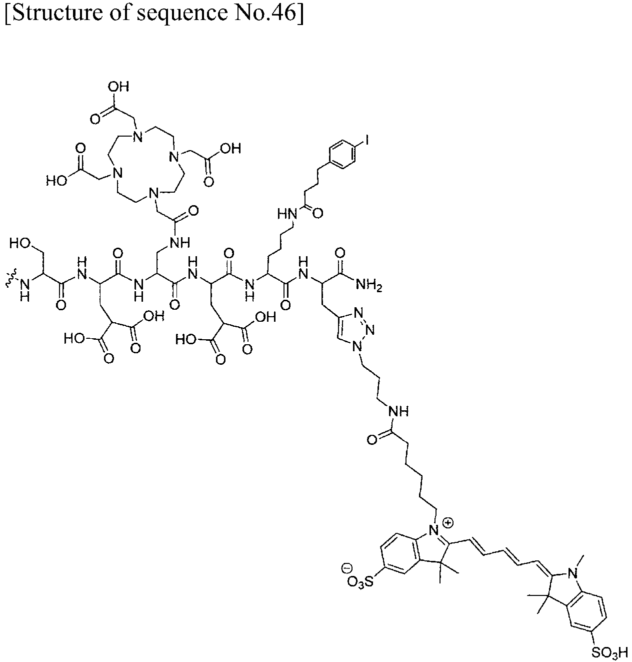

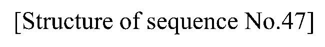

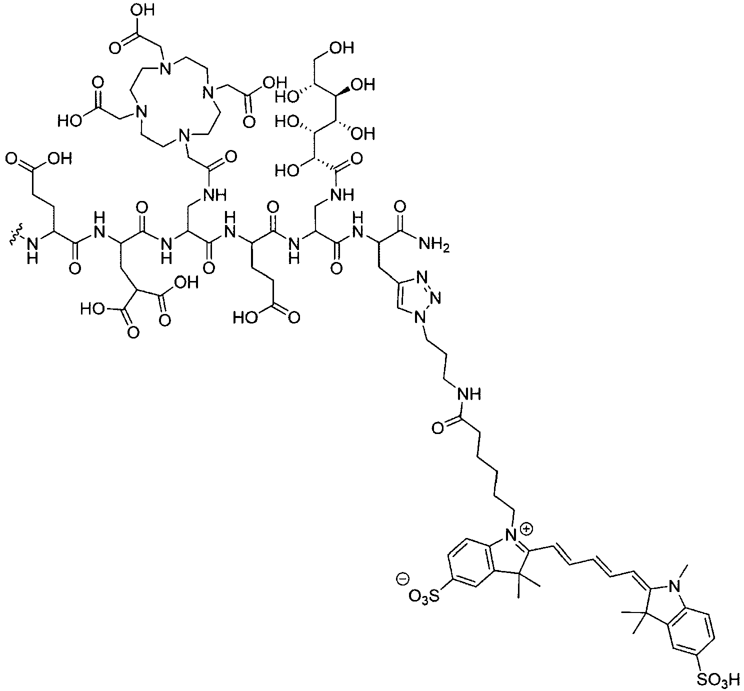

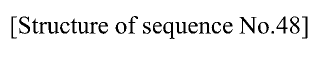

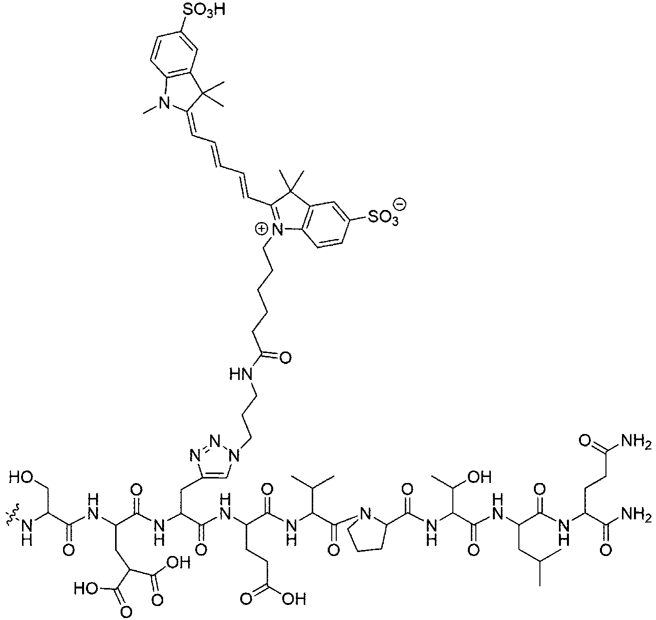

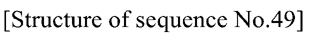

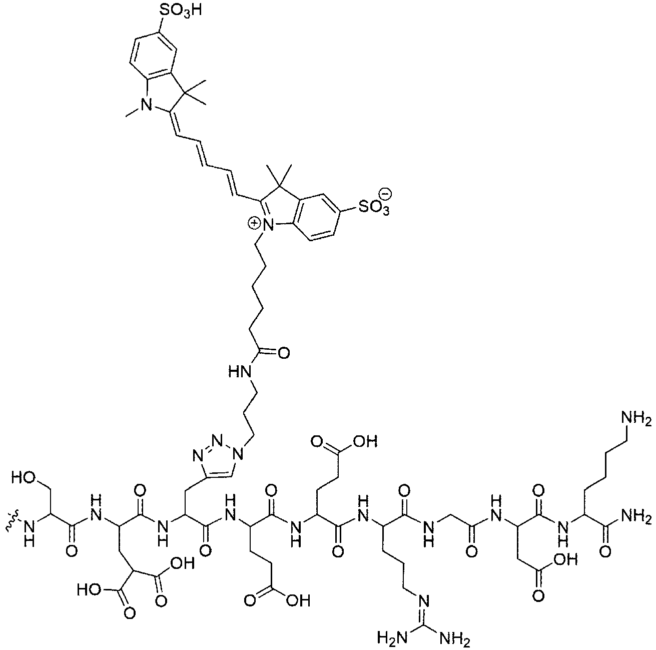

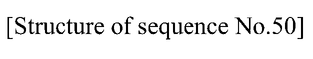

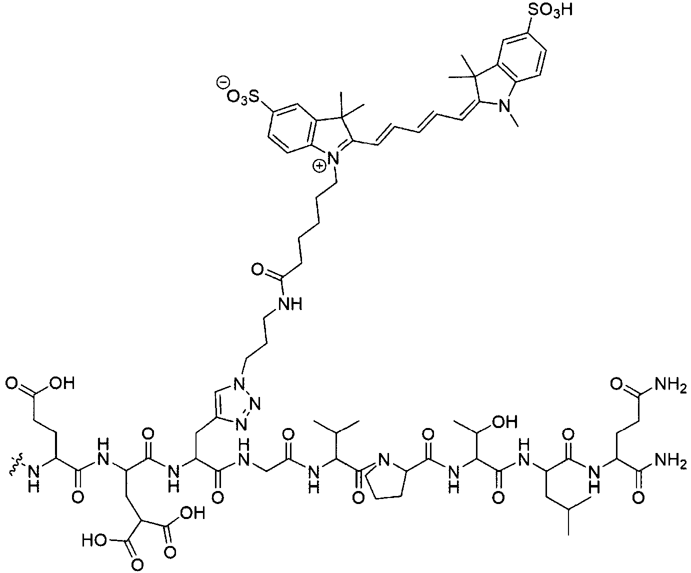

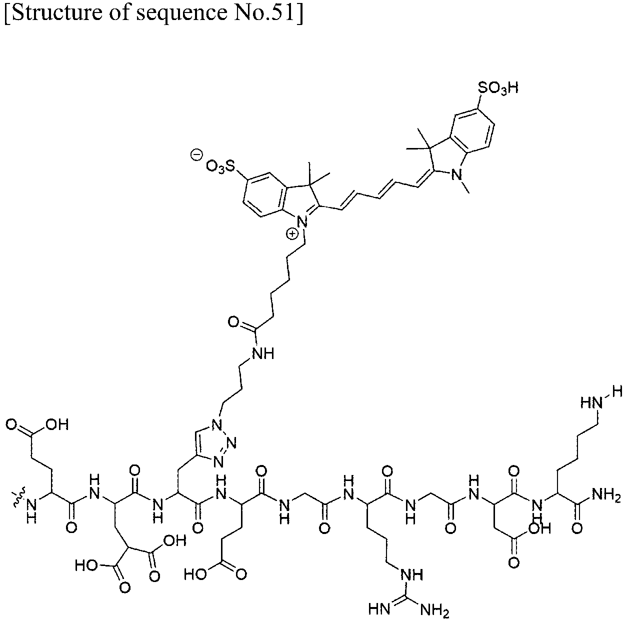

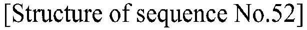

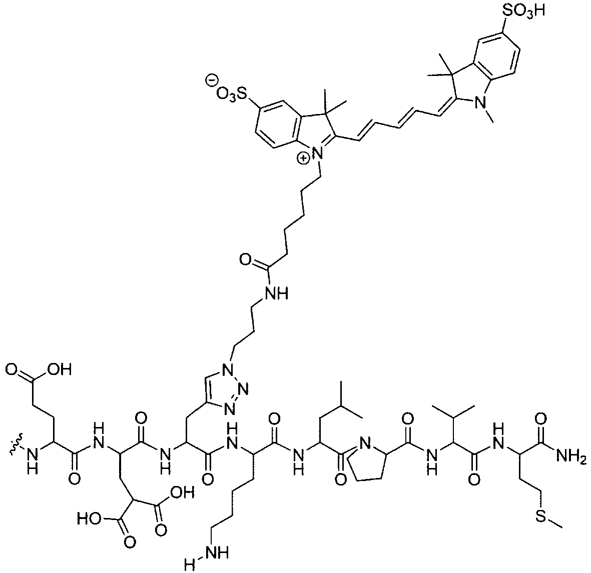

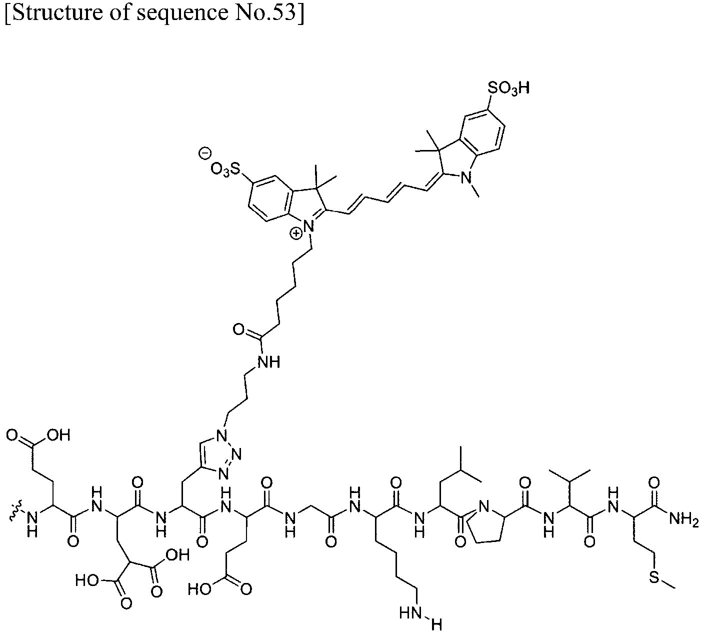

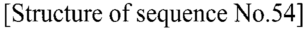

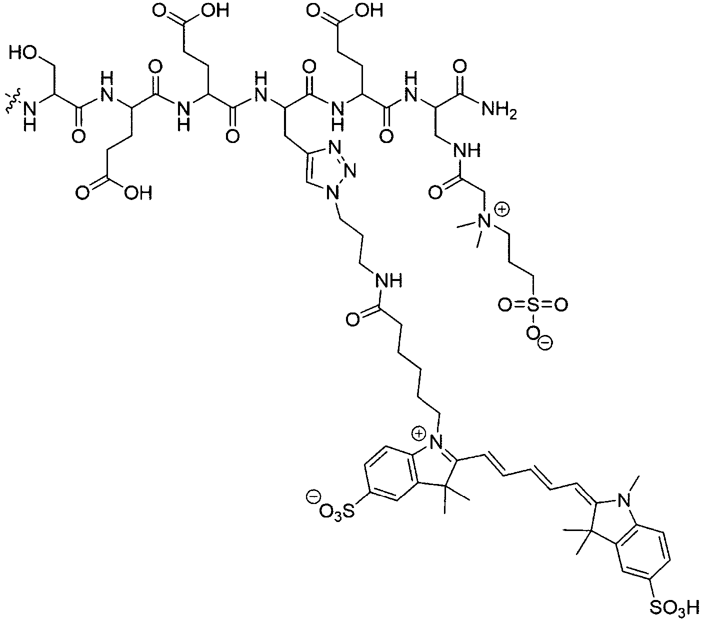

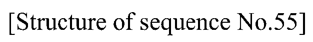

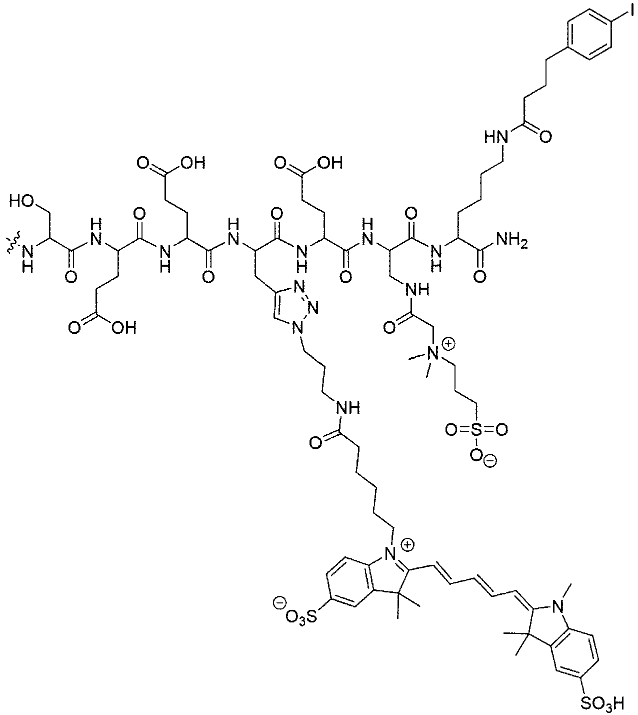

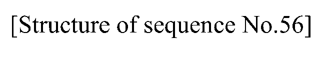

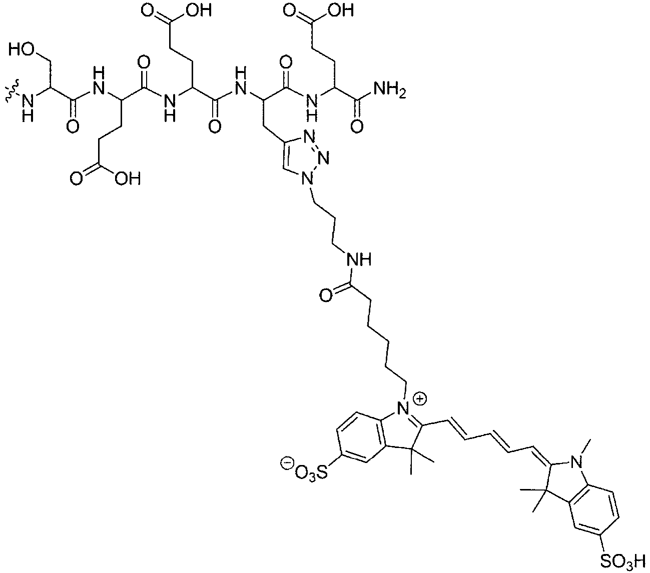

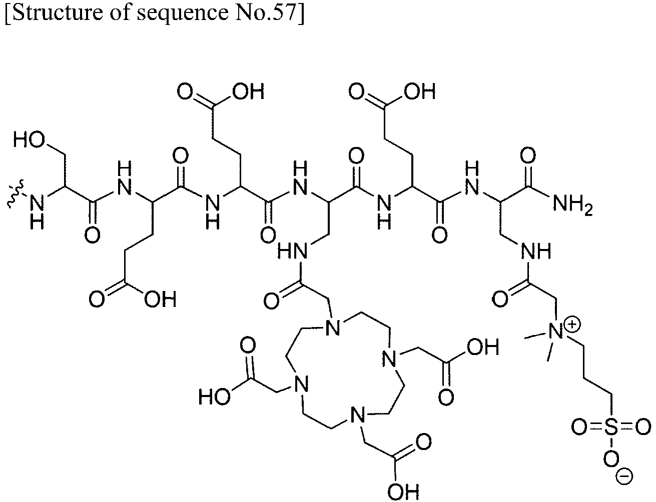

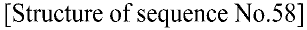

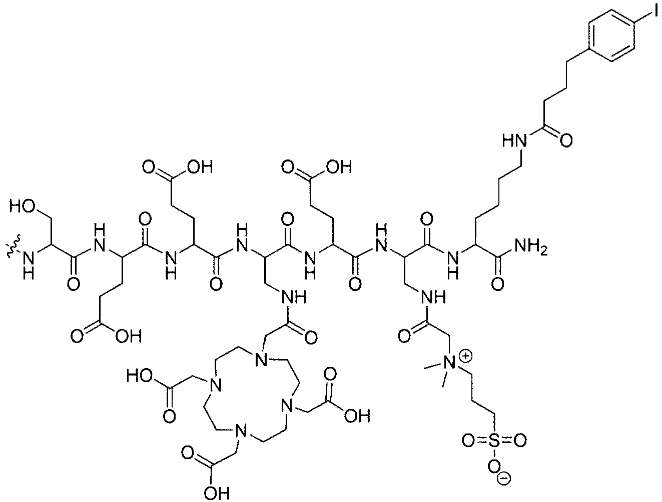

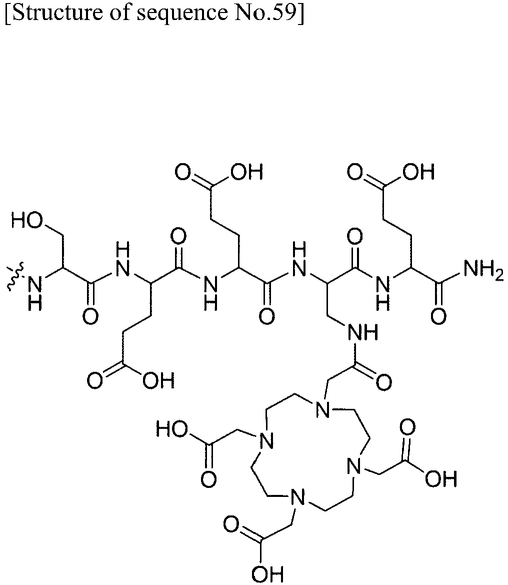

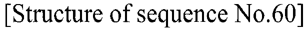

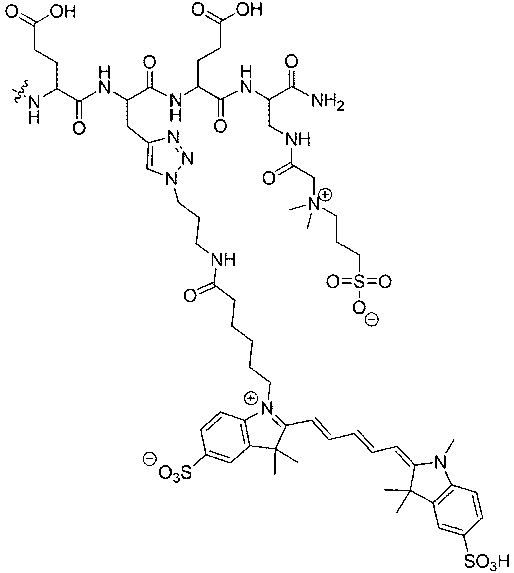

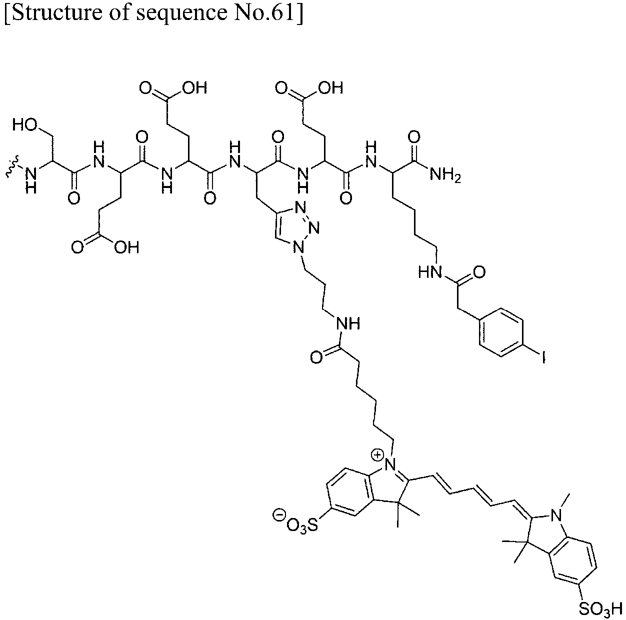

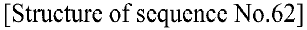

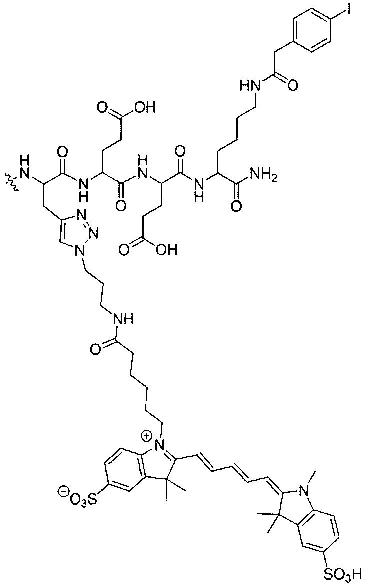

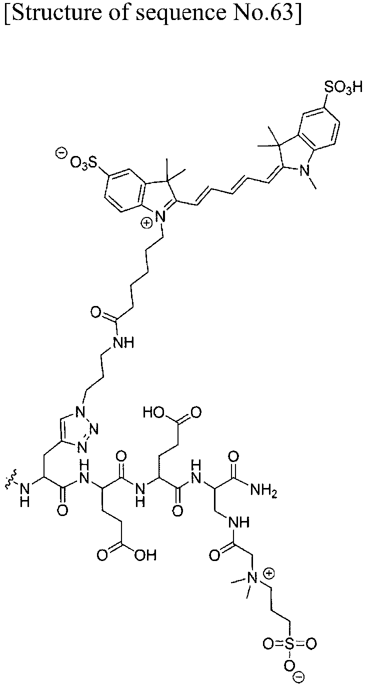

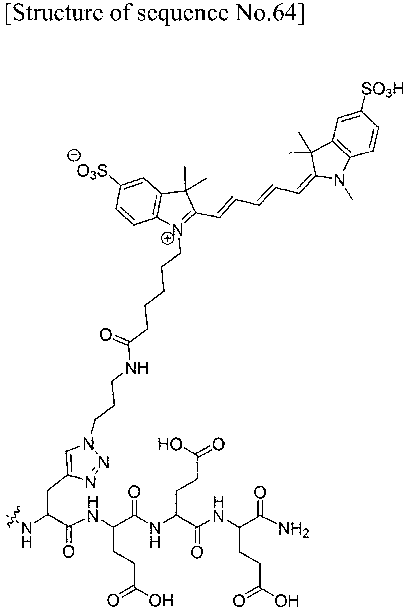

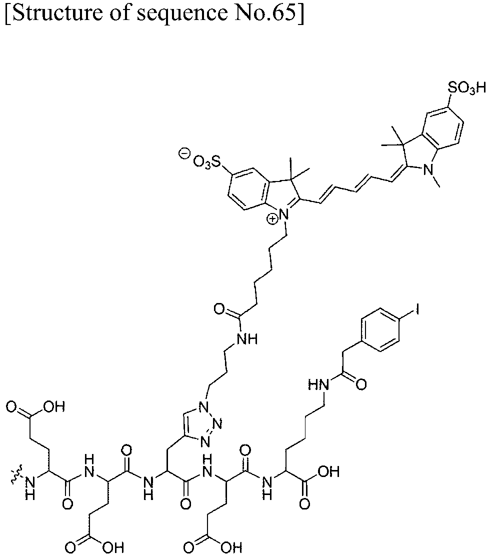

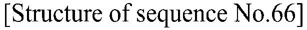

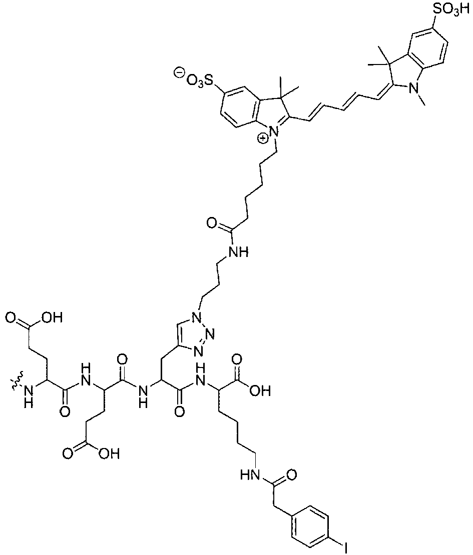

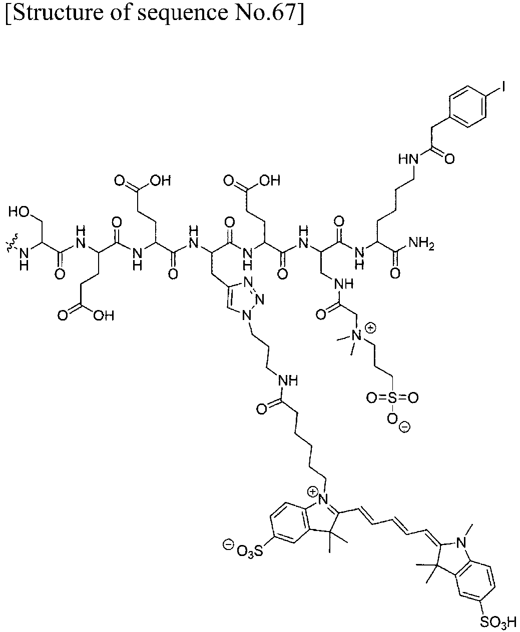

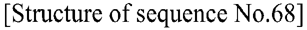

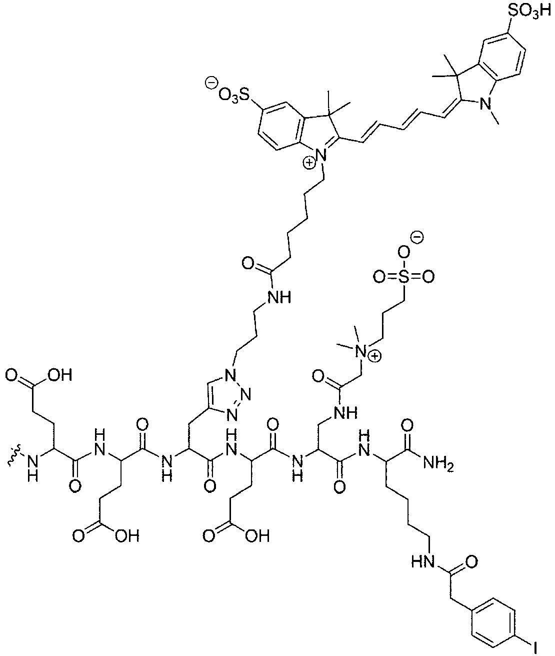

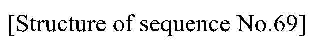

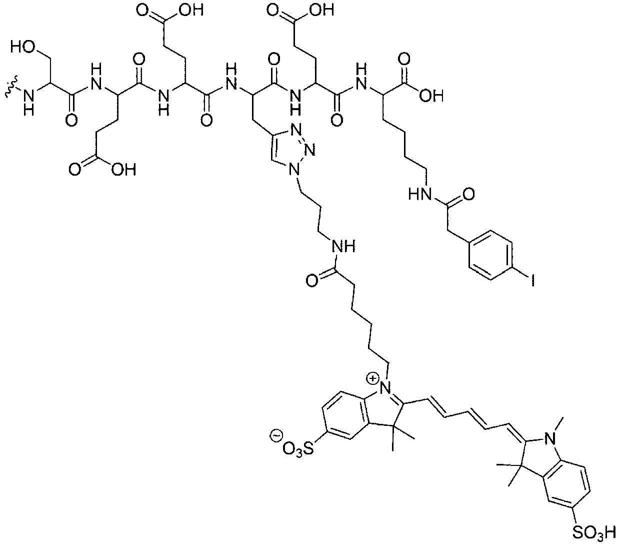

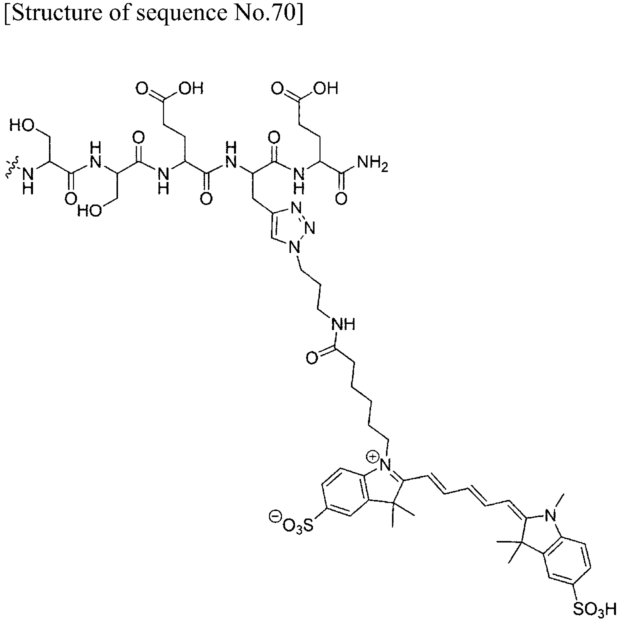

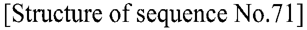

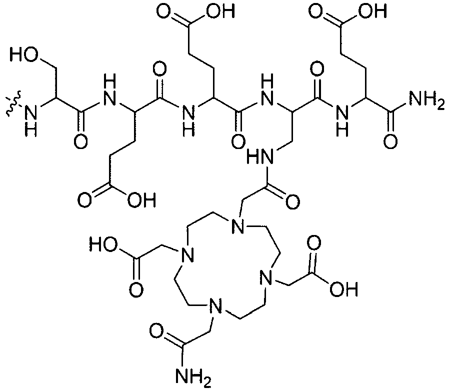

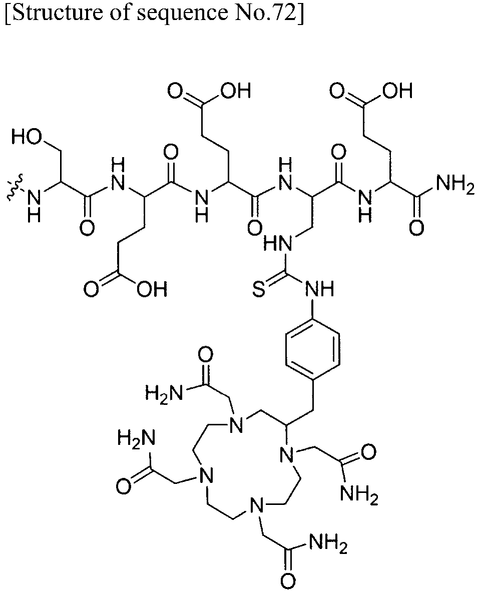

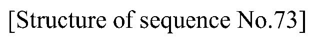

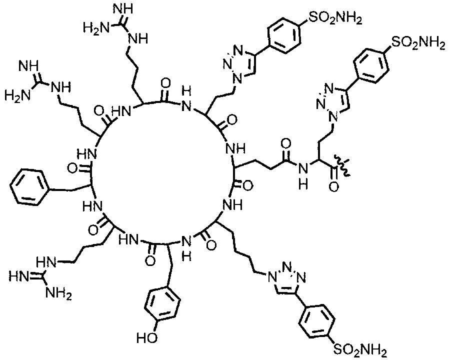

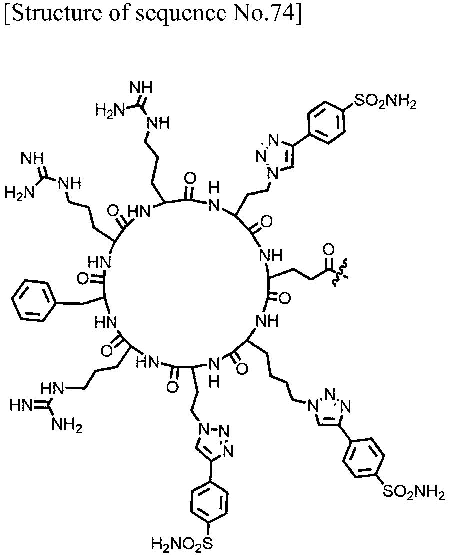

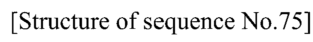

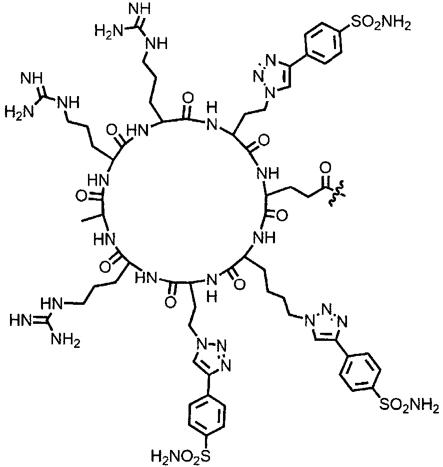

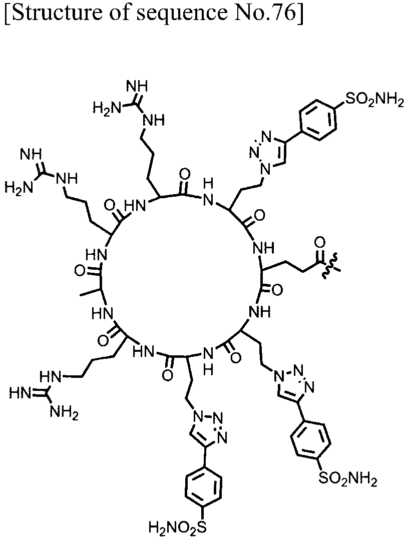

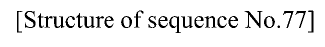

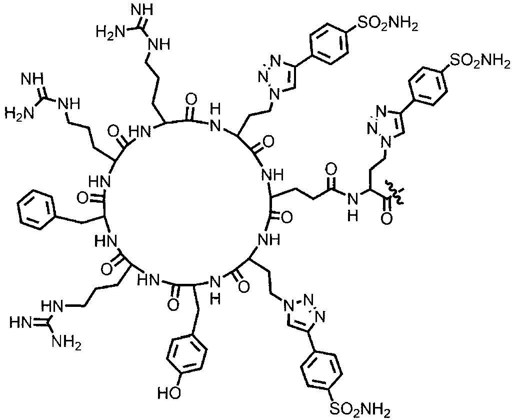

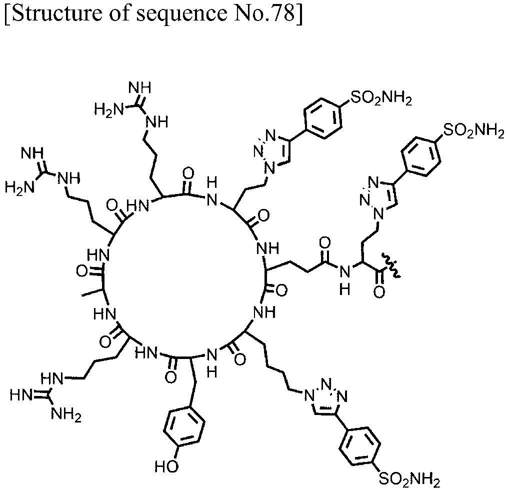

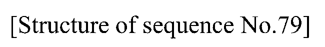

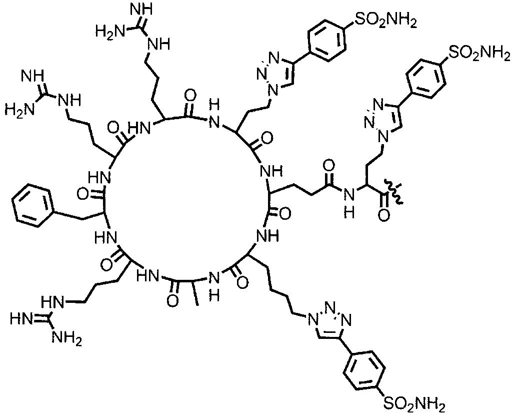

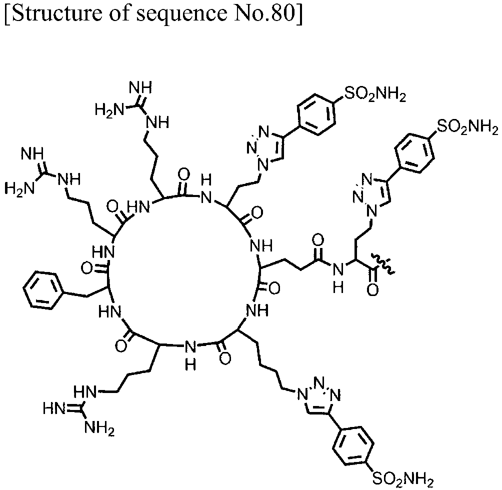

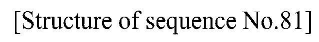

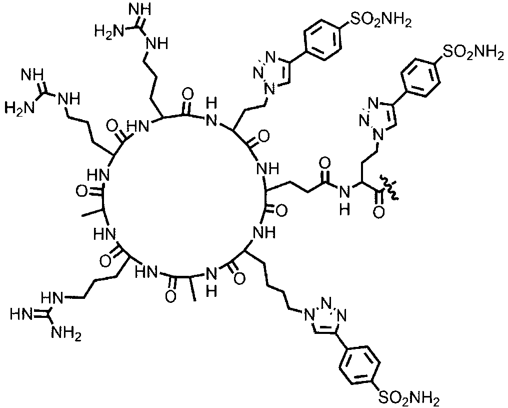

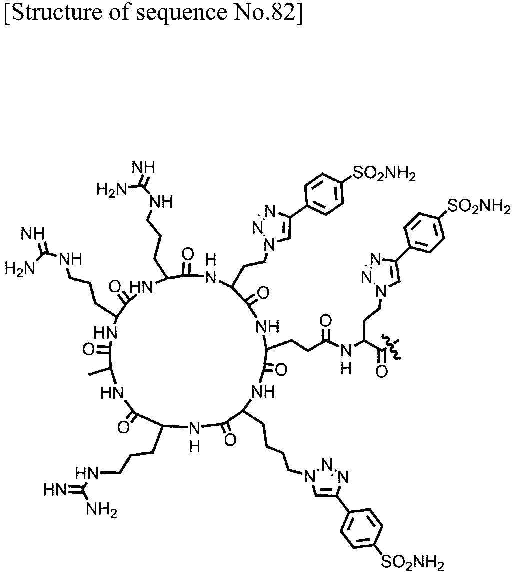

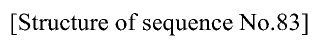

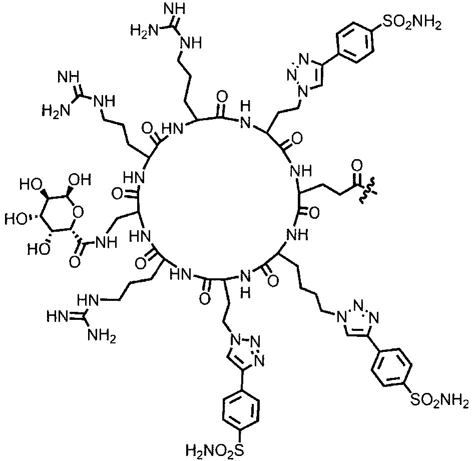

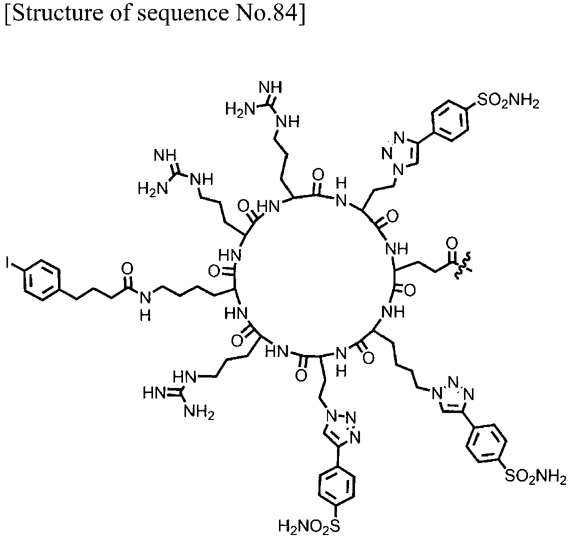

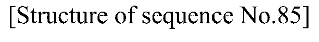

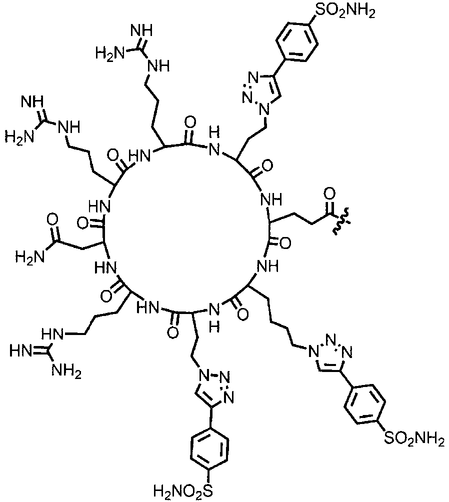

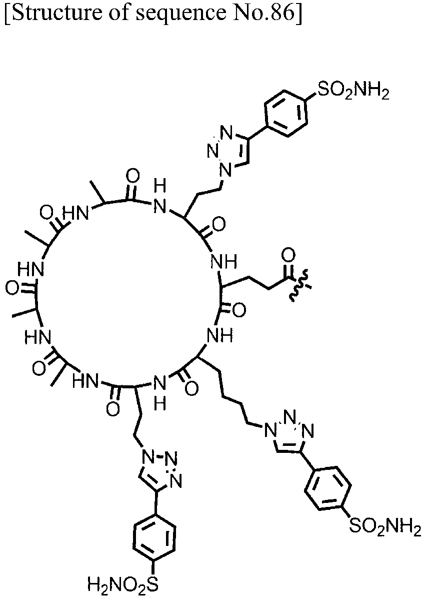

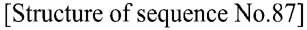

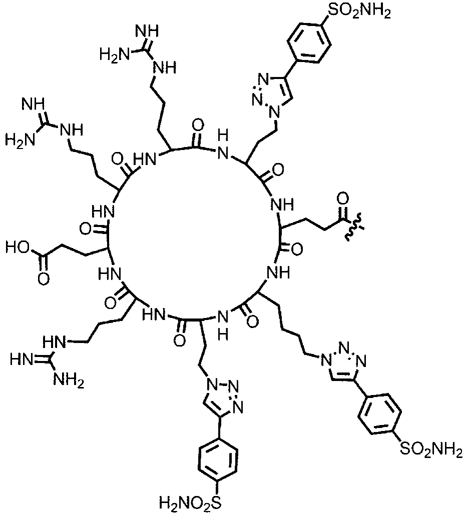

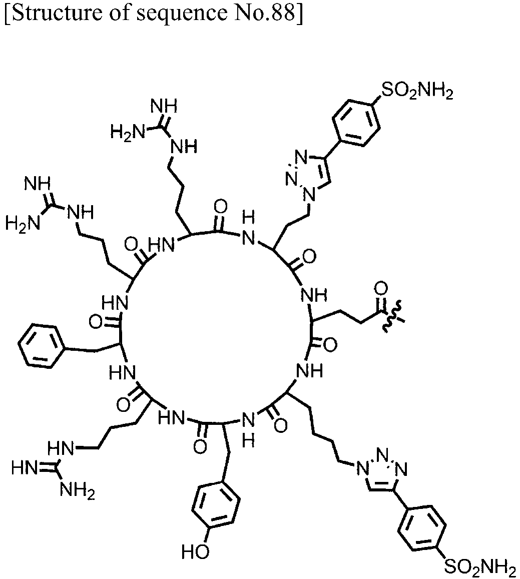

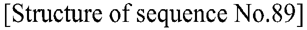

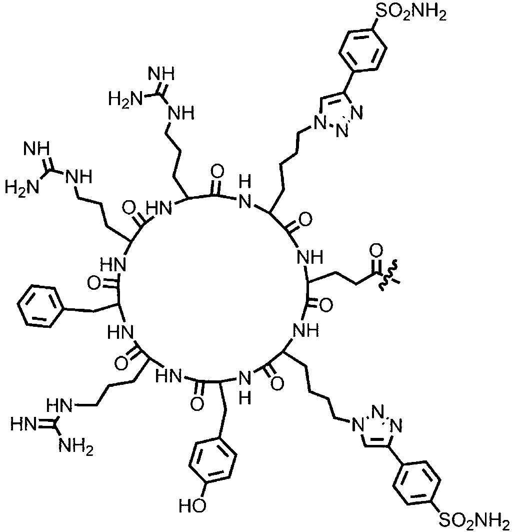

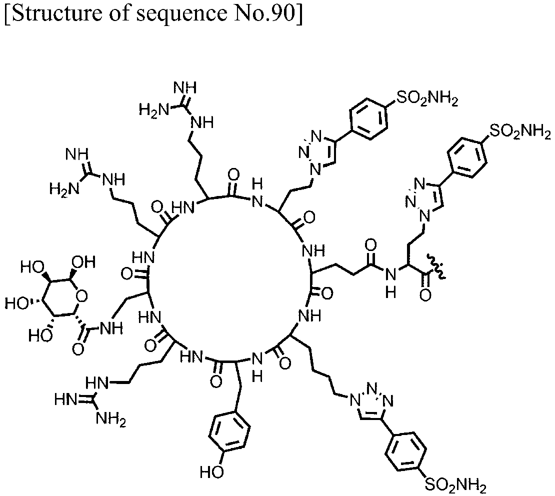

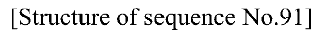

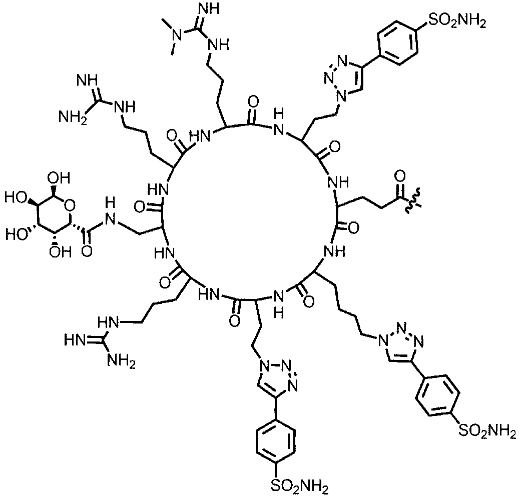

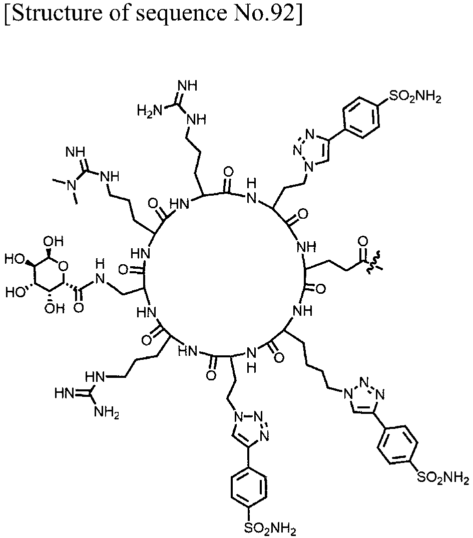

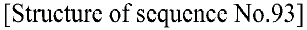

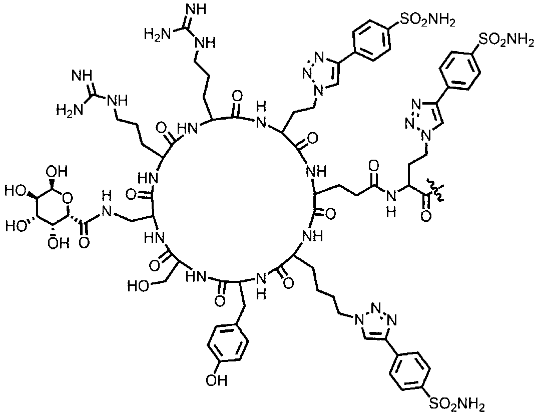

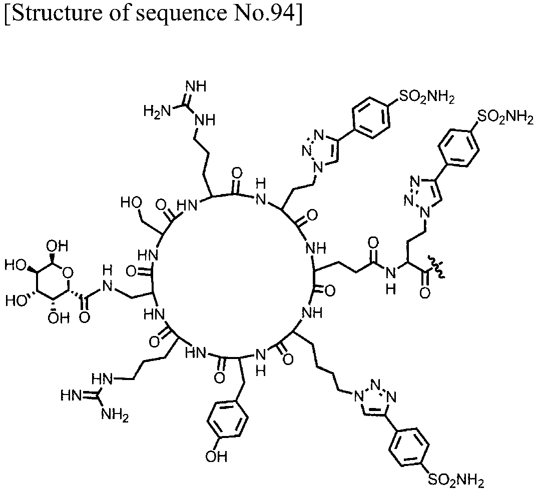

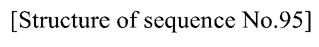

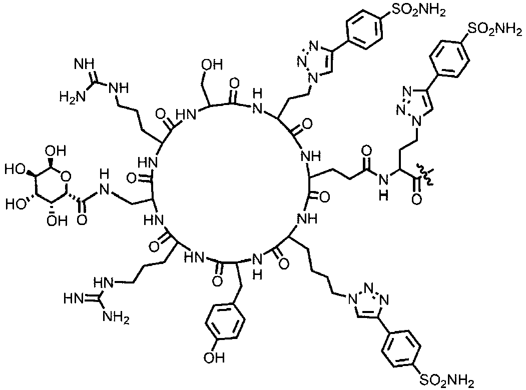

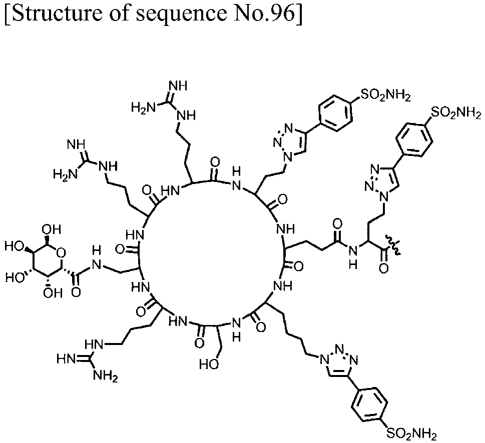

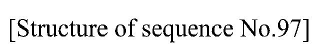

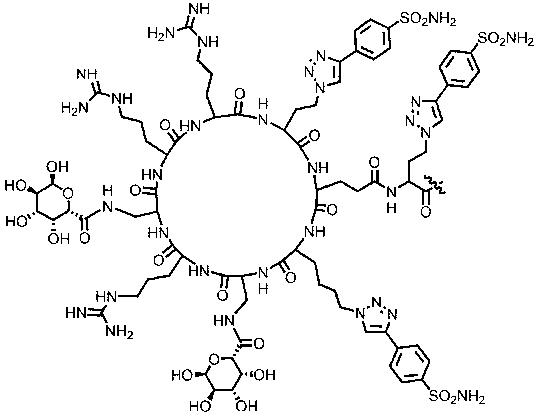

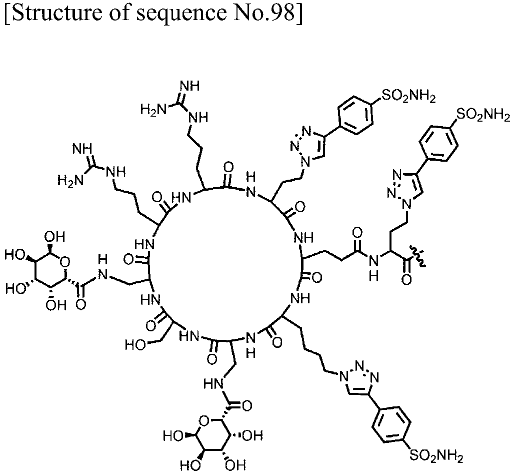

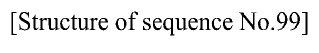

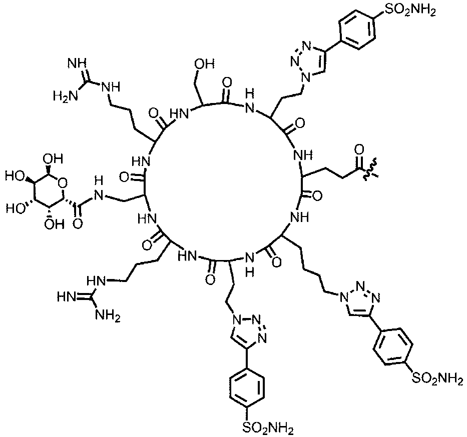

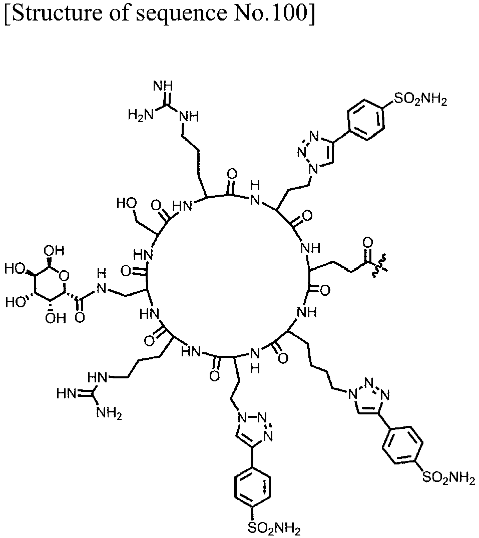

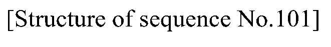

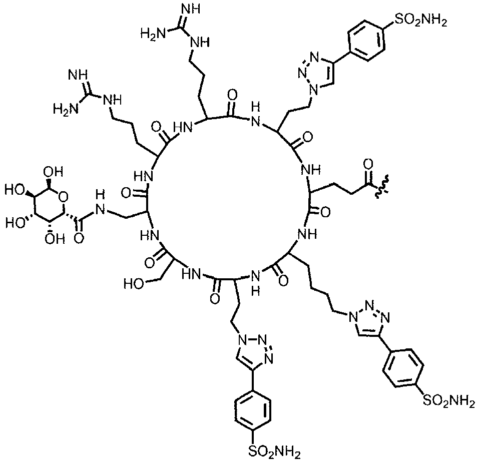

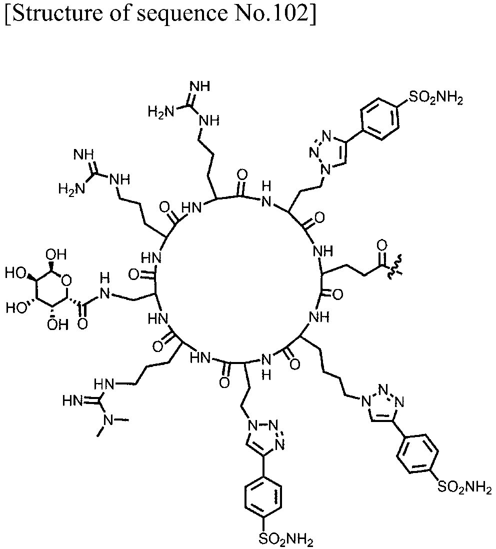

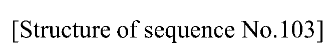

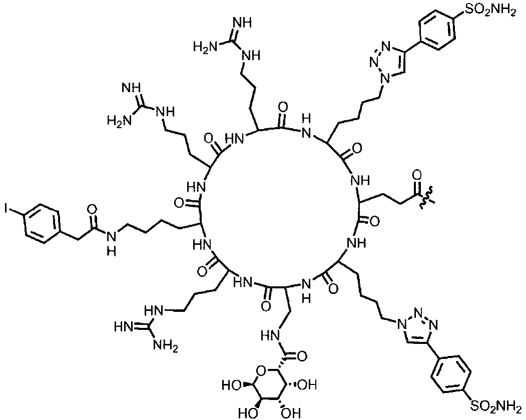

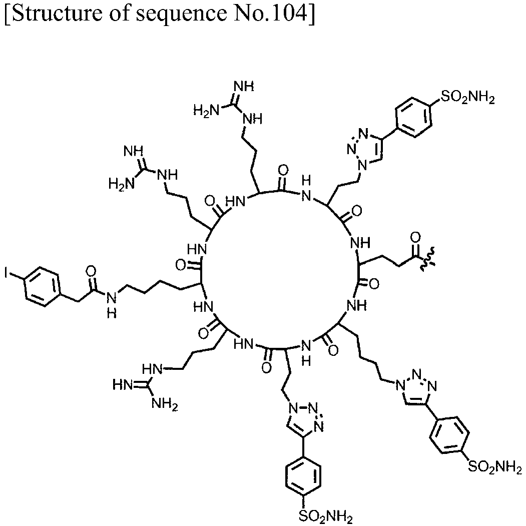

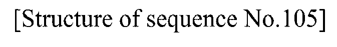

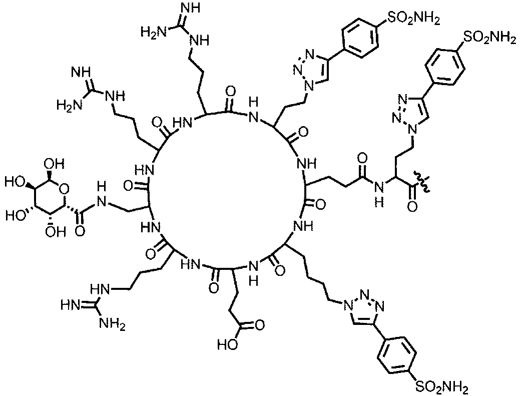

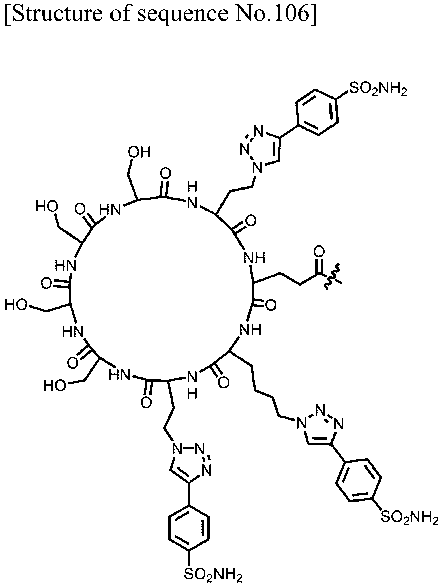

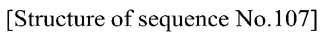

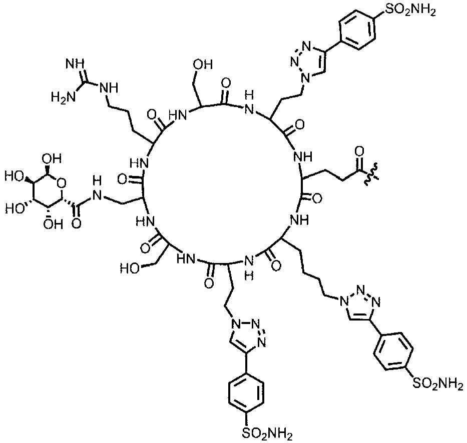

## Claims

1. A peptide construct **characterized by** comprising:
a peptide ligand comprising an amino acid sequence of any one of SEQ ID NOs: 1 to 29, wherein at least one of the constituent amino acids is composed of D-amino acids,
and a lysine (Lys) residue among the constituent amino acids is optionally substituted with a chemical functional group at the side chain ε-amino group, or a 2,3-diaminopropionic acid (DAP) residue among the constituent amino acids is optionally substituted with a chemical functional group at the side-chain β-amino group; and
a sulfonamide functional group-containing amino acid residue linked directly or via a spacer to the peptide ligand,
and having a structure represented by Formula 1 below: 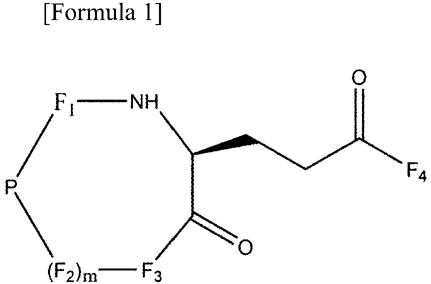
wherein P is the peptide ligand,
F₁, F₂ and F₃ are each independently a sulfonamide functional group-containing amino acid residue,
m is 0 or 1,
F₄ is a group of the general formula -(F₅)ₒ-(S₁)ₚ-(F₆)_{q}-(S₂)ᵣ-(F₇)ₛ-(S₃), wherein
F₅ is a sulfonamide functional group-containing amino acid residue,
S₁ and S₂ are each independently a spacer,
F₆ and F₇ are each independently a functional group-containing amino acid residue other than sulfonamide,
S₃ is -NH₂ or -OH,
o is an integer of 0 or 1, and
p, q, r, and s each independently represent an integer from 0 to 6.

2. The peptide construct according to claim 1,
**characterized in that** a chemical functional group that is optionally substituted at the side chain ε-amino group of a lysine (Lys) residue among the constituent amino acids is 4-(p-iodophenyl)butyric acid (IB) or 2-(4-iodophenyl)acetic acid (sIB).

3. The peptide construct according to claim 1 or 2,
**characterized in that** a chemical functional group that is optionally substituted at the side-chain β-amino group of a 2,3-diaminopropionic acid (DAP) residue among the constituent amino acids is α-D-galacturonic acid (Gal).

4. The peptide construct according to any one of claims 1 to 3,
**characterized in that** the sulfonamide functional group-containing amino acid residue has the following structure:

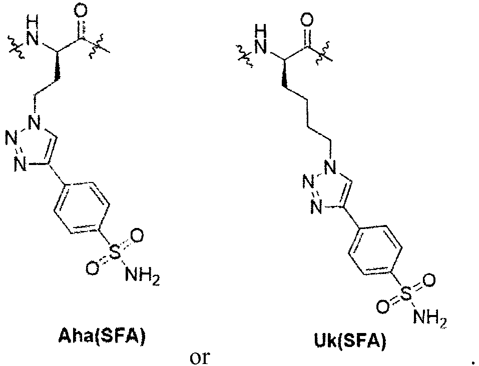

5. The peptide construct according to any one of claims 1 to 4,
**characterized in that** the peptide construct has a structure represented by Formula 2 below: 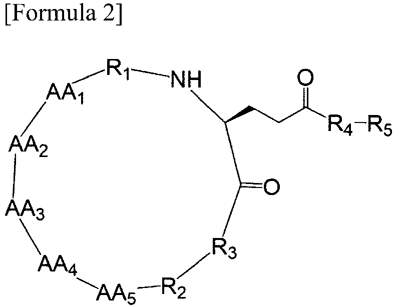 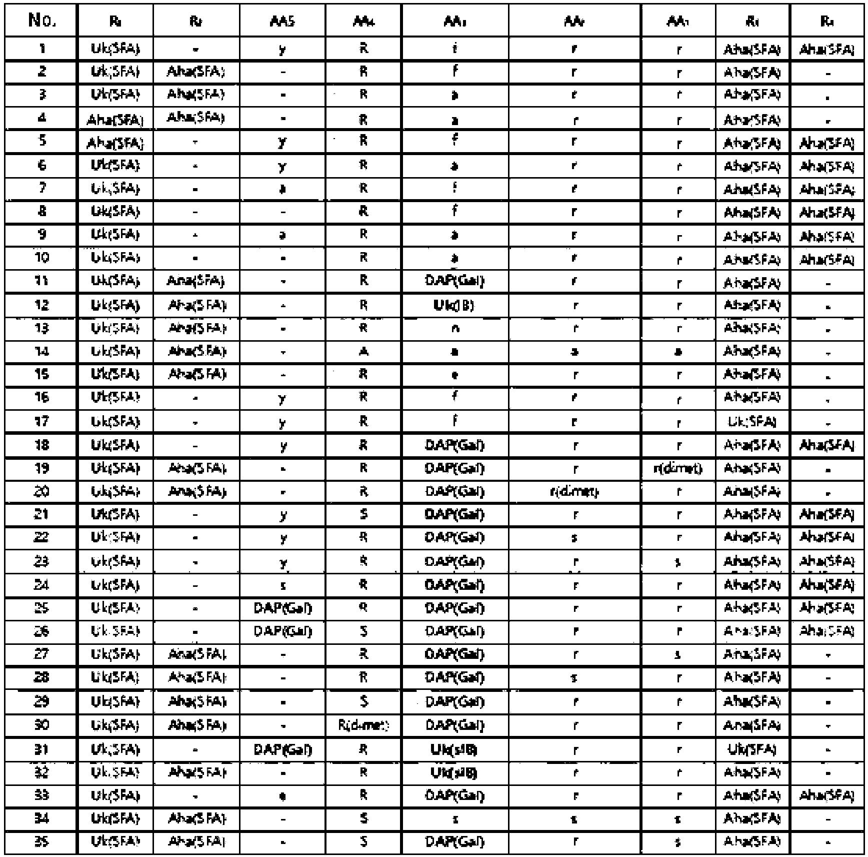
wherein R₅ is a group of the general formula -(S₁)ₚ-(F₆)_{q}-(S₂)ᵣ-(F₇)ₛ-(S₃),
S₁, S₂, S₃, F₆, F₇, p, q, r, and s are as defined in claim 1,
the lowercase letters represent D-amino acids and the uppercase letters represent L-amino acids, and
each of the other substituents indicated by abbreviations at R₁ to R₄ and AA₁ to AA₅ is defined as described below:

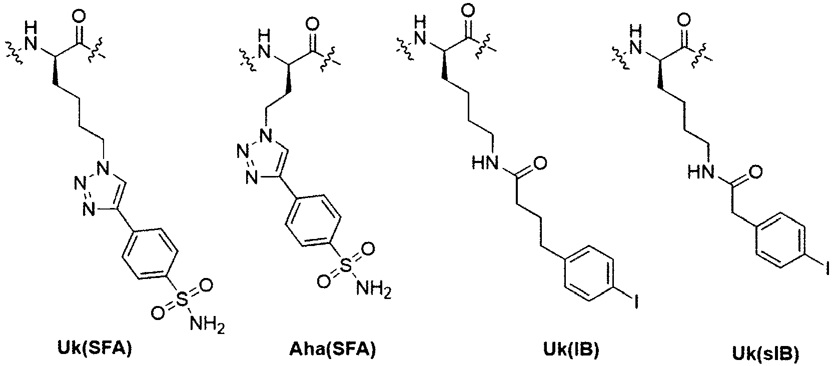

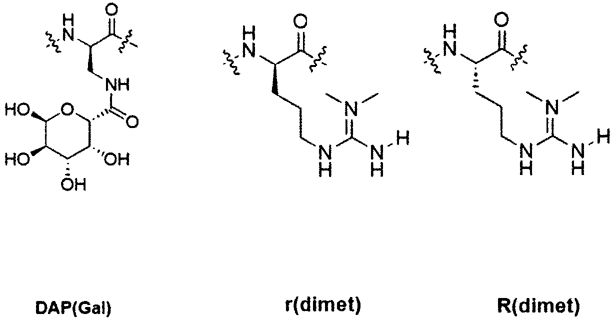

6. The peptide construct according to any one of claims 1 to 5,
**characterized in that** the functional group other than sulfonamide is introduced through the side chain ε-amino group of lysine residue or introduced through the side-chain β-amino group of 2,3-diaminopropionic acid (DAP) residue.

7. The peptide construct according to any one of claims 1 to 6,
**characterized in that** the functional group other than sulfonamide is chelators, biotin, glucoheptonic acid, 4-(p-iodophenyl) butyric acids (IB), 2-(4-iodophenyl)acetic acid (sIB), a zwitterionic group (SZW), α-D-galacturonic acid (Gal), cycloalkanes having 5 to 15 carbon atoms, fluorescent dyes, or cytotoxic agents.

8. The peptide construct according to claim 7,
**characterized in that** the chelator is one or more selected from 1,4,7,10-tetraazacyclododecane-1,4,7,10-tetraacetic acid (DOTA), 1,4,7-triazacyclononane-1,4,7-triacetic acid (NOTA), ethylenediaminetetraacetic acid-2,2',2",2"'-(ethane-1,2-diyldinitrilo)tetraacetic acid (EDTA), 1,4,7,10,13,16-hexaazacyclooctadecane-N,N',N",N‴,Nʺʺ,Nʺ‴-hexaacetic acid (HEHA), 2-[4-nitrobenzyl]-1,4,7,10, 13-pentaazacyclopentadecane-N,N',N",N‴,Nʺ‴-pentaacetic acid (PEPA), 1,4,7,10-tetraazacyclododecane-1,4,7,10-tetra(methylene phosphonic acid) (DOTP), (1R,4R,7R,10R)-α,α',α",α‴-tetramethyl-1,4,7,10-tetraazacyclododecane-1,4,7,10-tetraacetic acid tetrasodium salt (DOTMA), 2-[bis[2-[bis(carboxymethyl)amino]ethyl]amino]acetic acid (DTPA), triethylenetetramine (TETA), 1,4,7,10-tetraazacyclododecane-7-acetamide-1,4,10-triacetic acid (PSC) and 2-[4,7,10-tris(2-amino-2-oxoethyl)-1,4,7,10-tetraazacyclododecan-1-yl]acetamide (DOTAM or TCMC).

9. The peptide construct according to any one of claims 1 to 8,
**characterized in that** the spacer is at one or more selected from polyethyleneglycol (PEG) linker, glycine, sarcosine, γ-carboxyglutamic acid and peptide linkers consisting of 1 to 8 D-amino acids or L-amino acids.

10. The peptide construct according to any one of claims 1 to 9,
**characterized in that** the peptide construct has a structure represented by Formula 2: 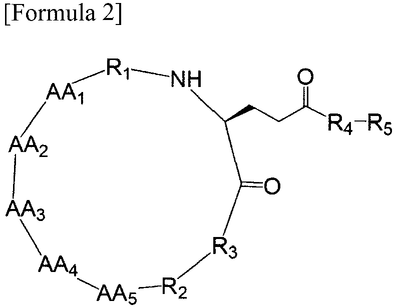 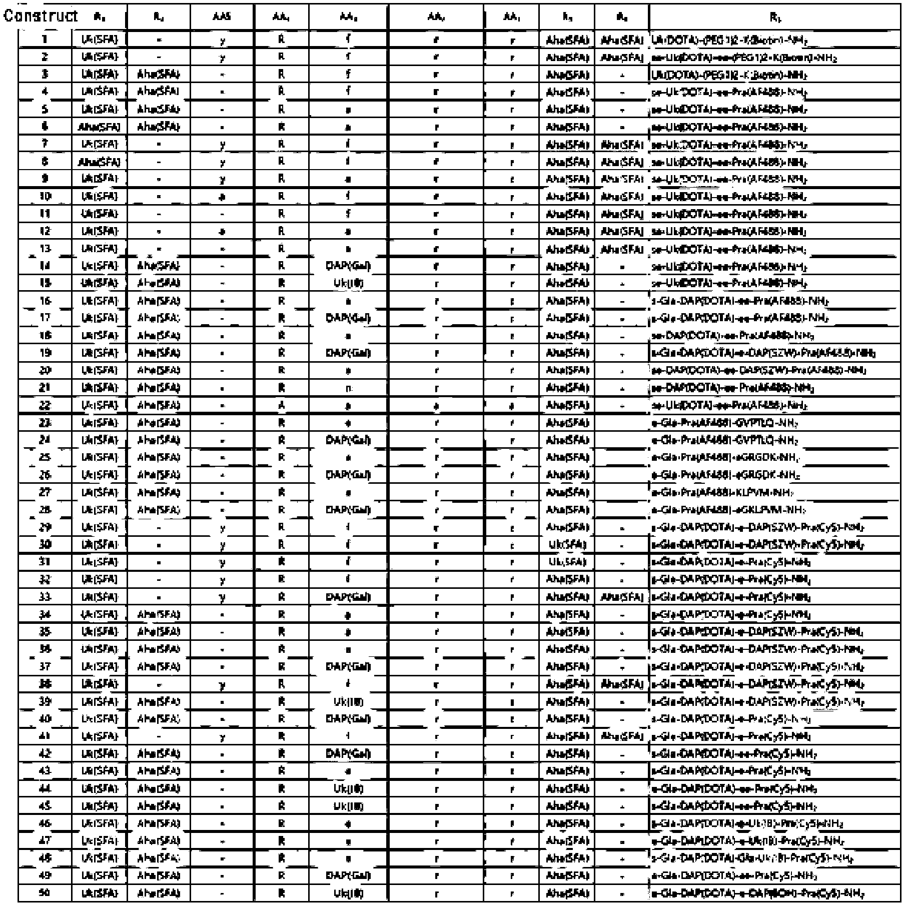 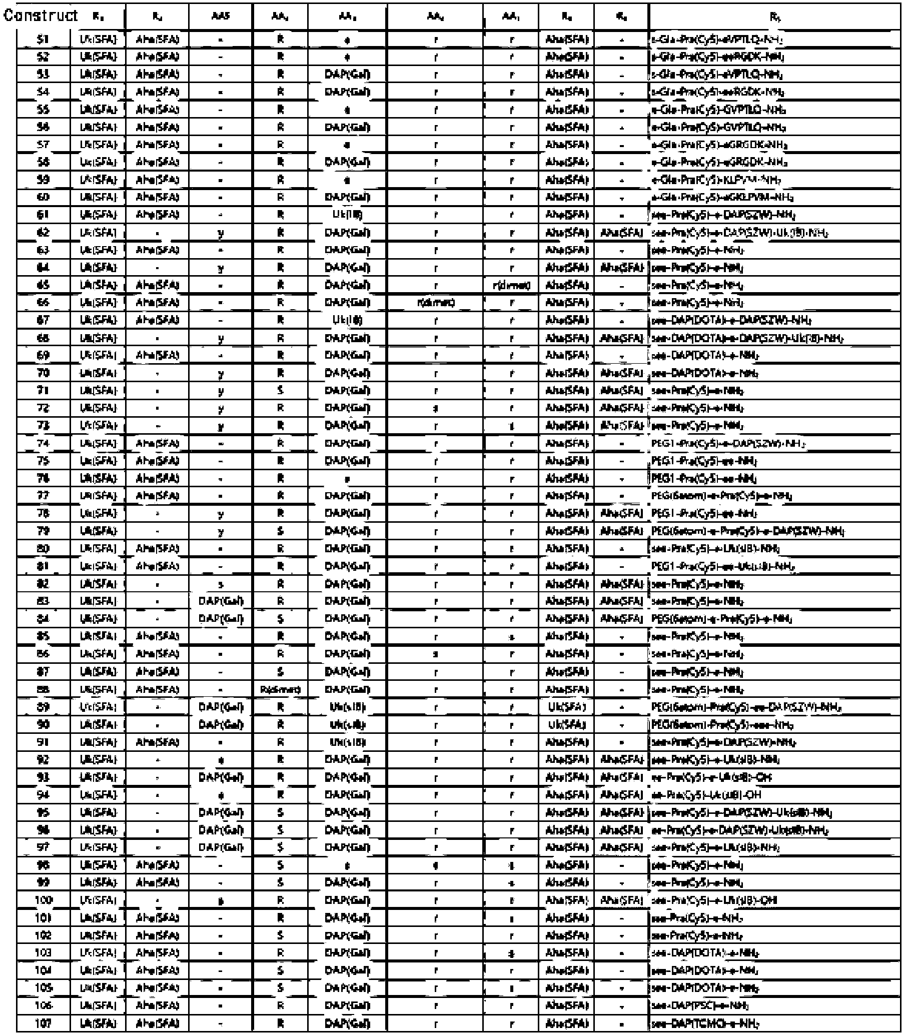
wherein the lowercase letters represent D-amino acids and the uppercase letters represent L-amino acids, and
each of the other substituents indicated by abbreviations at R₁ to R₅ and AA₁ to AA₅ is as defined below:

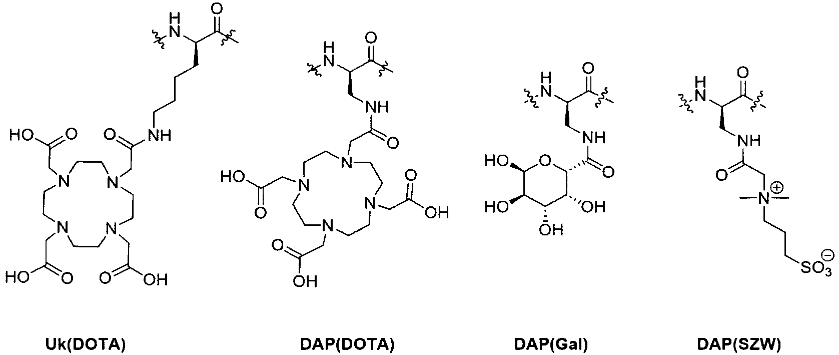

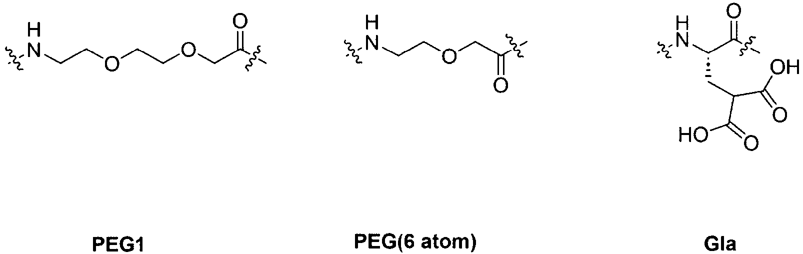

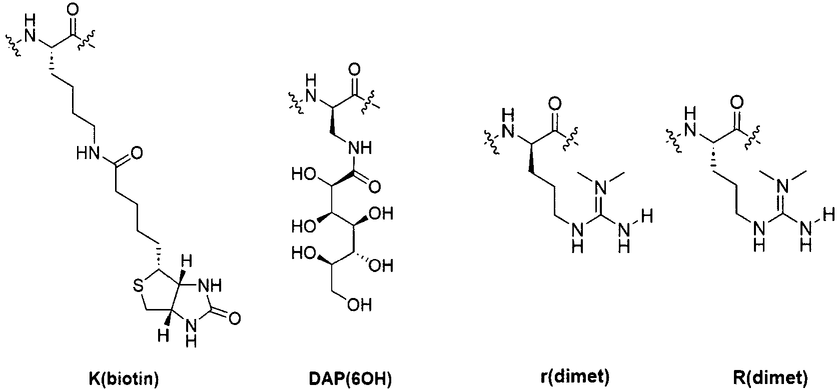

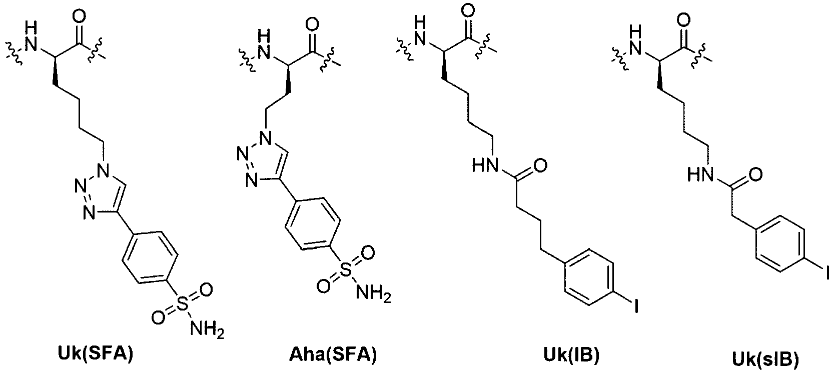

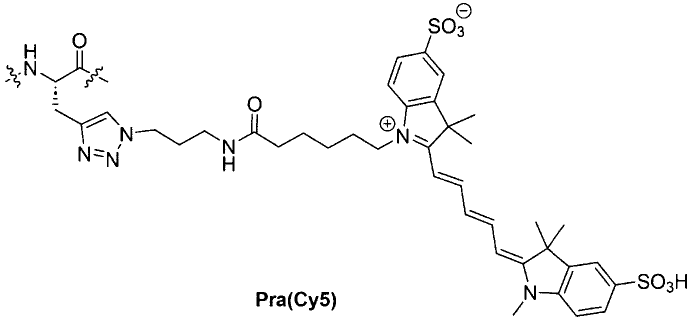

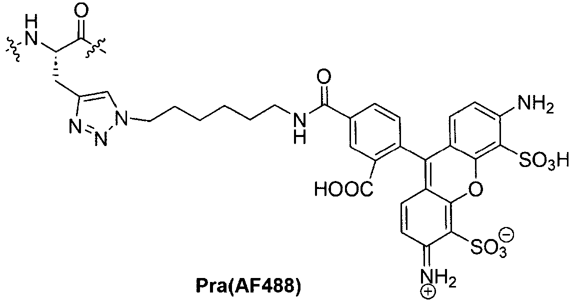

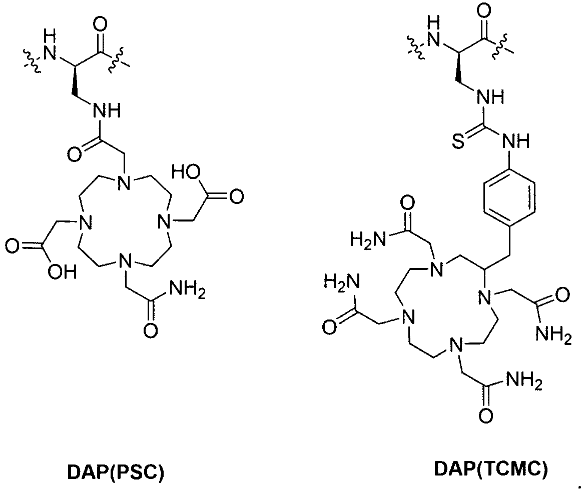

11. A conjugate comprising the peptide construct according to any one of claims 1 to 10 linked directly or via a linker to fluorescent dyes, cytotoxic agents or radioactive isotopes.

12. The conjugate according to claim 11,
**characterized in that** the linker is one or more selected from 6-maleimidocaproyl (MC), maleimidopropanoyl (MP), valine-citrulline (Val-Cit), alanine-phenylalanine (Ala-Phe), p-aminobenzyloxycarbonyl (PAB), N-succinimidyl 4-(2-pyridylthio)pentanoate (SPP), N-succinimidyl 4-(N-maleimidomethyl)cyclohexane-1 carboxylate (SMCC), 4-(2-pyridyldithio) butyric acid-N-hydroxysuccinimide ester (SPDB), and N-succinimidyl (4-iodo-acetyl) aminobenzoate (SIAB).

13. The conjugate according to any one of claim 11 or 12,
**characterized in that** the fluorescent dye is one or more selected from near-infrared fluorescent dye, fluorescein type, rhodamine type, Alexa Fluor, 4,4-difluoro-4-boro-3a, 4a-diaza-S-indacene (BODIPY), Texas Red, dansyl, Lissamine, cyanine (Cy), and phycoerythrin.

14. The conjugate according to any one of claims 11 to 13,
**characterized in that** the radioisotope is one or more selected from fluorine-18 (F-18), carbon-11 (C-11), carbon-14 (C-14), technetium-99m (Tc-99m) , copper-64 (Cu-64), copper-67 (Cu-67), dysprosium-168 (Dy-168), bismuth-213 (Bi-213), samarium-153 (Sm-153), strontium-89 (Sr-89), strontium-90 (Sr-90), erbium-169 (Er-169), phosphorus-32 (P-32), palladium-103 (Pd-103), rhenium-186 (Re-186), rhenium-188 (Re-188), oxygen-15 (O-15), selenium-75 (Se-75), sodium-24 (Na-24), strontium-85 (Sr-85), lutetium-177 (Lu-177), yttrium-90 (Y-90), iodine-123 (I-123), iodine-125 (I-125), iodine-131 (I-131), iridium-192 (Ir-192), iridium-196 (Ir-196), ytterbium-166 (Yb-166), indium-111 (In-111), xenon-133 (Xe-133), nitrogen-13 (N-13), calcium-47 (Ca-47), cobalt-57 (Co-57), cobalt-60 (Co-60), chromium-51 (Cr-51), krypton-81 (Kr-81), potassium-42 (K-42), holmium-166 (Ho-166), gallium-67 (Ga-67), gallium-68 (Ga-68), actinium-225 (Ac-225), zirconium-89 (Zr-89), thorium-227 (Th-227), radium-223 (Ra-223), tin-117m (Sn-117m), lead-103 (Pb-103), terbium-161 (Tb-161), lead-212 (Pb-212), and astatine-211 (At-211).

15. A conjugate comprising the peptide construct selected from the group consisting of Peptide Constructs 67 to 70 and 103 to 105 linked, directly or via a linker, to radioactive isotopes:

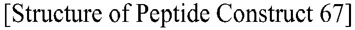

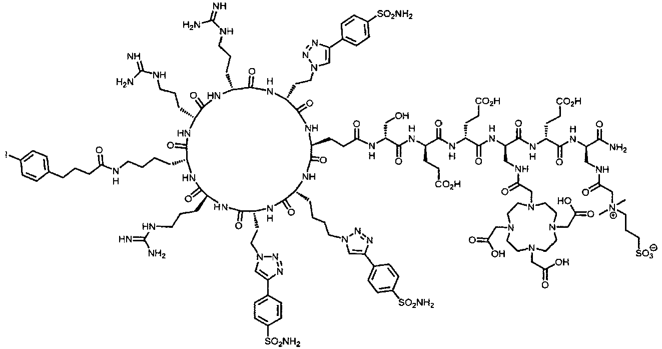

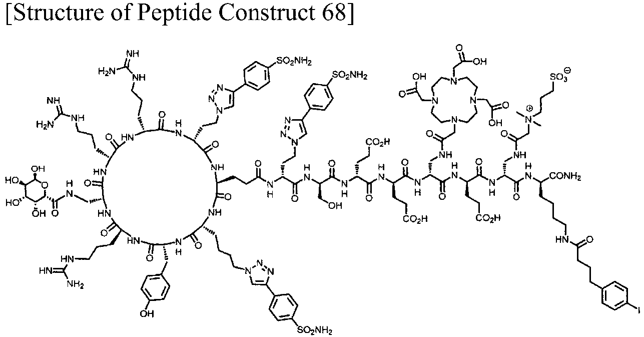

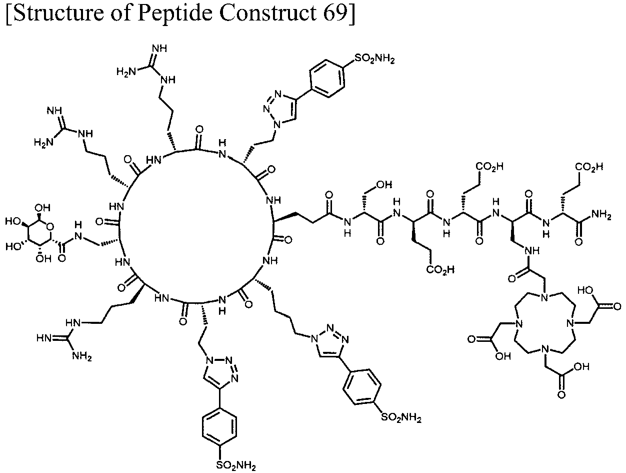

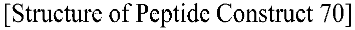

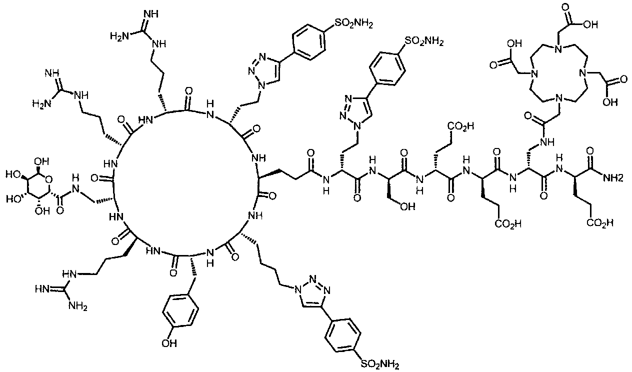

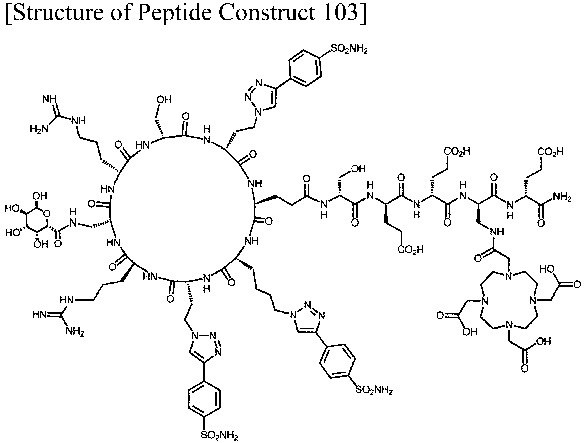

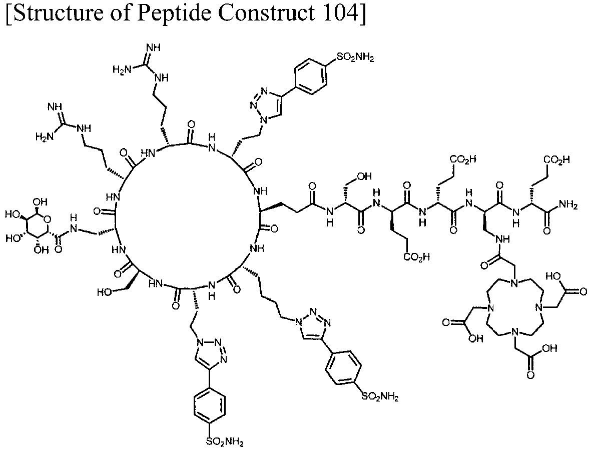

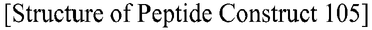

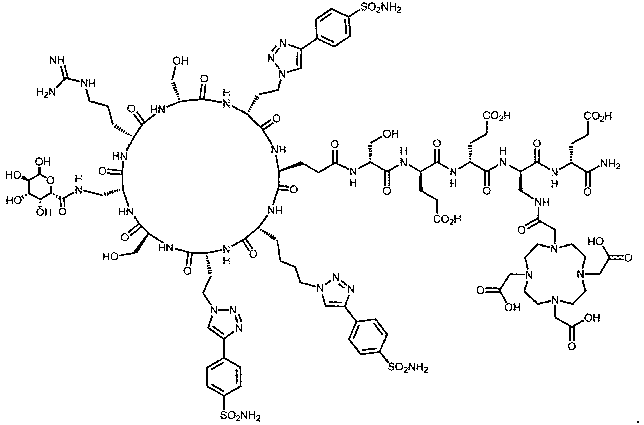

16. The conjugate according to claim 15,
**characterized in that** the radioisotope is one or more selected from fluorine-18 (F-18), copper-64 (Cu-64), indium-111 (In-111), gallium-68 (Ga-68), actinium-225 (Ac-225), lead-212 (Pb-212), and lutetium-177 (Lu-177).

17. The pharmaceutical composition for diagnosis, prevention, or treatment of cancer comprising the peptide construct according to any one of claims 1 to 10.

18. The pharmaceutical composition for diagnosis, prevention, or treatment of cancer comprising the conjugate according to any one of claims 11 to 16.

19. The pharmaceutical composition according to claim 17, wherein the cancer expresses carbonic anhydrase IX.

20. The pharmaceutical composition according to claim 17,
wherein the cancer is liver cancer, lung cancer, colorectal cancer, stomach cancer, breast cancer, colon carcinoma, bone cancer, pancreatic cancer, head and neck cancer, uterine cancer, ovarian cancer, rectal cancer, esophageal cancer, small intestine cancer, perianal cancer, fallopian tube carcinoma, endometrial carcinoma, cervical carcinoma, vaginal carcinoma, vulvar carcinoma, prostate cancer, biliary tract cancer, bladder cancer, kidney cancer, ureteric cancer, renal cell carcinoma, renal pelvic carcinoma, melanoma, thyroid cancer, astrocytoma or glioblastoma.

21. The pharmaceutical composition according to claim 18, wherein the cancer expresses carbonic anhydrase IX.

22. A pharmaceutical composition according to claim 18,
wherein the cancer is liver cancer, lung cancer, colorectal cancer, stomach cancer, breast cancer, colon carcinoma, bone cancer, pancreatic cancer, head and neck cancer, uterine cancer, ovarian cancer, rectal cancer, esophageal cancer, small intestine cancer, perianal cancer, fallopian tube carcinoma, endometrial carcinoma, cervical carcinoma, vaginal carcinoma, vulvar carcinoma, prostate cancer, biliary tract cancer, bladder cancer, kidney cancer, ureteric cancer, renal cell carcinoma, renal pelvic carcinoma, melanoma, thyroid cancer, astrocytoma or glioblastoma.
